# EUROPÄISCHE PATENTSCHRIFT

(11) **EP 1 296 979 B1**
(45) Veröffentlichungstag und Bekanntmachung des Hinweises auf die Patenterteilung: **04.01.2006**
(21) Anmeldenummer: 01953967.5
(22) Anmeldetag: 31.05.2001
(51) Int. Cl.: C07D 417/04, A01N 43/78

(54) **HETeroARYLSUBSTITUIERTE HETEROCYCLEN und ihre Verwendung als Pesticide**
HETeroARYL-SUBSTITUTED HETEROCYCLES and their use as pesticides
HETEROCYCLES A SUBSTITUTION HETEROARYLE et leur utilisation comme pesticides

(30) Priorität: 13.06.2000 DE 10029077
(43) Veröffentlichungstag der Anmeldung: 02.04.2003
(73) Patentinhaber: Bayer CropScience AG, 40789 Monheim (DE)
(72) Erfinder: FISCHER, Reiner, 40789 Monheim (DE); BRETSCHNEIDER, Thomas, 53797 Lohmar (DE); TRAUTWEIN, Axel, 51467 Bergisch Gladbach (DE); ULLMANN, Astrid, 50677 Köln (DE); DREWES, Mark, Wilhelm, 40764 Langenfeld (DE); ERDELEN, Christoph, 42799 Leichlingen (DE); DAHMEN, Peter, 41470 Neuss (DE); FEUCHT, Dieter, 40789 Monheim (DE); PONTZEN, Rolf, 42799 Leichlingen (DE)
(86) Internationale Anmeldenummer: PCT/EP2001/006174
(87) Internationale Veröffentlichungsnummer: WO 2001/096333

(56) Entgegenhaltungen:
- EP-A- 0 017 850
- WO-A-99/32464
- US-A- 4 377 588

## Beschreibung

Die vorliegende Erfindung betrifft neue hetarylsubstituierte Heterocyclen, mehrere Verfahren zu ihrer Herstellung und ihre Verwendung als Schädlingsbekämpfungsmittel und Herbizide.

Bekannt sind 4-Thiazolyl-3-hydroxy-3-pyrrolin-2,5-dione als Glykolsäureoxidase-Inhibitoren; US 4,296,237; US 4,377,588; EP-A-025 232; Rooney C.S. et al., J. Med. Chem. 26 700-714 (1983); Guzel Y. et al. THEOCHEM 366 131-137 (1996).

Ebenfalls bekannt sind Pyrrolinderivate aus US-A-4,377,588

Es wurden nun neue Verbindungen der Formel (I) gefunden 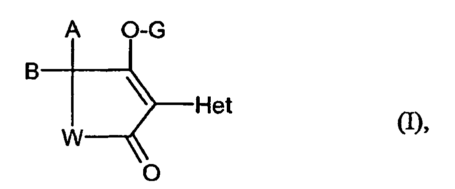
in welcher
- W: für N-D ⁽¹⁾, Sauerstoff ⁽²⁾ oder Schwefel ⁽³⁾ steht,
- Het: für jeweils gegebenenfalls substituiertes Thiazolyl, Oxazolyl oder Pyrazolyl steht,
- A: für Wasserstoff, jeweils gegebenenfalls durch Halogen substituiertes Alkyl, Alkenyl, Alkoxyalkyl, Polyalkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
- D: für Wasserstoff oder einen gegebenenfalls substituierten Rest aus der Reihe Alkyl, Alkenyl, Alkinyl, Alkoxyalkyl, Polyalkoxyalkyl, Alkylthioalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
- A und D: gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen,
- G: für Wasserstoff (a) oder für eine der Gruppe 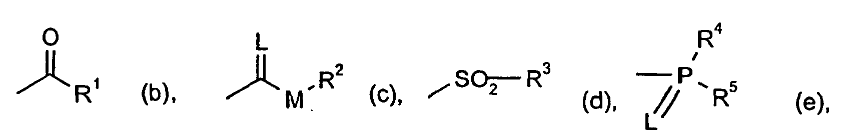 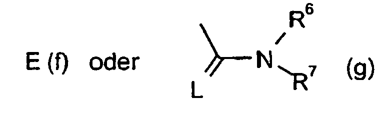 steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³ für Alkyl, Halogenalkyl oder für jeweils gegebenenfalls substituiertes Phenyl oder Benzyl steht,
R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio und für jeweils gegebenenfalls substituiertes Phenyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung von W = N-D ⁽¹⁾, Sauerstoff ⁽²⁾ oder Schwefel ⁽³⁾ ergeben sich folgende hauptsächliche Strukturen (I-1) bis (I-3): 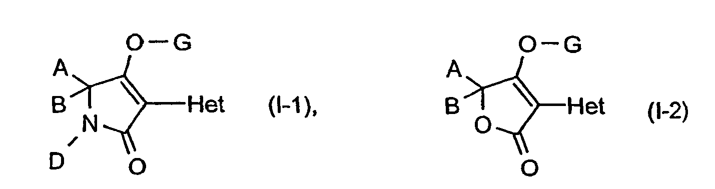 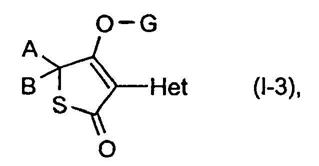
worin
- A, B, D und G: die oben angegebene Bedeutung haben und
- Het: für Thiazolyl oder Oxazolyl steht.

Unter Einbeziehung der verschiedenen Bedeutungen von Het ergeben sich folgende hauptsächlichen Strukturen:

(I-1-A) bis (I-3-B) wenn W für N-D ⁽¹⁾, Sauerstoff ⁽²⁾ oder Schwefel ⁽³⁾ steht 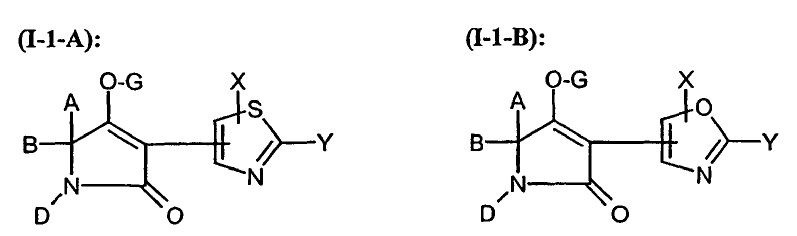 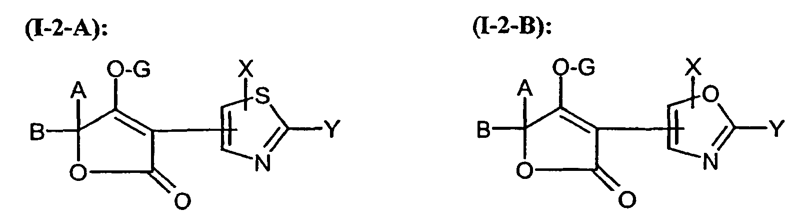 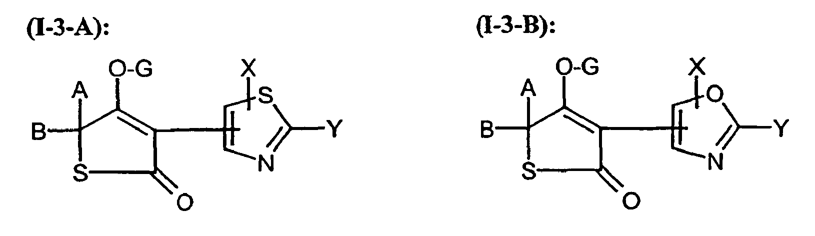
in welchen
- A, B, D und G: die oben angegebene Bedeutung haben und
- X: für Wasserstoff, Halogen, Alkyl, Halogenalkyl, Alkoxy, Halogenalkoxy, Alkenyloxy, Haloalkenyloxy, Nitro, Cyano oder gegebenenfalls substituiertes Phenyl steht,
- Y: für Halogen, Alkyl, Halogenalkyl, Alkoxy, Halogenalkoxy, Alkenyloxy, Alkylthio, Alkylsulfinyl, Alkylsulfoxinyl, Alkylsulfonyl oder für jeweils gegebenenfalls substituiertes Phenyl, Benzyl, Phenoxy oder Phenylthio steht.

Unter Einbeziehung der verschiedenen Bedeutungen (a), (b), (c), (d), (e), (f) und (g) der Gruppe G ergeben sich folgende hauptsächliche Strukturen (I-1-A-a) bis (I-1-A-g), wenn W für N-D ⁽¹⁾ steht, 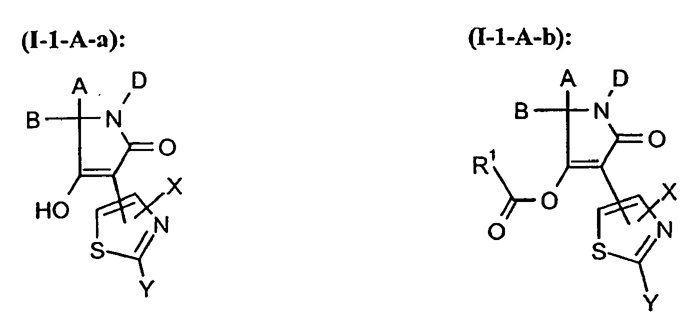 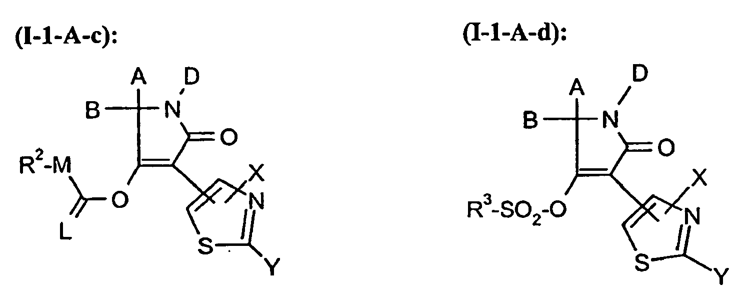 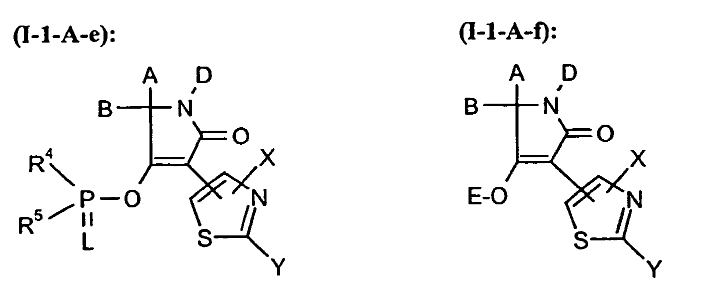 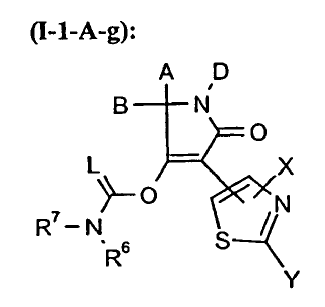
worin
A, B, D, E, L, M, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-1-B-a) bis (I-1-B-g), wenn W für N-D ⁽¹⁾ steht, 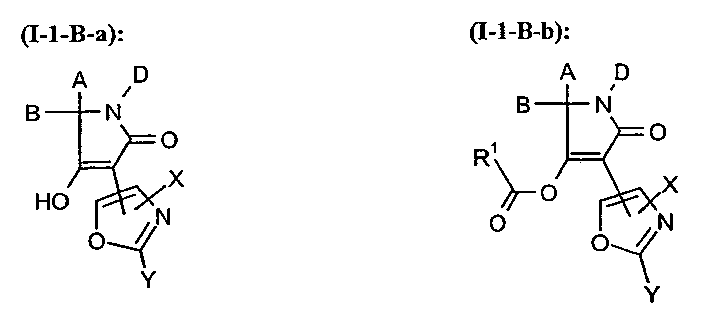 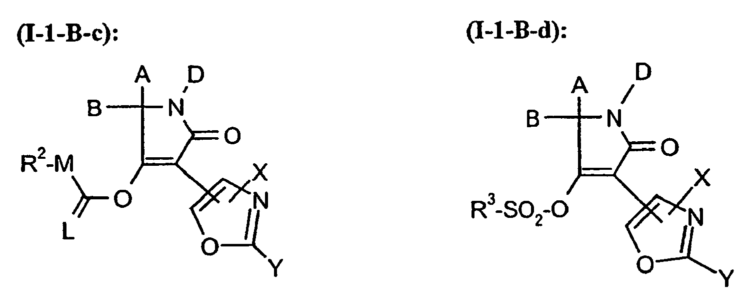 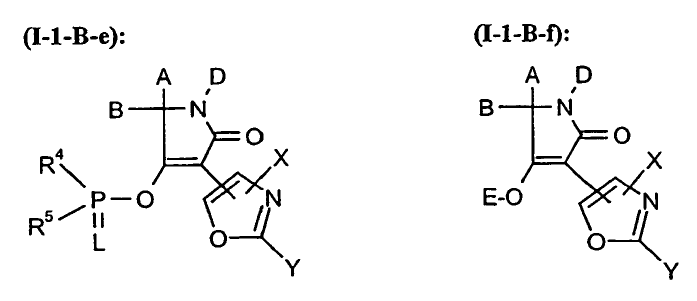 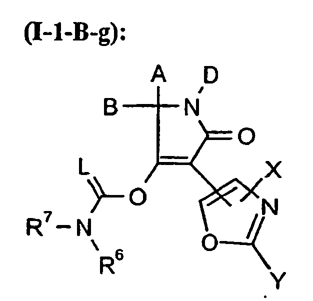
worin
A, B, D, E, L, M, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-A-a) bis (I-2-A-g), wenn W für Sauerstoff⁽²⁾ steht, 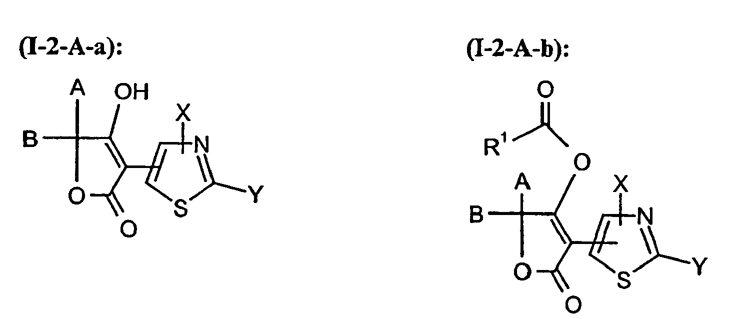 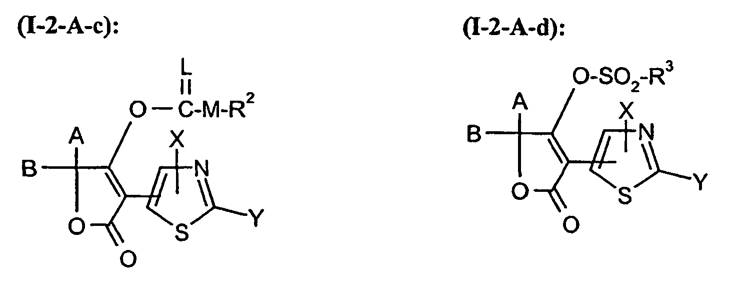 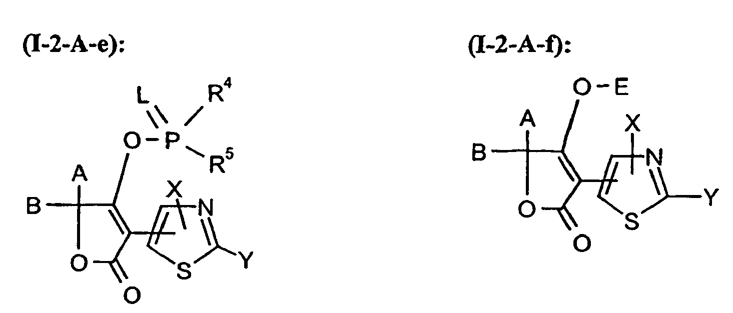 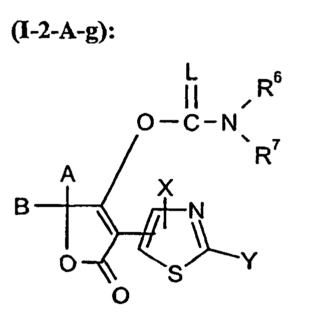
worin
A, B, E, L, M, X, Y, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-2-B-a) bis (I-2-B-g), wenn W für Sauerstoff⁽²⁾ steht, 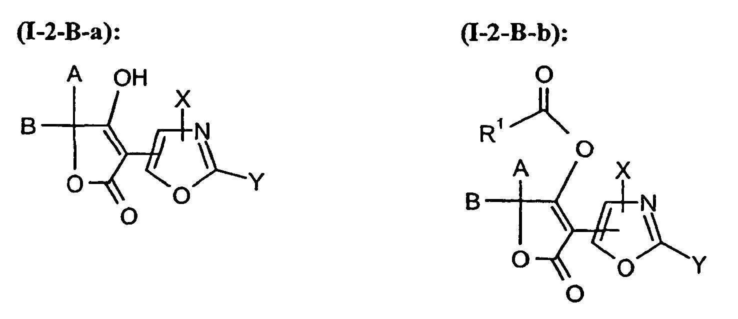 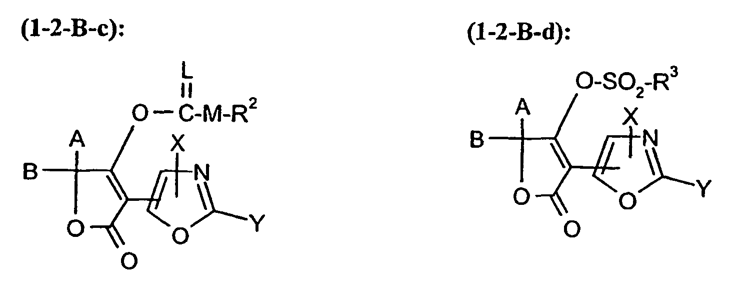 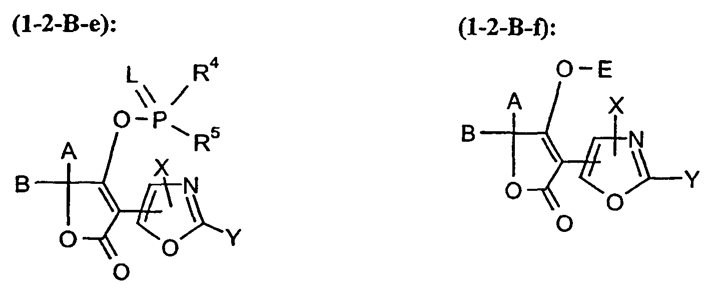 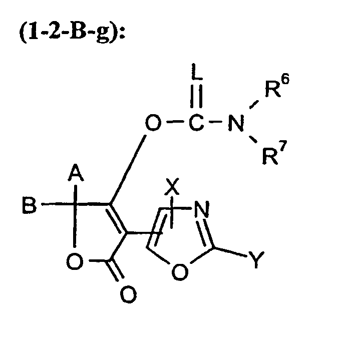
worin
A, B, E, L, M, X, Y, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-A-a) bis (I-3-A-g), wenn W für Schwefel ⁽³⁾ steht, 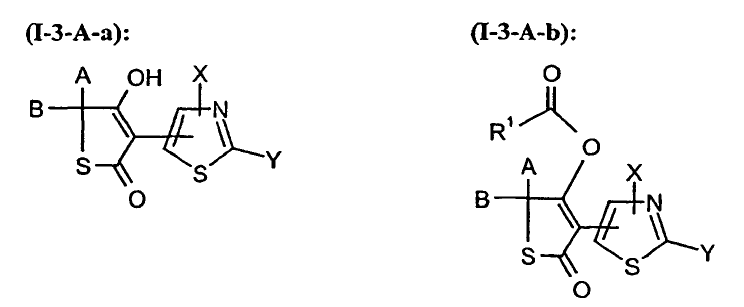 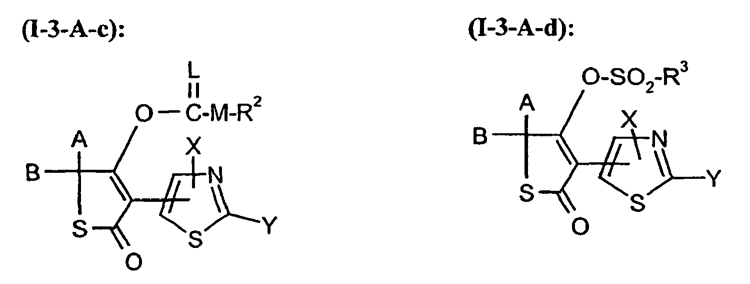 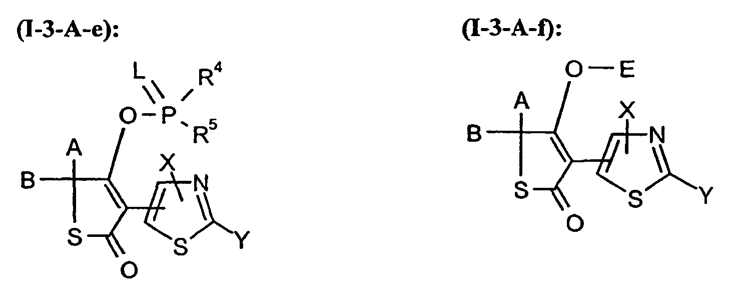 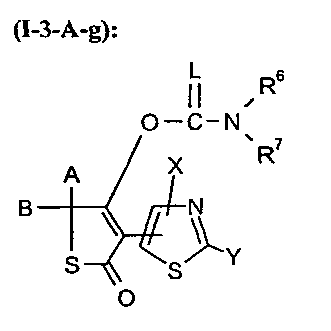
worin
A, B, E, L, M, X, Y, R¹, R², R³, R⁴, R⁵, R⁶ und R⁷ die oben angegebenen Bedeutung besitzen.

Unter Einbeziehung der verschiedenen Bedeutungen (a), (b), (c), (d), (e), (f) und (g) der Gruppe G ergeben sich folgende hauptsächliche Strukturen (I-3-B-a) bis (I-3-B-g), wenn W für Schwefel ⁽³⁾ steht, 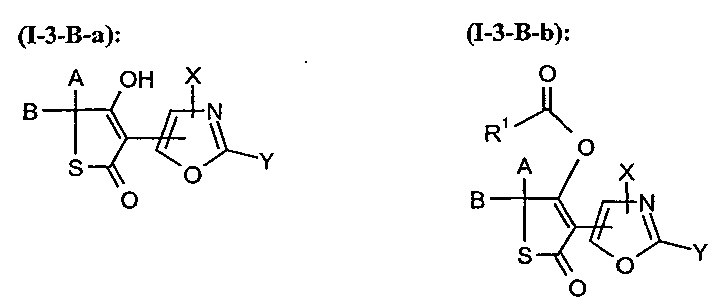 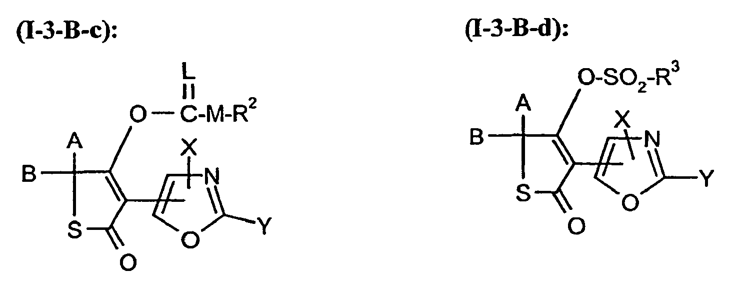 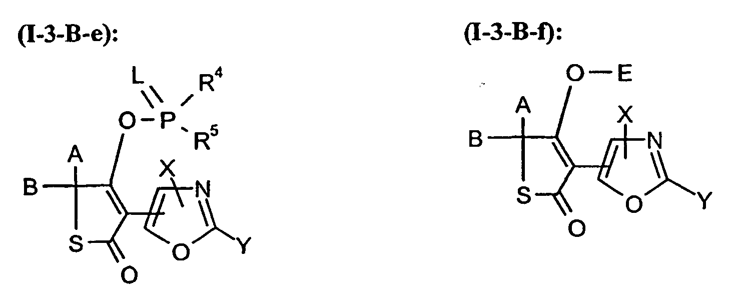 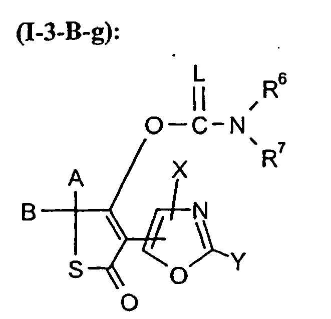
worin
A, B, E, L, M, X, Y, R¹, R², R³, R⁴, R⁵, R⁶ und R⁷ die oben angegebenen Bedeutung besitzen.

Weiterhin wurde gefunden, dass man die neuen Verbindungen der Formel (I) nach einem der im folgenden beschriebenen Verfahren erhält:
(A) Man erhält substituierte 3-Hetaryl-pyrrolidin-2,4-dione bzw. deren Enole der Formel (I-1-A-a) bis (I-1-B-a) 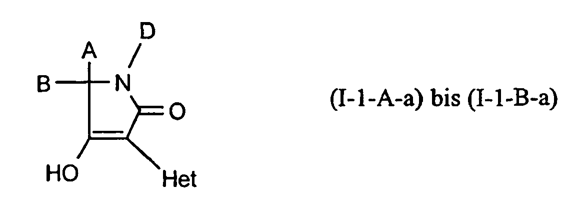
   in welcher
   - A, B, D und Het: die oben angegebenen Bedeutungen haben,

   wenn man
   N-Acylaminosäureester der Formel (II) 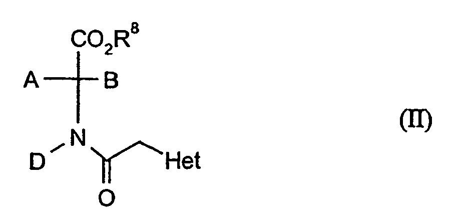
   in welcher
   - A, B, D und Het: die oben angegebenen Bedeutungen haben,

   und
   - R⁸: für Alkyl (bevorzugt C₁-C₆-Alkyl) steht,

   in Gegenwart eines Verdünnungsmittels und in Gegenwart einer Base intramolekular kondensiert.
(B) Außerdem wurde gefunden, dass man substituierte 3-Hetaryl-4-hydroxy-Δ³-dihydrofuranon-Derivate der Formel (I-2-A-a) bis (I-2-B-a) 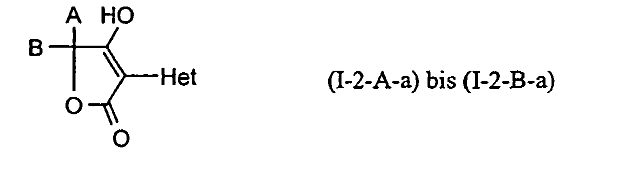
   in welcher
   - A, B und Het: die oben angegebenen Bedeutungen haben,

   erhält, wenn man
   Carbonsäureester der Formel (III) 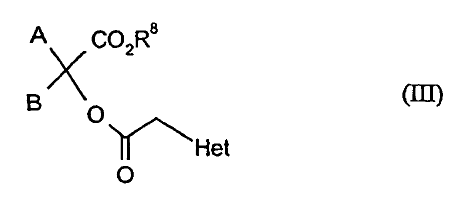
   in welcher
   - A, B, Het und R⁸: die oben angegebenen Bedeutungen haben,

   in Gegenwart eines Verdünnungsmittels und in Gegenwart einer Base intramolekular kondensiert.
(C) Weiterhin wurde gefunden, dass man substituierte 3-Hetaryl-4-hydroxy-Δ³-dihydrothiophen-2-on-Derivate der Formel (I-3-A-a) bis (I-3-B-a) 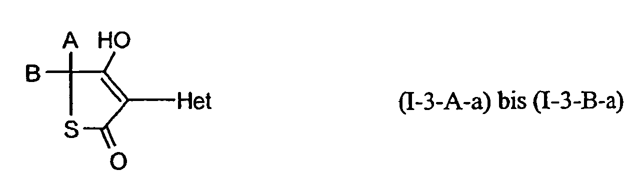
   in welcher
   - A, B und Het: die oben angegebenen Bedeutungen haben,

   erhält, wenn man
   β-Ketocarbonsäureester der Formel (IV) 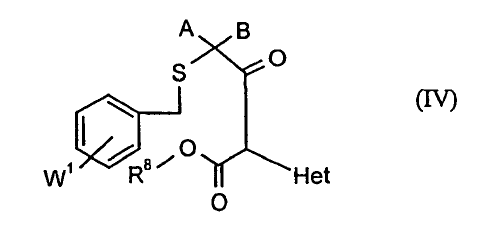
   in welcher
   - A, B, Het und R⁸: die oben angegebenen Bedeutungen haben und
   - W¹: für Wasserstoff, Halogen, Alkyl (bevorzugt C₁-C₆-Alkyl) oder Alkoxy (bevorzugt C₁-C₈-Alkoxy) steht,

   gegebenenfalls in Gegenwart eines Verdünnungsmittels und in Gegenwart einer Säure intramolekular cyclisiert.
   Außerdem wurde gefunden
(D) dass man die Verbindungen der oben gezeigten Formeln (I-1-A-b) bis (I-3-B-b), in welchen A, B, D, R¹ und Het die oben angegebenen Bedeutungen haben, erhält, wenn man Verbindungen der oben gezeigten Formeln (I-1-A-a) bis (I-3-B-a), in welchen A, B, D und Het die oben angegebenen Bedeutungen haben, jeweils
   (α) mit Säurehalogeniden der Formel (V) 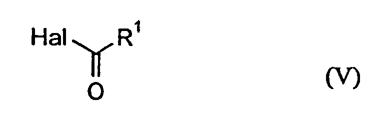
      in welcher
      - R¹: die oben angegebene Bedeutung hat und
      - Hal: für Halogen (insbesondere Chlor oder Brom) steht

      oder
   (β) mit Carbonsäureanhydriden der Formel (VI)

      R¹-CO-O-CO-R¹ (VI)

      in welcher
      - R¹: die oben angegebene Bedeutung hat,

      gegebenenfalls in Gegenwart eines Verdünnungsmittels und gegebenenfalls in Gegenwart eines Säurebindemittels umsetzt;
(E) dass man die Verbindungen der oben gezeigten Formeln (I-1-A-c) bis (I-3-B-c), in welchen A, B, D, R², M und Het die oben angegebenen Bedeutungen haben und L für Sauerstoff steht, erhält, wenn man Verbindungen der oben gezeigten Formeln (I-1-A-a) bis (I-3-B-a), in welchen A, B, D und Het die oben angegebenen Bedeutungen haben, jeweils
   mit Chlorameisensäureestern oder Chlorameisensäurethioestem der Formel (VII)

   R²-M-CO-Cl (VII)

   in welcher
   - R² und M: die oben angegebenen Bedeutungen haben,

   gegebenenfalls in Gegenwart eines Verdünnungsmittels und gegebenenfalls in Gegenwart eines Säurebindemittels umsetzt;
(F) dass man Verbindungen der oben gezeigten Formeln (I-1-A-c) bis (I-3-B-c), in welchen A, B, D, R², M und Het die oben angegebenen Bedeutungen haben und L für Schwefel steht, erhält, wenn man Verbindungen der oben gezeigten Formeln (I-1-A-a) bis (I-3-B-a), in welchen A, B, D und Het die oben angegebenen Bedeutungen haben, jeweils
   mit Chlormonothioameisensäureestem oder Chlordithioameisensäureestern der Formel (VIII) 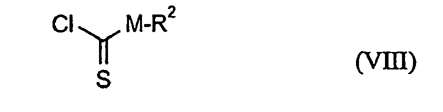
   in welcher
   - M und R²: die oben angegebenen Bedeutungen haben,

   gegebenenfalls in Gegenwart eines Verdünnungsmittels und gegebenenfalls in Gegenwart eines Säurebindemittels umsetzt
   und
(G) dass man Verbindungen der oben gezeigten Formeln (I-1-A-d) bis (I-3-B-d), in welchen A, B, D, R³ und Het die oben angegebenen Bedeutungen haben, erhält, wenn man Verbindungen der oben gezeigten Formeln (I-1-A-a) bis (I-3-B-a), in welchen A, B, D und Het die oben angegebenen Bedeutungen haben, jeweils
   mit Sulfonsäurechloriden der Formel (IX)

   R³-SO₂-Cl (IX)

   in welcher
   - R³: die oben angegebene Bedeutung hat,

   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-A-e) bis (I-3-B-e), in welchen A, B, D, L, R⁴, R⁵ und Het die oben angegebenen Bedeutungen haben, erhält, wenn man Verbindungen der oben gezeigten Formeln (I-1-A-a) bis (I-3-B-a), in welchen A, B, D und Het die oben angegebenen Bedeutungen haben, jeweils
   mit Phosphorverbindungen der Formel (X) 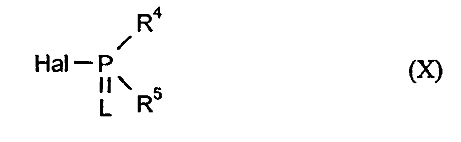
   in welcher
   - L, R⁴ und R⁵: die oben angegebenen Bedeutungen haben und
   - Hal: für Halogen (insbesondere Chlor oder Brom) steht,

   gegebenenfalls in Gegenwart eines Verdünnungsmittels und gegebenenfalls in Gegenwart eines Säurebindemittels umsetzt,
(I) dass man Verbindungen der oben gezeigten Formeln (I-1-A-f) bis (I-3-B-f), in welchen A, B, D, E und Het die oben angegebenen Bedeutungen haben, erhält, wenn man Verbindungen der Formeln (I-1-A-a) bis (I-3-B-a), in welchen A, B, D und Het die oben angegebenen Bedeutungen haben, jeweils
   mit Metallverbindungen oder Aminen der Formeln (XI) oder (XII)

   Me(OR¹⁰)ₜ (XI)

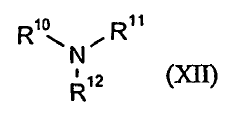

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,

   gegebenenfalls in Gegenwart eines Verdünnungsmittels umsetzt,
(J) dass man Verbindungen der oben gezeigten Formeln (I-1-A-g) bis (I-3-B-g), in welchen A, B, D, L, R⁶, R⁷ und Het die oben angegebenen Bedeutungen haben, erhält, wenn man Verbindungen der oben gezeigten Formeln (I-1-A-a) bis (I-3-B-a), in welchen A, B, D und Het die oben angegebenen Bedeutungen haben, jeweils
   (α) mit Isocyanaten oder Isothiocyanaten der Formel (XIII)

      R⁶-N=C=L (XIII)

      in welcher
      - R⁶ und L: die oben angegebenen Bedeutungen haben,

      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XIV) 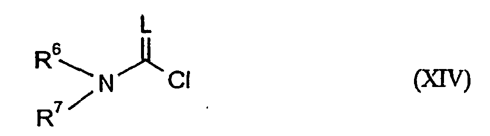
      in welcher
      - L, R⁶ und R⁷: die oben angegebenen Bedeutungen haben,

      gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- Het: steht bevorzugt für 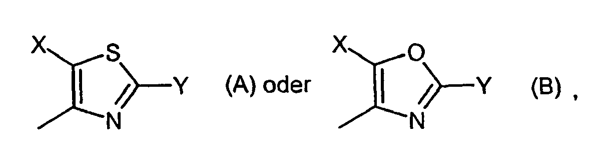
X steht bevorzugt für Wasserstoff, Halogen, C₁-C₆-Alkyl, C₁-C₄-Halogenalkyl, C₁-C₆-Alkoxy, C₃-C₆-Alkenyloxy, Nitro oder Cyano,
Y steht bevorzugt für Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Alkylsulfoxinyl oder für die Gruppe 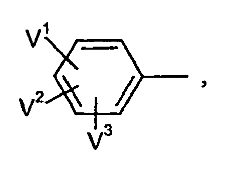
oder im Falle von Het = Thiazolyl ((I-1-A) bis (I-3-A)) auch für 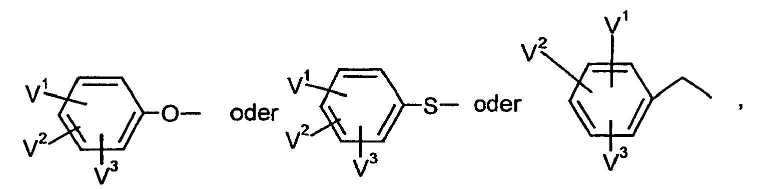
V¹ steht bevorzugt für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano, Phenoxy oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
V² und V³ stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy oder
V¹ und V² stehen zusammen gemeinsam mit den Kohlenstoffatomen, an die sie gebunden sind bevorzugt für einen gegebenenfalls durch C₁-C₄-Alkyl oder Halogen substituierten gesättigten oder ungesättigten 5- oder 6-gliedrigen Cyclus, in welchem gegebenenfalls ein bis drei Kohlenstoffatome durch Sauerstoff, Schwefel oder Stickstoff ersetzt sein können,
W steht bevorzugt für N-D ⁽¹⁾, Sauerstoff⁽²⁾ oder Schwefel ⁽³⁾,
A steht bevorzugt für Wasserstoff oder jeweils gegebenenfalls durch Fluor oder Chlor substituiertes C₁-C₁₂-Alkyl, C₃-C₈-Alkenyl, C₁-C₆-Alkoxy-C₁-C₆-alkyl, Poly-C₁-C₆-alkoxy-C₁-C₆-alkyl, C₁-C₁₀-Alkylthio-C₁-C₆-alkyl, gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl (Phenyl oder Naphthyl), Hetaryl mit 5 bis 6 Ringatomen (beispielsweise Furanyl, Pyridyl, Imidazolyl, Triazolyl, Pyrazolyl, Pyrimidyl, Thiazolyl oder Thienyl) oder C₆- oder C₁₀-Aryl-C₁-C₆-alkyl (Phenyl-C₁-C₆-alkyl oder Naphthyl-C₁-C₆-alkyl),
B steht bevorzugt für Wasserstoff, C₁-C₆-Alkyl oder C₁-C₄-Alkoxy-C₁-C₄-alkyl oder
A, B und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₄-Halogenalkyl, C₁-C₆-Alkoxy, C₁-C₆-Alkylthio, Halogen oder Phenyl substituiert sind oder
A, B und das Kohlenstoffatom, an das sie gebunden sind, stehen bevorzugt für C₅-C₆-Cycloalkyl, welches durch eine gegebenenfalls ein Sauerstoff- oder Schwefelatom enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis sechsgliedrigen Ring bildet der gegebenenfalls einfach bis vierfach durch C₁-C₄-Alkyl substituiert sein kann, oder
A, B und das Kohlenstoffatom, an das sie gebunden sind, stehen bevorzugt für C₃-C₈-Cycloalkyl oder C₅-C₈-Cycloakenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D steht bevorzugt für Wasserstoff, jeweils gegebenenfalls durch Halogen oder Cyano substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogen-alkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl), Phenyl-C₁-C₆-alkyl oder Hetaryl-C₁-C₆-alkyl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl) oder
A und D stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1-A) bis (I-1-B) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 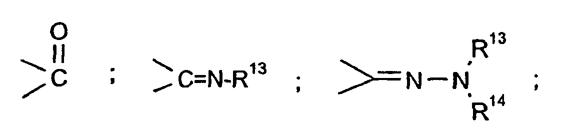 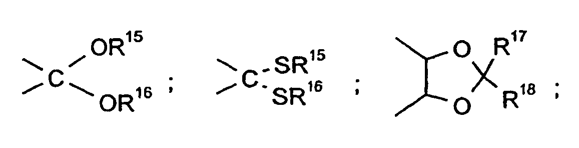 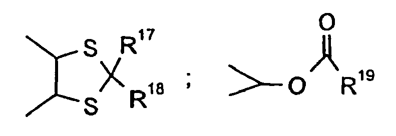 oder 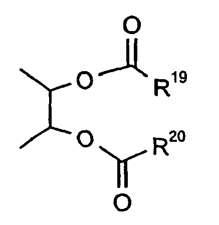
   enthalten ist,
   G steht bevorzugt für Wasserstoff (a) oder für eine der Gruppen 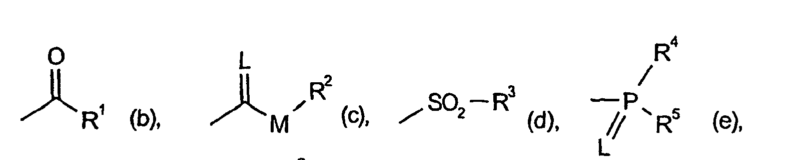 E (f) oder 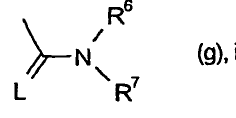 insbesondere für (a), (b), (c) oder (g),

in welchen
E für ein Metallionäquivalent oder ein Ammoniumion steht,
L für Sauerstoff oder Schwefel steht und
M für Sauerstoff oder Schwefel steht,
R¹ steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen, C₁-C₆-Alkyl, C₁-C₂-Halogenalkyl oder C₁-C₄-Alkoxy substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl).
R² steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl in welchem gegebenenfalls ein Ringatom durch Sauerstoff ersetzt ist, oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.
R³ steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
R⁴ und R⁵ stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio,
R⁶ und R⁷ stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls ein Kohlenstoffatom durch Sauerstoff oder Schwefel ersetzt ist,
R¹³ steht bevorzugt für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenakoxy, Nitro oder Cyano substituiertes Phenyl, Phenyl-C₁-C₄-alkyl oder Phenyl-C₁-C₄-alkoxy,
R¹⁴ steht bevorzugt für Wasserstoff oder C₁-C₈-Alkyl oder
R¹³ und R¹⁴ stehen gemeinsam bevorzugt für C₄-C₆-Alkandiyl.
R¹⁵ und R¹⁶ sind gleich oder verschieden und stehen bevorzugt für C₁-C₆-Alkyl oder
R¹⁵ und R¹⁶ stehen gemeinsam bevorzugt für einen C₂-C₄-Alkandiylrest, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist.
R¹⁷ und R¹⁸ stehen unabhängig voneinander bevorzugt für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
R¹⁷ und R¹⁸ stehen gemeinsam mit dem Kohlenstoffatom, an das sie gebunden sind, bevorzugt für eine Carbonylgruppe oder für gegebenenfalls durch Halogen, C₁-C₄-Alkyl oder C₁-C₄-Alkoxy substituiertes C₅-C₇-Cycloalkyl, in dem gegebenenfalls eine Methylengruppe durch Sauerstoff oder Schwefel ersetzt ist.
R¹⁹ und R²⁰ stehen unabhängig voneinander bevorzugt für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino.

In den als bevorzugt genannten Restedefinitionen steht Halogen für Fluor, Chlor, Brom und Iod, insbesondere für Fluor, Chlor und Brom.
- Het: steht besonders bevorzugt für 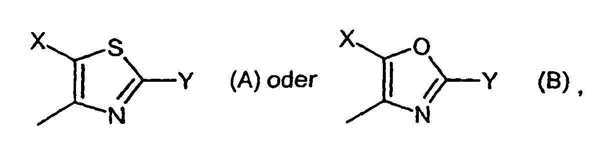
- X: steht besonders bevorzugt für Wasserstoff, Chlor, Brom, C₁-C₄-Alkyl, C₁-C₂-Halogenalkyl, Nitro oder Cyano,
- Y: steht besonders bevorzugt für Chlor, Brom, C₁-C₄-Alkyl, C₁-C₄-Halogen-alkyl, C₁-C₄-Alkoxy, C₁-C₄-Halogenalkoxy, C₁-C₄-Alkylthio, C₁-C₄-Alkylsulfonyl oder für die Gruppe 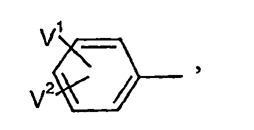
oder im Falle von Het = Thiazolyl ((I-1-A) bis (I-3-A)) auch für 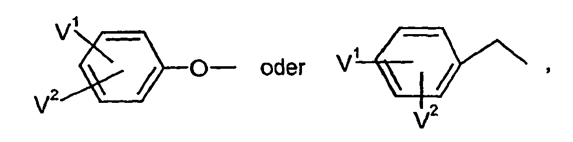
- V¹: steht besonders bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano, Phenoxy oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio,
- V²: steht besonders bevorzugt für Wasserstoff, Fluor, Chlor, Brom, C₁-C₄-Alkyl, C₁-C₄-Alkoxy, C₁-C₂-Halogenalkyl oder C₁-C₂-Halogenalkoxy oder
- V¹ und V²: stehen zusammen gemeinsam mit den Kohlenstoffatomen, an die sie gebunden sind besonders bevorzugt für einen gegebenenfalls durch Fluor oder Methyl substituierten 5- oder 6-gliedrigen Cyclus, in welchem gegebenenfalls ein bis zwei Kohlenstoffatome durch Sauerstoff ersetzt sein können.
- W: steht besonders bevorzugt für N-D ⁽¹⁾, Sauerstoff⁽²⁾ oder Schwefel ⁽³⁾,
- A: steht besonders bevorzugt für Wasserstoff, jeweils gegebenenfalls durch Fluor substituiertes C₁-C₁₀-Alkyl, C₁-C₄-Alkoxy-C₁-C₄-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Phenyl-C₁-C₂-alkyl,
- B: steht besonders bevorzugt für Wasserstoff oder C₁-C₄-Alkyl oder
- A, B: und das Kohlenstoffatom an das sie gebunden sind, stehen besonders bevorzugt für gesättigtes C₅-C₇-Cycloalkyl, worin gegebenenfalls ein Ringglied durch Sauerstoff oder Schwefel ersetzt ist und welches gegebenenfalls einfach oder zweifach durch C₁-C₆-Alkyl, C₅-C₆-Cycloalkyl, C₁-C₂-Halogenalkyl, C₁-C₆-Alkoxy, Fluor, Chlor oder Phenyl substituiert ist oder
- A, B: und das Kohlenstoffatom, an das sie gebunden sind, stehen besonders bevorzugt für C₅-C₆-Cycloalkyl, welches durch eine gegebenenfalls ein Sauerstoff- oder Schwefelatom enthaltende Alkylendiyl- oder durch eine Alkylendioxyl- oder durch eine Alkylendithioyl-Gruppe substituiert ist, die mit dem Kohlenstoffatom, an das sie gebunden ist, einen weiteren fünf- oder sechsgliedrigen Ring bildet der gegebenenfalls einfach bis dreifach durch C₁-C₃-Alkyl substituiert sein kann, oder
- A, B: und das Kohlenstoffatom, an das sie gebunden sind, stehen besonders bevorzugt für C₅-C₆-Cycloalkyl oder C₅-C₆-Cycloalkenyl, in welchen zwei Sub- stituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl, C₂-C₄-Alkendiyl, worin gegebenenfalls eine Methylengruppe durch Sauerstoff oder Schwefel ersetzt ist oder Butadiendiyl stehen,
- D: steht besonders bevorzugt für Wasserstoff, für jeweils gegebenenfalls durch Fluor substituiertes C₁-C₁₀-Alkyl, C₃-C₆-Alkenyl, C₁-C₆-Alkoxy-C₂-C₄-alkyl oder C₁-C₆-Alkylthio-C₂-C₄-alkyl, für gegebenenfalls durch Fluor, C₁-C₄-Alkyl, C₁-C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₄-Alkyl, C₁-C₂-Halogenalkyl, C₁-C₄-Alkoxy oder C₁-C₂-Halogenalkoxy substituiertes Phenyl, oder Phenyl-C₁-C₄-alkyl oder
- A und D: stehen gemeinsam besonders bevorzugt für gegebenenfalls substituiertes C₃-C₅-Alkandiyl, in welchem gegebenenfalls eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
- A und D: stehen gemeinsam besonders bevorzugt mit den Atomen, an die sie gebunden sind, für eine der Gruppen AD-1 bis AD-10: 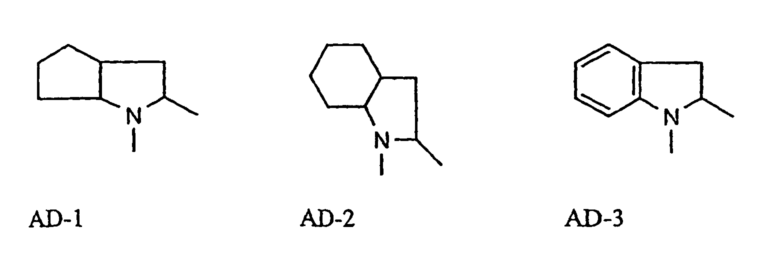 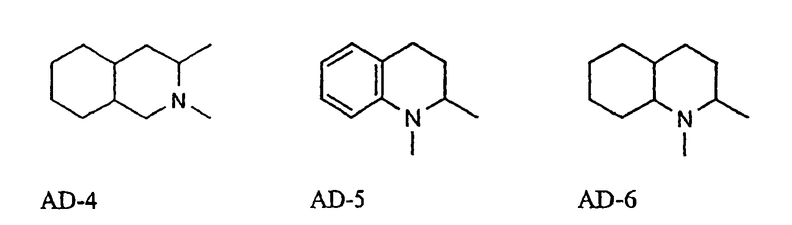 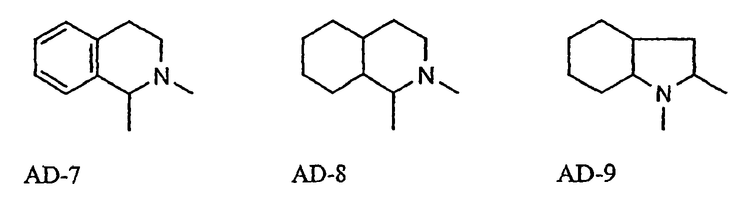 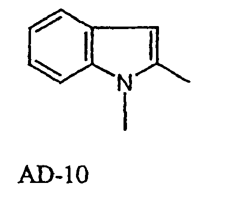
- G: steht besonders bevorzugt für Wasserstoff (a) oder für eine der Gruppen 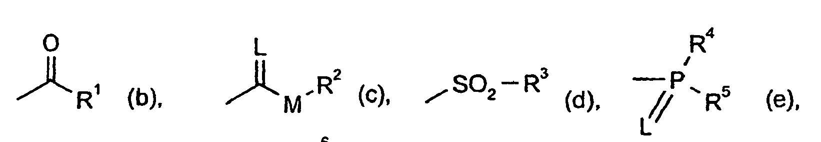 E (f) oder 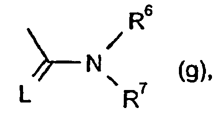 insbesondere für (a), (b), (c) oder (g),
in welchen
E für ein Metallionäquivalent oder ein Ammoniumion steht,
L für Sauerstoff oder Schwefel steht und
M für Sauerstoff oder Schwefel steht,
R¹ steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom, C₁-C₄-Alkyl, Trifluormethyl oder C₁-C₂-Alkoxy substituiertes Pyrazolyl, Thiazolyl, Pyridyl, Pyrimidyl, Furanyl oder Thienyl.
R² steht besonders bevorzugt für jeweils gegebenenfalls durch Fluor substituiertes C₁-C₁₆-Alkyl, C₂-C₁₆-Alkenyl oder C₁-C₆-Alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₂-Halogenalkyl oder C₁-C₂-Halogenalkoxy substituiertes Phenyl oder Benzyl.
R³ steht besonders bevorzugt für gegebenenfalls durch Fluor substituiertes C₁-C₆-Alkyl oder für gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl.
R⁴ steht besonders bevorzugt für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
R⁵ steht besonders bevorzugt für C₁-C₆-Alkoxy oder C₁-C₆-Alkylthio.
R⁶ steht besonders bevorzugt für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl,
R⁷ steht besonders bevorzugt für Wasserstoff, C₁-C₆-Alkyl, C₃-C₆-Alkenyl, oder
R⁶ und R⁷ stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor, Brom und Iod, insbesondere für Fluor, Chlor und Brom.
- Het: steht ganz besonders bevorzugt für 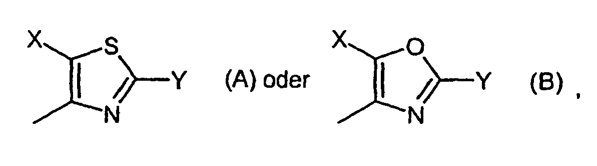
X steht ganz besonders bevorzugt für Wasserstoff, Chlor, Brom, Methyl, Ethyl, n-Propyl, iso-Propyl, n-Butyl oder iso-Butyl,
Y steht ganz besonders bevorzugt für Methyl, Ethyl, n-Propyl, i-Propyl, n-Butyl, i-Butyl, tert.-Butyl, Trifluormethyl oder für die Gruppe 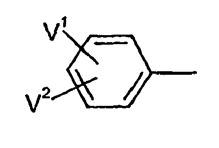
oder im Falle von Het = Thiazolyl ((I-1-A) bis (I-3-A)) auch für 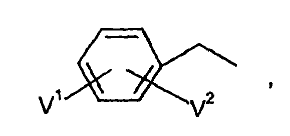
V¹ steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano, Phenoxy oder gegebenenfalls einfach oder zweifach durch Fluor, Chlor, Methyl, Methoxy, Trifluormethyl oder Trifluormethoxy substituiertes Phenyl,
V² steht ganz besonders bevorzugt für Wasserstoff, Fluor, Chlor, Methyl, Ethyl, n-Propyl, iso-Propyl, Methoxy, Ethoxy, Trifluormethyl oder Trifluormethoxy oder
V¹ und V² stehen zusammen ganz besonders bevorzugt für -O-CH₂-O-, -O-CF₂-O-oder -O-CF₂-CF₂-O-,
W steht ganz besonders bevorzugt für N-D ⁽¹⁾, Sauerstoff⁽²⁾ oder Schwefel ⁽³⁾,
A steht ganz besonders bevorzugt für Wasserstoff, für jeweils gegebenenfalls durch Fluor substituiertes C₁-C₈-Alkyl oder C₁-C₄-Alkoxy-C₁-C₂-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder für jeweils gegebenenfalls durch Fluor, Chlor, Brom, Methyl, Ethyl, n-Propyl, iso-Propyl, tert.-Butyl, Methoxy, Ethoxy, Trifluormethyl, Trifluormethoxy, Cyano oder Nitro substituiertes Phenyl oder Benzyl,
B steht ganz besonders bevorzugt für Wasserstoff, Methyl oder Ethyl oder
A, B und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und welches gegebenenfalls einfach oder zweifach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy oder iso-Butoxy substituiert ist oder
A, B und das Kohlenstoffatom, an das sie gebunden sind, stehen ganz besonders bevorzugt für C₆-Cycloalkyl, welches durch eine gegebenenfalls durch Methyl oder Ethyl einfach bis zweifach substituierte Alkylendioxyl-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
D steht ganz besonders bevorzugt für Wasserstoff, für jeweils gegebenenfalls durch Fluor substituiertes C₁-C₆-Alhyl, C₃-C₄-Alkenyl, C₁-C₄-Alkoxy-C₂-C₃-alkyl, C₁-C₄-Alkylthio-C₂-C₃-alkyl oder C₃-C₆-Cycloall-yl, in welchem gegebenenfalls eine Methylengruppe durch Sauerstoff oder Schwefel ersetzt ist oder für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl oder Benzyl,
oder
A und D stehen gemeinsam ganz besonders bevorzugt für gegebenenfalls substituiertes C₃-C₄-Alkandiyl, worin gegebenenfalls ein Kohlenstoffatom durch Sauerstoff oder Schwefel ersetzt ist und welches gegebenenfalls durch Methyl substituiert ist oder
A und D stehen gemeinsam ganz besonders bevorzugt mit den Atomen, an die sie gebunden sind, für eine der folgenden Gruppen AD: 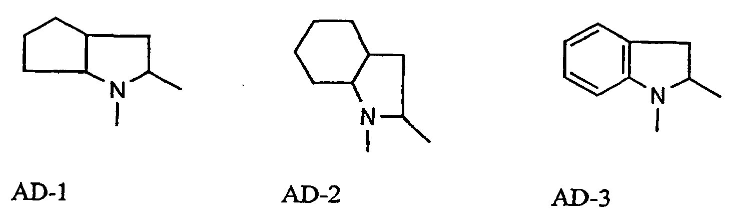 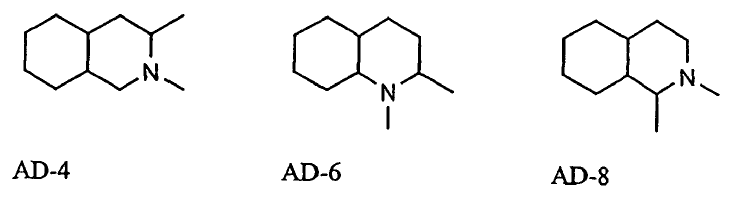 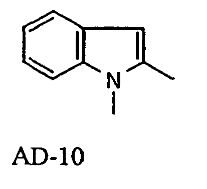
G steht ganz besonders bevorzugt für Wasserstoff (a) oder für eine der Gruppen 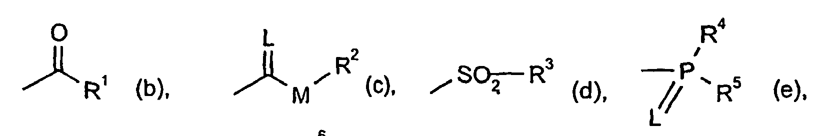 E (f) oder 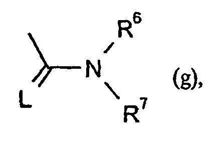 insbesondere für (a), (b) oder (c),
in welchen
E für ein Metallionäquivalent oder ein Ammoniumion, steht,
L für Sauerstoff oder Schwefel steht und
M für Sauerstoff oder Schwefel steht,
R¹ steht ganz besonders bevorzugt für jeweils gegebenenfalls durch Fluor substituiertes C₁-C₁₄-Alkyl, C₂-C₁₄-Alkenyl, C₁-C₄-Alkoxy-C₁-C₂-alkyl, C₁-C₄-Alkylthio-C₁-C₂-alkyl oder jeweils gegebenenfalls durch Fluor, Chlor, Methyl, Ethyl oder Methoxy substituiertes Cyclopropyl, Cyclopentyl oder Cyclohexyl,
für gegebenenfalls einfach bis zweifach durch Fluor, Chlor, Brom, Cyano, Nitro, Methyl, Ethyl, tert.-Butyl, Methoxy, Ethoxy, Trifluormethyl oder Trifluormethoxy substituiertes Phenyl,
für jeweils gegebenenfalls einfach durch Fluor, Chlor, Brom oder Methyl substituiertes Thienyl oder Pyridyl,
R² steht ganz besonders bevorzugt für jeweils gegebenenfalls durch Fluor substituiertes C₁-C₈-Alkyl, C₂-C₈-Alkenyl oder C₁-C₄-Alkoxy-C₂-C₃-alkyl,
für gegebenenfalls durch Fluor, Chlor, Methyl, Ethyl, n-Propyl, iso-Propyl oder Methoxy substituiertes Cyclohexyl,
oder für jeweils gegebenenfalls durch Fluor, Chlor, Cyano, Nitro, Methyl, Ethyl, Methoxy, Trifluormethyl oder Trifluormethoxy substituiertes Phenyl oder Benzyl,
- R³: steht ganz besonders bevorzugt für Methyl, Ethyl, n-Propyl oder gegebenenfalls durch Fluor, Chlor, Brom, Methyl, tert.-Butyl, Methoxy, Trifluormethyl, Trifluormethoxy, Cyano oder Nitro einfach substituiertes Phenyl,
- R⁴: steht ganz besonders bevorzugt für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio,
- R⁵: steht ganz besonders bevorzugt für Methoxy, Ethoxy, Methylthio oder Ethylthio,
- R⁶: steht ganz besonders bevorzugt für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, Trifluormethyl, Methyl oder Methoxy substituiertes Phenyl, für gegebenenfalls durch Fluor, Chlor, Brom, Methyl, Trifluormethyl oder Methoxy substituiertes Benzyl,
- R⁷: steht ganz besonders bevorzugt für Wasserstoff, Methyl, Ethyl, Propyl oder Allyl oder
- R⁶ und R⁷: stehen ganz besonders bevorzugt zusammen für einen C₅-C₆-Alkylenrest, in welchem gegebenenfalls eine Methylengruppe durch Sauerstoff oder Schwefel ersetzt ist.
- Het: steht hervorgehoben für 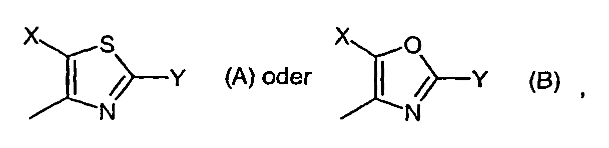
X steht hervorgehoben für Wasserstoff, Chlor, Brom, Methyl, Ethyl, n-Propyl, oder iso-Propyl,
Y steht hervorgehoben für Methyl, Ethyl, n-Propyl, i-Propyl, n-Butyl, i-Butyl, tert.-Butyl, Trifluormethyl oder für die Gruppe 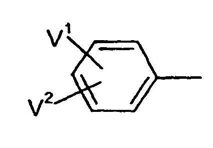
oder im Fall von Het = Thiazolyl ((I-1-A) bis (I-3-A)) auch für 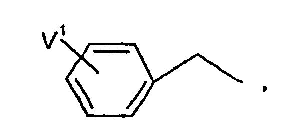
V¹ steht hervorgehoben für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Phenoxy, für gegebenenfalls durch Chlor oder Trifluormethyl substituiertes Phenyl,
V² steht hervorgehoben für Wasserstoff, Fluor, Chlor, Methyl, Ethyl, Methoxy oder Trifluormethyl oder
V¹ und V² stehen zusammen hervorgehoben für -O-CH₂-O- oder -O-CF₂-O-,
W steht hervorgehoben für N-D ⁽¹⁾, Sauerstoff⁽²⁾ oder Schwefel ⁽³⁾,
A steht hervorgehoben für Wasserstoff oder C₁-C₄-Alkyl oder Cyclopropyl,
B steht hervorgehoben für Wasserstoff oder Methyl oder
A, B und das Kohlenstoffatom, an das sie gebunden sind, stehen hervorgehoben für gesättigtes C₅-C₆-Cycloalkyl, in welchem gegebenenfalls ein Ringglied durch Sauerstoff oder Schwefel ersetzt ist und welches gegebenenfalls einfach oder zweifach durch Methyl, Ethyl, Propyl, Isopropyl, Trifluormethyl, Methoxy, Ethoxy, Propoxy, Butoxy oder iso-Butoxy substituiert ist oder für C₅-C₆-Cycloalkyl, worin zwei nicht direkt benachbarte Kohlenstoffatome einen weiteren fünfgliedrigen Ring bilden,
D steht hervorgehoben für Wasserstoff,
D steht hervorgehoben auch für i-Propyl,
A und D stehen zusammen mit den Atomen, an die sie gebunden sind, hervorgehoben für Cyclohexyl, in welchem ein Ringatom durch Schwefel ersetzt sein kann,
G steht hervorgehoben für Wasserstoff (a) oder im Fall von Het = Thiazolyl (I-1-A) bis (I-3-A) für eine der Gruppen 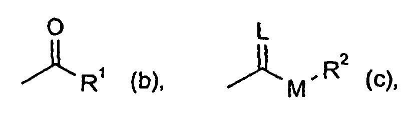
in welchen
L für Sauerstoff steht und
M für Sauerstoff steht,
R¹ steht hervorgehoben für jeweils gegebenenfalls durch Fluor oder Chlor substituiertes C₁-C₄-Alkyl, C₂-C₄-Alkenyl, C₁-C₄-Alkoxy-C₁-C₂-alkyl, C₁-C₄-Alkylthio-C₁-C₂-alkyl oder gegebenenfalls durch Fluor, Chlor, Methyl, Ethyl oder Methoxy substituiertes Cyclopropyl oder Cyclohexyl,
für gegebenenfalls einfach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl,
für jeweils gegebenenfalls einfach durch Chlor oder Methyl substituiertes Thienyl oder Pyridyl,
R² steht hervorgehoben für jeweils gegebenenfalls durch Fluor substituiertes C₁-C₄-Alkyl, C₂-C₄-Alkenyl oder C₁-C₄-Alkoxy-C₂-C₃-alkyl,
für gegebenenfalls durch Methyl, Ethyl, n-Propyl, iso-Propyl oder Methoxy substituiertes Cyclohexyl,
oder für jeweils gegebenenfalls einfach durch Fluor, Chlor, Cyano, Nitro, Methyl, Ethyl, Methoxy, Trifluormethyl oder Trifluormethoxy substituiertes Phenyl oder Benzyl,
Het steht insbesondere bevorzugt für 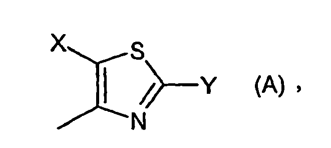
X steht insbesondere bevorzugt für Methyl, Ethyl, n-Propyl, i-Propyl, Chlor oder Brom,
Y steht insbesondere bevorzugt für Methyl, Ethyl, n-Propyl oder i-Propyl oder für die Gruppen 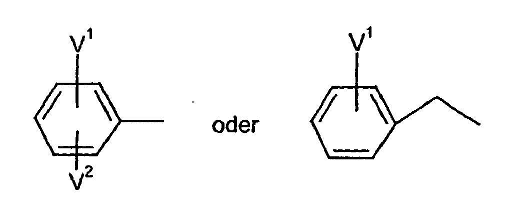
in welcher
V¹ insbesondere bevorzugt für Wasserstoff, Brom, Chlor, Methyl, Trifluormethyl, Phenoxy oder t-Butyl oder für gegebenenfalls durch Chlor oder Trifluormethyl substituiertes Phenyl steht,
V² insbesondere bevorzugt für Wasserstoff, Chlor, Fluor oder Methoxy steht oder
V¹ und V² zusammen insbesondere bevorzugt für -O-CH₂-O- oder -O-CF₂-O-stehen.
W steht insbesondere bevorzugt für N-D ⁽¹⁾, Sauerstoff ⁽²⁾ oder Schwefel ⁽³⁾,
D steht insbesondere bevorzugt für Wasserstoff oder i-Propyl (speziell für Wasserstoff),
A steht insbesondere bevorzugt für Methyl, Ethyl, n- oder i-Propyl oder Cyclopropyl oder Wasserstoff (speziell für Methyl),
B steht insbesondere bevorzugt für Wasserstoff oder Methyl oder
A und B und das Kohlenstoffatom, an das sie gebunden sind, stehen insbesondere bevorzugt für Cyclohexyl, in welchem gegebenenfalls ein Ringatom durch Sauerstoff ersetzt ist und welches gegebenenfalls einfach oder zweifach durch Methyl, Ethyl, Methoxy oder Ethoxy substituiert ist oder für Cyclohexyl, worin zwei nicht direkt benachbarte Kohlenstoffatome einen weiteren 5-gliedrigen C-Ring bilden,
A und D stehen insbesondere bevorzugt zusammen mit den Atomen, an die sie gebunden sind, für Cyclohexyl, in welchem ein Ringatom durch Schwefel ersetzt sein kann,
G steht insbesondere bevorzugt für Wasserstoff (a) oder für eine der Gruppen 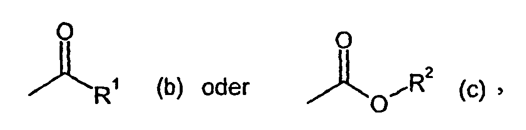
worin
R¹ insbesondere bevorzugt für Methyl, Ethyl, n-Propyl oder i-Propyl oder für jeweils gegebenenfalls durch Chlor substituiertes Phenyl oder Pyridyl steht,
R² insbesondere bevorzugt für Methyl, Ethyl, Phenyl, Benzyl, n- oder i-Propyl steht.
Het steht insbesondere bevorzugt für 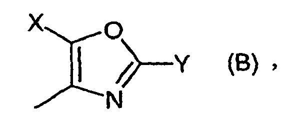
X steht insbesondere bevorzugt für Methyl oder Ethyl,
Y steht insbesondere bevorzugt für gegebenenfalls durch Chlor substituiertes Phenyl,
A steht insbesondere bevorzugt für Methyl, Ethyl, n- oder i-Propyl,
B steht insbesondere bevorzugt für Methyl, oder
A und B und das Kohlenstoffatom, an das sie gebunden sind, stehen gemeinsam insbesondere bevorzugt für Cyclohexyl, welches gegebenenfalls durch Methoxy oder Methyl substituiert ist,
W steht insbesondere bevorzugt für N-D oder Sauerstoff,
D steht insbesondere bevorzugt für Wasserstoff,
G steht insbesondere bevorzugt für Wasserstoff.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien beispielsweise außer den bei den Herstellungsbeispielen genannten Verbindungen die folgenden Verbindungen der Formel (I-1-a) genannt. 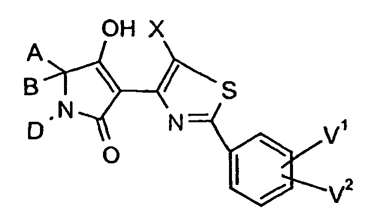
X = CH₃, V¹ = 3-Cl, V² = H.

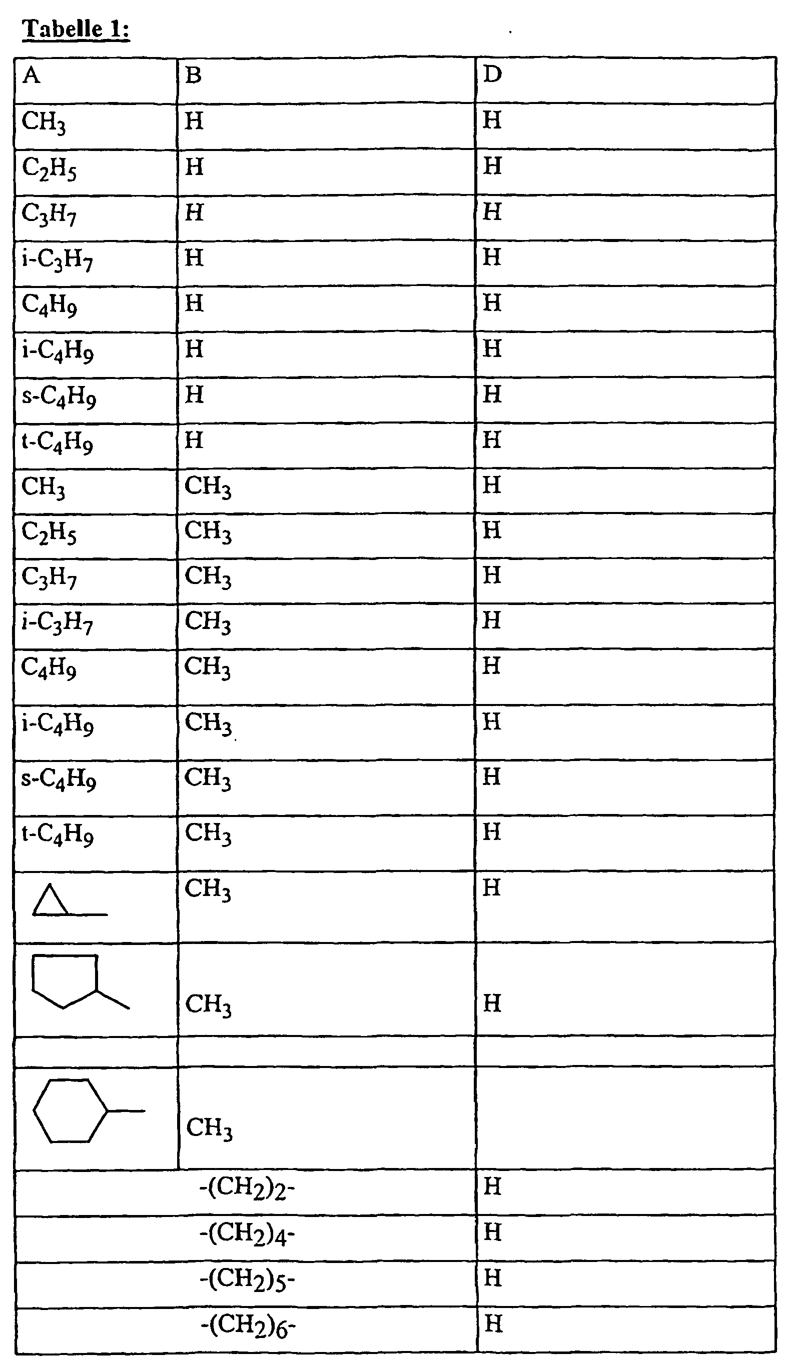

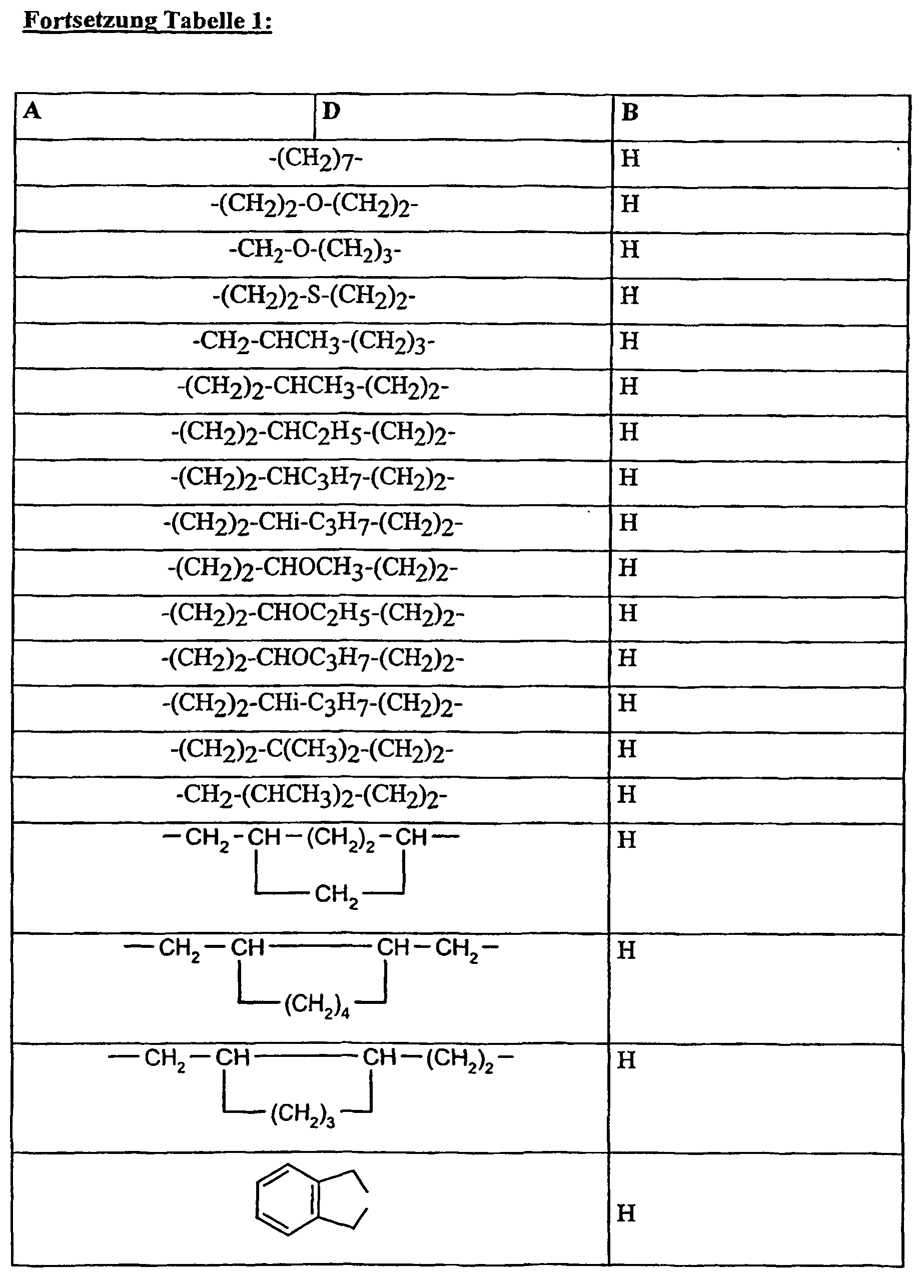

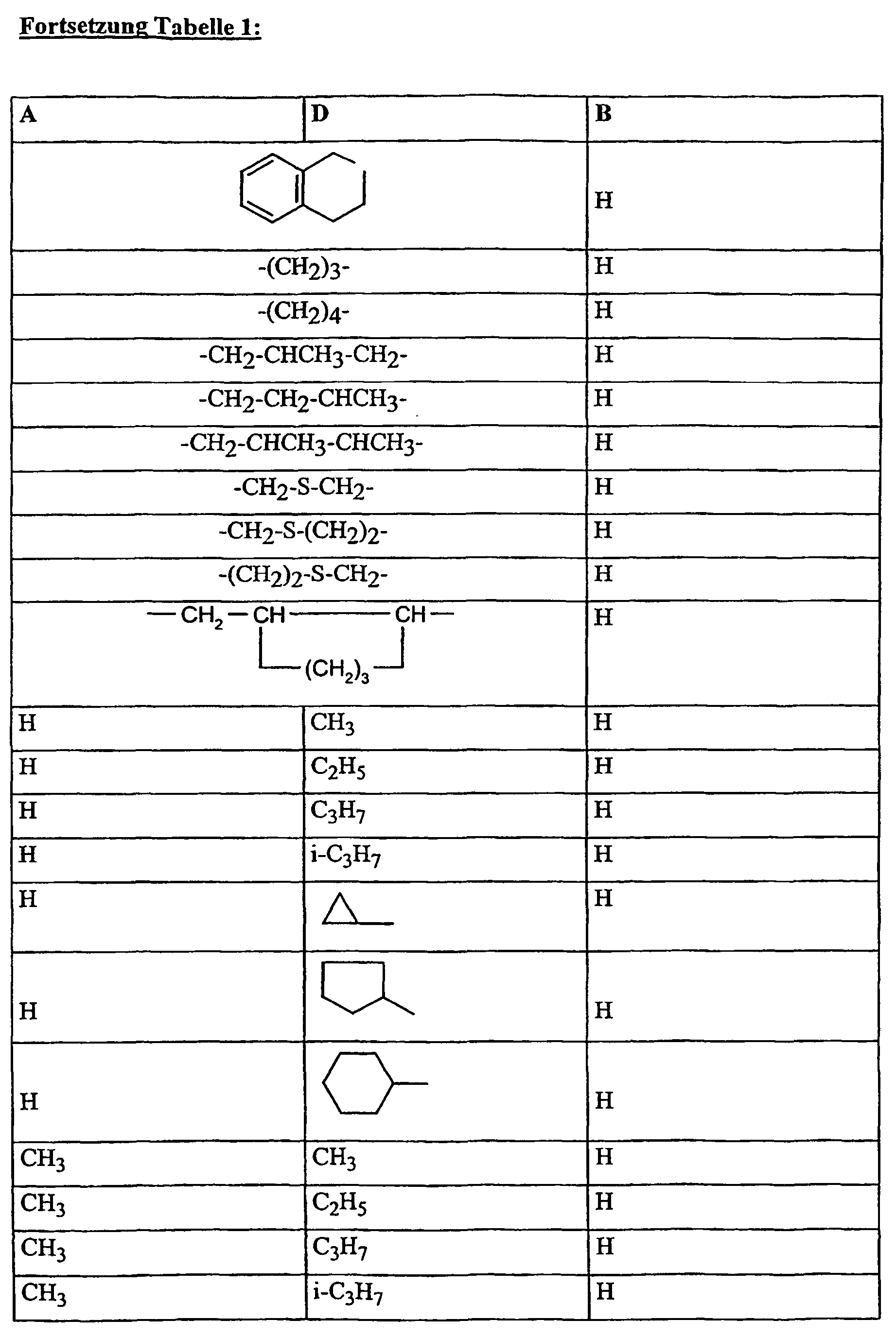

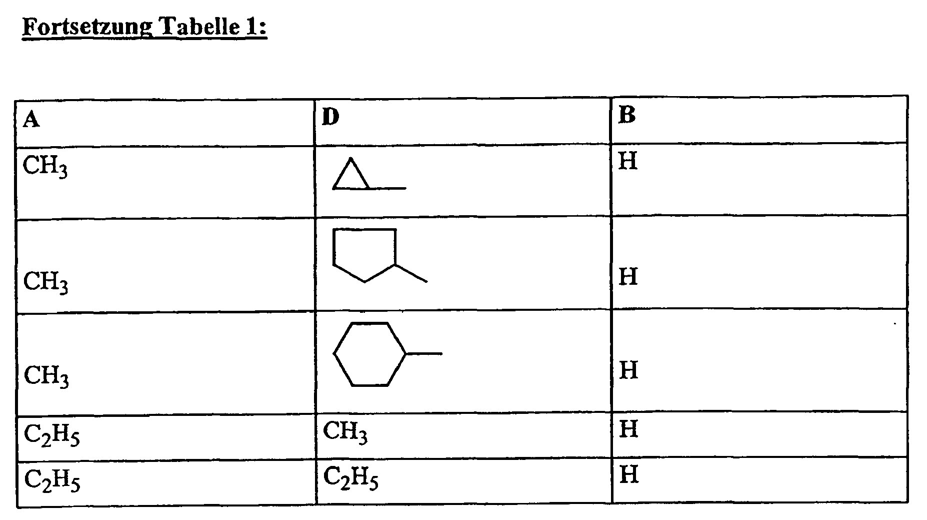

- **Tabelle 2:**: A, B und D wie in Tabelle 1 angegeben
X = CH₃; V¹ = 4-Cl; V² = H.
- **Tabelle 3**:: A, B und D wie in Tabelle 1 angegeben
X = CH₃; V¹ = 3-CF₃; V² = H.
- **Tabelle 4**:: A, B und D wie in Tabelle 1 angegeben
X = CH₃; V¹ = 4-CF₃; V² = H.
- **Tabelle 5**:: A, B und D wie in Tabelle 1 angegeben
X = CH₃; V¹ = 4-Cl; V² = 2-Cl.
- **Tabelle 6**:: A, B und D wie in Tabelle 1 angegeben
X = CH₃; V¹ = 4-Cl; V² = 3-Cl.
- **Tabelle 7**:: A, B und D wie in Tabelle 1 angegeben
X = CH₃; V¹ = 4-CH₃; V² = H.

- **Tabelle 8**:: A, B und D wie in Tabelle 1 angegeben
X = CH₃; V¹ = 2-Cl; V² = 6-OCH₃.
- **Tabelle 9**:: A, B und D wie in Tabelle 1 angegeben
X = C₂H₅; V¹ = 4-Cl; V² = H.
- **Tabelle 10**:: A, B und D wie in Tabelle 1 angegeben
X = C₃H₇; V¹ = 4-Cl; V² = H.
- **Tabelle 11**:: A, B und D wie in Tabelle 1 angegeben
X = C₂H₅; V¹ = 3-Cl; V² = H
- **Tabelle 12**:: A, B und D wie in Tabelle 1 angegeben
X = C₃H₇; V¹= 3-Cl; V² = H.
- **Tabelle 13**:: A, B und D wie in Tabelle 1 angegeben
X = C₂H₅; V¹ = 4-CF₃; V² = H.
- **Tabelle 14**:: A, B und D wie in Tabelle 1 angegeben
X = C₂H₅; V¹ = 3-CF₃; V² = H.
- **Tabelle 15**:: A, B und D wie in Tabelle 1 angegeben
X = C₃H₇; V¹ = 4-CF₃; V² = H.
- **Tabelle 16**:: A, B und D wie in Tabelle 1 angegeben
X = C₃H₇; V¹ = 3-CF₃; V² = H.
- **Tabelle 17**:: A, B und D wie in Tabelle 1 angegeben
X = Cl; V¹ = 4-Cl; V² = H.

- **Tabelle 18**:: A, B und D wie in Tabelle 1 angegeben
X = Br; V¹ = 4-Cl; V² = H.
- **Tabelle 19**:: A, B und D wie in Tabelle 1 angegeben
X = C₂H₅; V¹ = 3-Cl; V² = 4-Cl.
- **Tabelle 20**:: A, B und D wie in Tabelle 1 angegeben
X = C₃H₇; V¹ = 3-Cl; V² = 4-Cl.
- **Tabelle 21**:: A, B und D wie in Tabelle 1 angegeben
X = CH₃; V¹ = 4-Br; V² = H.

Im einzelnen seien außer den bei den Herstellungsbeispielen genannten Verbindungen die folgenden Verbindungen der Formel (I-2-a) genannt:

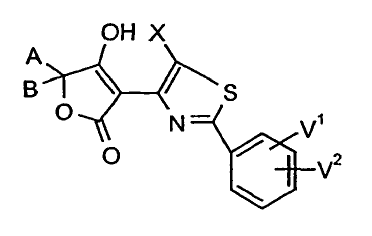

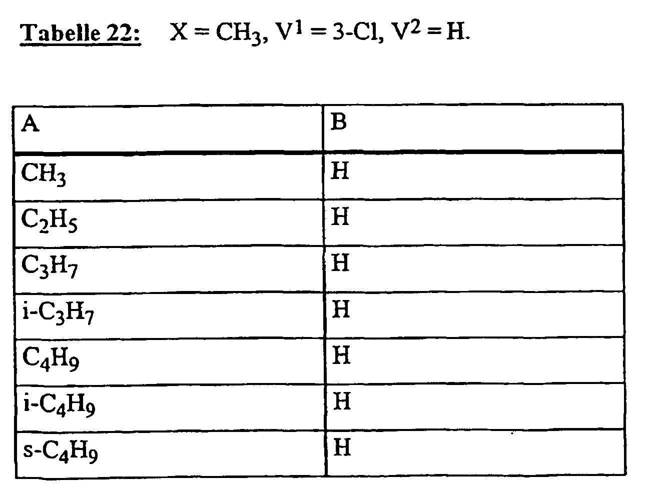

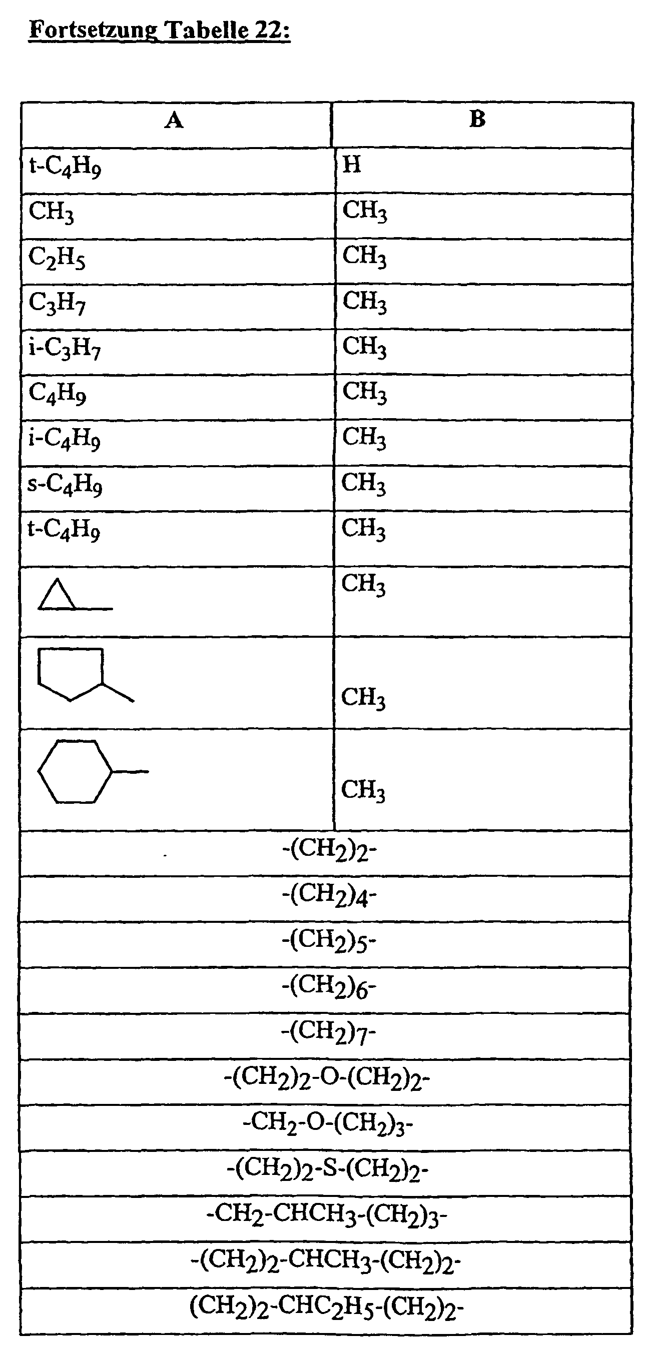

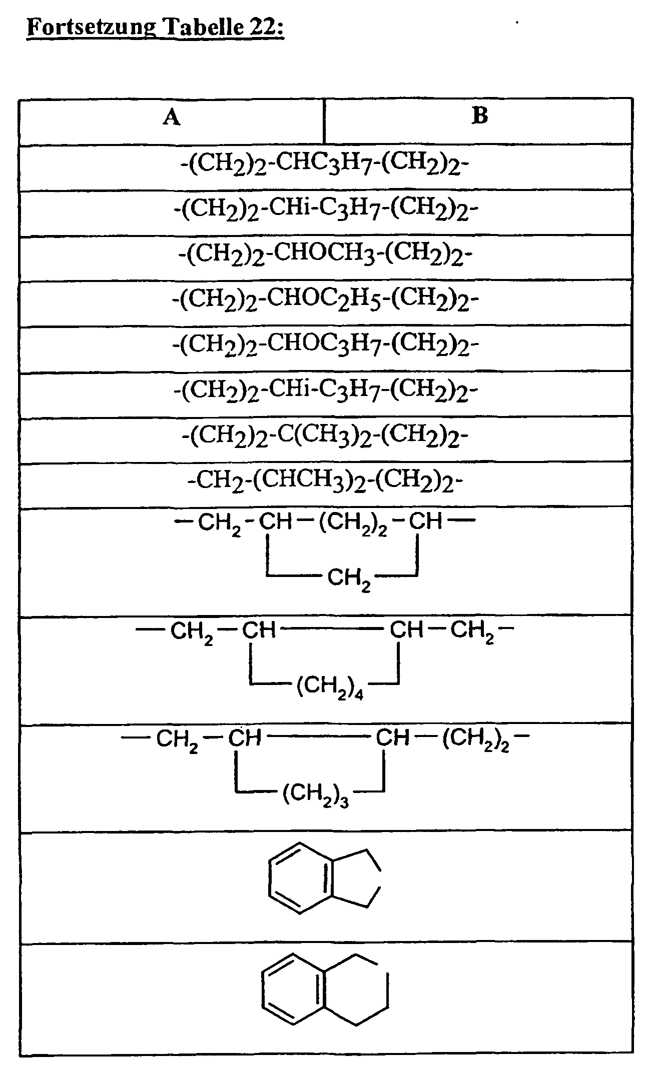

- **Tabelle 23:**: A und B wie in Tabelle 22 angegeben
X = CH₃; V¹ = 4-Cl; V² = H.
- **Tabelle 24**:: A und B wie in Tabelle 22 angegeben
X = CH₃; V¹ = 3-CF₃; V² =H.

- **Tabelle 25**:: A und B wie in Tabelle 22 angegeben
X = CH₃; V¹ = 4-CF₃; V² = H.
- **Tabelle 26**:: A und B wie in Tabelle 22 angegeben
X = CH₃; V¹ = 4-Cl; V² = 2-Cl.
- **Tabelle 27**:: A und B wie in Tabelle 22 angegeben
X = CH₃; V¹ = 4-Cl; V² = 3-Cl.
- **Tabelle 28**:: A und B wie in Tabelle 22 angegeben
X = CH₃; V¹ = 4-CH₃; V² = H.
- **Tabelle 29**:: A und B wie in Tabelle 22 angegeben
X = CH₃; V¹ = 2-Cl; V² = 6-OCH₃.
- **Tabelle 30**:: A und B wie in Tabelle 22 angegeben
X = C₂H₅; V¹ = 4-Cl; V² = H.
- **Tabelle 31:**: A und B wie in Tabelle 22 angegeben
X = C₃H₇; V¹ = 4-Cl; V² = H.
- **Tabelle 32**:: A und B wie in Tabelle 22 angegeben
X = C₂H₅; V¹ = 3-Cl; V² = H.
- **Tabelle 33**:: A und B wie in Tabelle 22 angegeben
X = C₃H₇; V¹ = 3-Cl V² = H.
- **Tabelle 34**:: A und B wie in Tabelle 22 angegeben
X = C₂H₅; V¹ = 4-CF₃; V² = H

- **Tabelle 35**:: A und B wie in Tabelle 22 angegeben
X = C₃H₇; V¹ = 4-CF₃; V² = H.
- **Tabelle 36**:: A und B wie in Tabelle 22 angegeben
X = C₂H₅; V¹ = 3-CF₃; V¹ = H.
- **Tabelle 37**:: A und B wie in Tabelle 22 angegeben
X = C₃H₇; V¹ = 3-CF₃; V² = H.
- **Tabelle 38**:: A und B wie in Tabelle 22 angegeben
X = Cl; V¹ = 4-Cl; V² = H.
- **Tabelle 39**:: A und B wie in Tabelle 22 angegeben
X = Br; V¹ = 4-Cl; V² = H.
- **Tabelle 40**:: A und B wie in Tabelle 22 angegeben
X = C₂H₅; V¹ = 3-Cl; V² = 4-Cl.
- **Tabelle 41**:: A und B wie in Tabelle 22 angegeben
X = C₃H₇; V¹ = 3-Cl; V² = 4-Cl.
- **Tabelle 42**:: A und B wie in Tabelle 22 angegeben
X = CH₃; V¹ = 4-Br; V² = H.

Verwendet man gemäß Verfahren (A) N-[4-(5-Methyl-2-phenyl)-thiazolylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

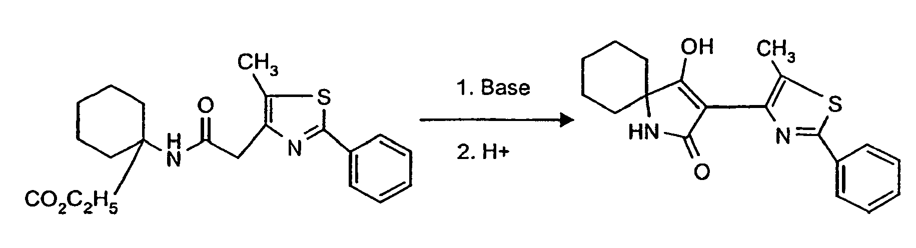

Verwendet man gemäß Verfahren (B) O-[4-(5-Methyl-2-(4-chlor)-phenyl)-thiazolyl acetyl]-2-hydroxyisobuttersäureethylester, so kann der Verlauf des erfindungsgemäßen Verfahrens durch folgendes Reaktionsschema wiedergegeben werden:

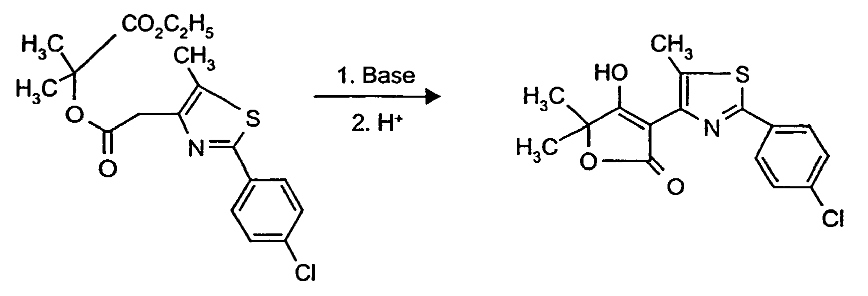

Verwendet man gemäß Verfahren (C) 2-[4-(5-Methyl-2-phenyl)-thiazolyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

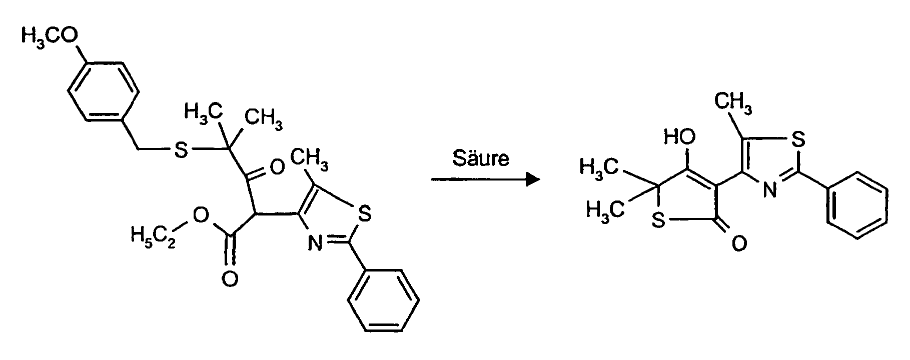

Verwendet man gemäß Verfahren (Dα) 3-[4-(5-Methyl-2-(3-chlor-phenyl)-thiazolyl]-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

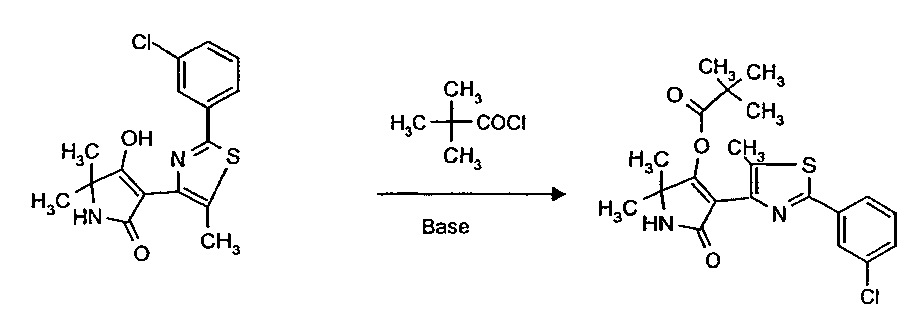

Verwendet man gemäß Verfahren (Dβ) 3-[4-(5-Ethyl-2-(4-methoxy-phenyl))-thiazolyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

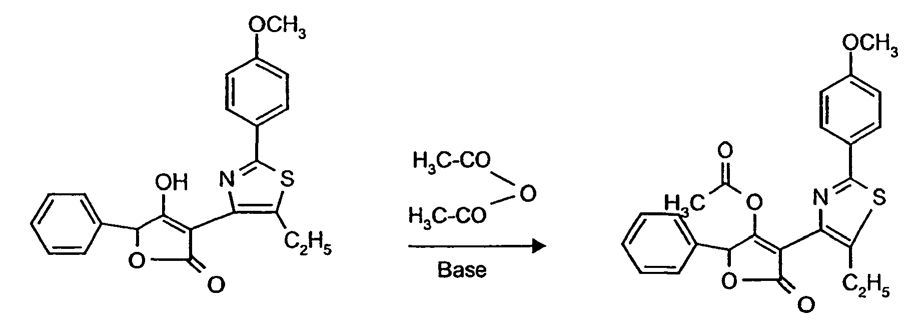

Verwendet man gemäß Verfahren (E) 3-[4-(5-Methyl-2-phenyl)-thiazolyl]-5,5-dimethylpyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

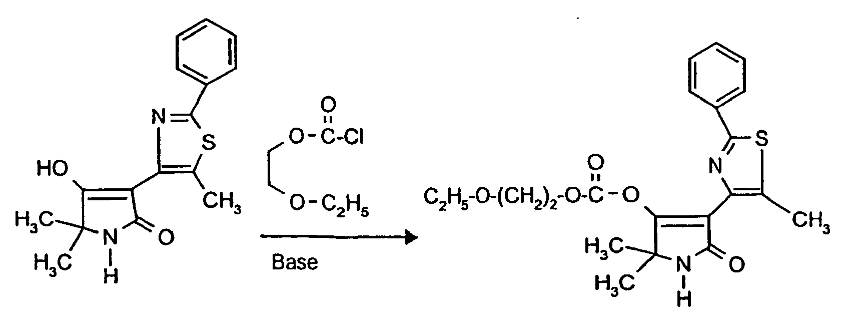

Verwendet man gemäß Verfahren (F), 3-[4-(5-Methyl-2-(4-fluor-phenyl))-thiazolyl]-4-hydroxy-5,5-dimethyl-Δ³-dihydrofuran-2-on und Chlormonothioameisensäuremethylester als Ausgangsprodukte, so kann der Reaktionsverlauf folgendermaßen wiedergegeben werden:

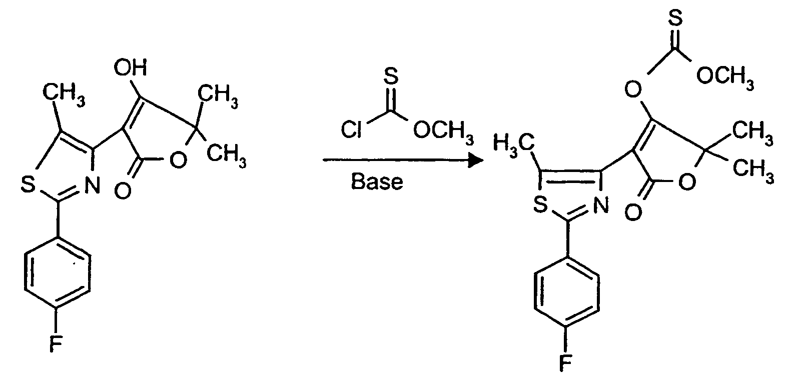

Verwendet man gemäß Verfahren (G) 3-[4-(5-Methyl-3-(4-methyl-phenyl))-thiazolyl]-5,5-pentamethylen-pyrrolidin-2,4-dion und Methansulfonsäurechlorid als Ausgangsprodukte, so kann der Reaktionsverlauf durch folgendes Reaktionsschema wiedergegeben werden:

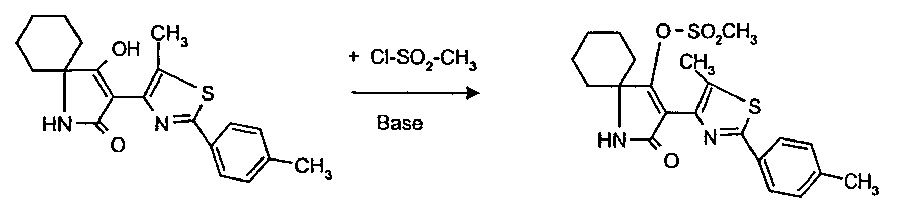

Verwendet man gemäß Verfahren (H) 3-[4-(5-Methyl-2-phenyl)-thiazolyl]-4-hydroxy-5,5-dimethyl-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

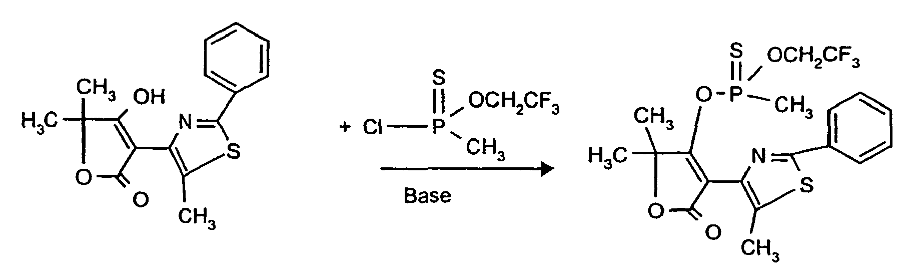

Verwendet man gemäß Verfahren (I) 3-[4-(5-Methyl-2-(4-trifluormethyl-phenyl))-thiazolyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

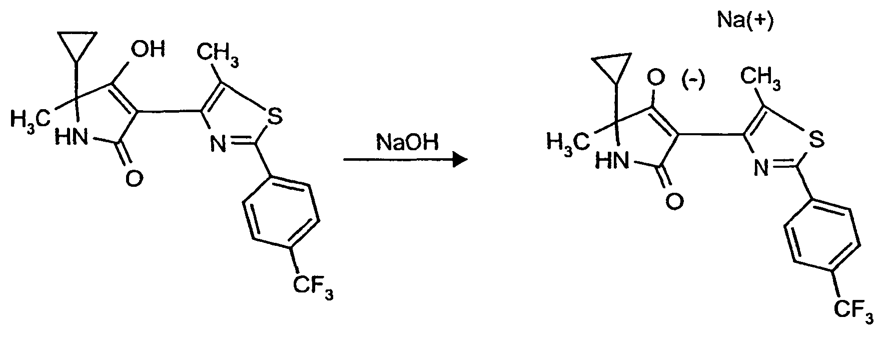

Verwendet man gemäß Verfahren (Jα) 3-[4-(5-Methyl-2-(3-trifluormethyl-phenyl))-thiazolyl]-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

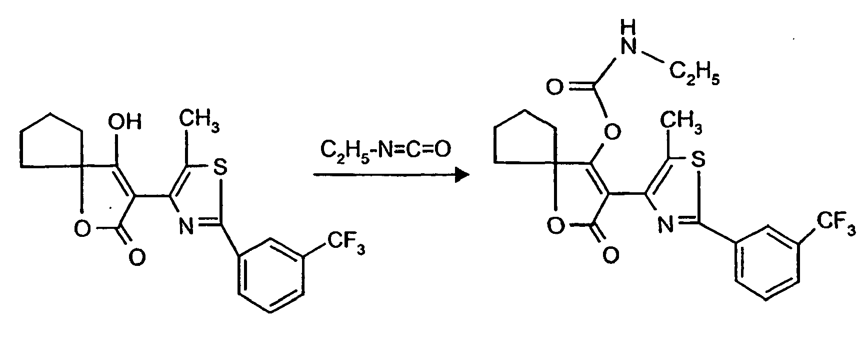

Verwendet man gemäß Verfahren (Jβ) 3-[4-(5-Methyl-2-phenyl)-thiazolyl]-5-methyl-pyrrolidin-2,4-dion und Dimethylcarbamidsäurechlorid als Ausgangsprodukte, so kann der Reaktionsverlauf durch folgendes Schema wiedergegeben werden:

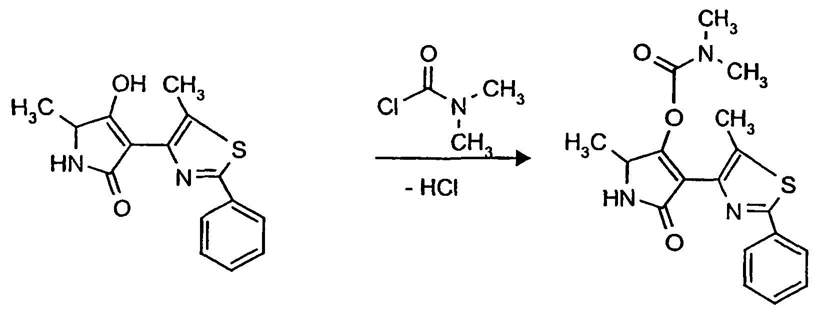

Die beim erfindungsgemäßen Verfahren (a) als Ausgangsstoffe benötigten Verbindungen der Formel (II) 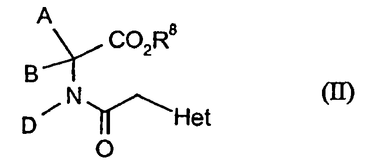
in welcher
A, B, D, Het und R⁸ die oben angegebenen Bedeutungen haben,
-

sind neu abgeschen von folgenden Verbindung 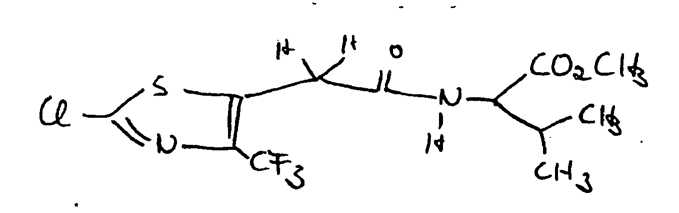 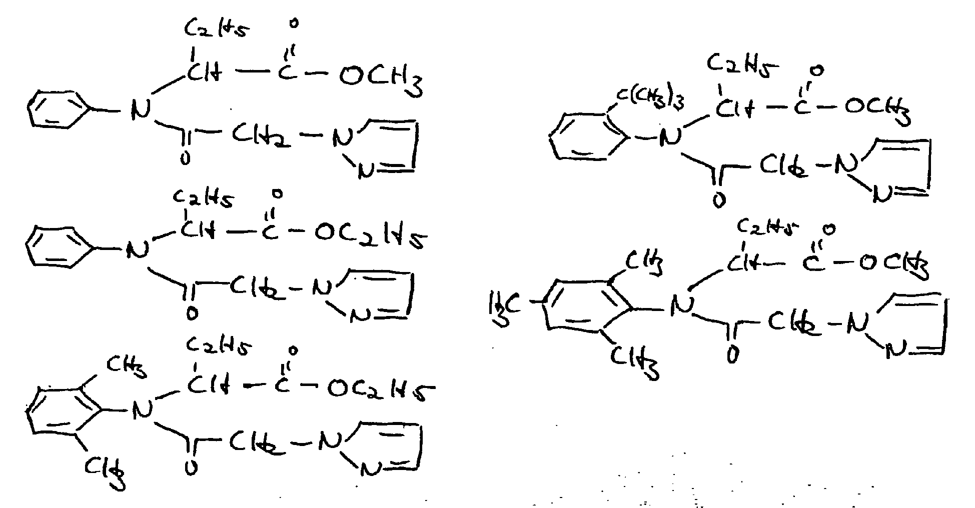 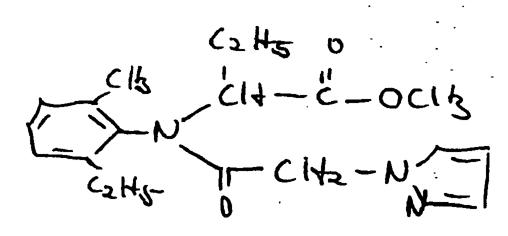 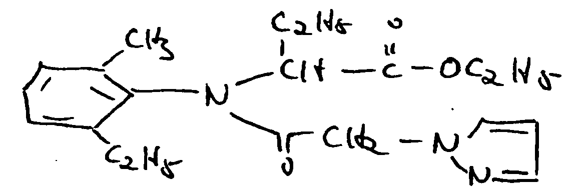 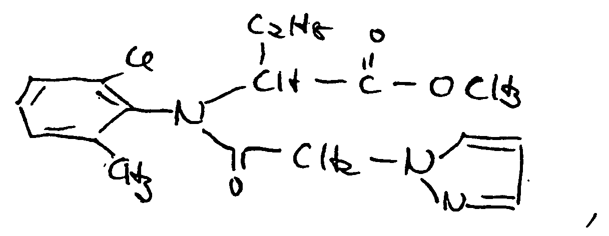
welche aus der EP- bekannt sind.

Man erhält die Acylaminosäureester der Formel (II) beispielsweise, wenn man Aminosäurederivate der Formel (XV) 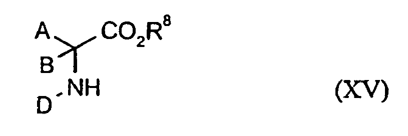
in welcher
- A, B, R⁸ und D: die oben angegebenen Bedeutungen haben,

mit substituierten Hetarylessigsäurederivaten der Formel (XVI) 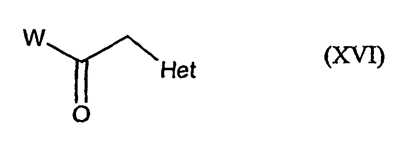
in welcher
- Het: die oben angegebene Bedeutung hat und
- W: für eine durch Carbonsäureaktivierungsreagenzien wie Carbonyldiimidazol, Carbonyldiimide (wie z.B. Dicyclohexylcarbodiimid), Phosphorylierungsreagenzien (wie z.B. POCl₃, BOP-Cl), Halogenierungsmittel z.B. Thionylchlorid, Oxalylchlorid, Phosgen oder Chlorameisensäurester eingeführte Abgangsgruppe steht

acyliert (Chem. Reviews 52, 237-416 (1953); Bhattacharya, Indian J. Chem. 6, 341-5, 1968)
oder wenn man Acylaminosäuren der Formel (XVII) 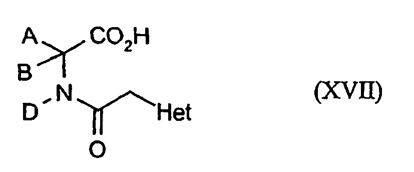
in welcher
- A, B, D und Het: die oben angegebenen Bedeutungen haben,

verestert (Chem. Ind. (London) 1568 (1968)).

Die Verbindungen der Formel (XVII) 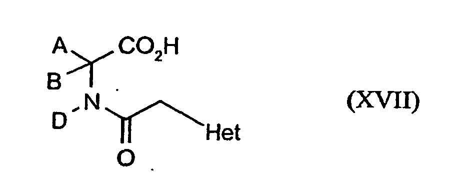
in welcher
- A, B, D und Het: die oben angegebenen Bedeutungen haben,

sind neu.

Man erhält die Verbindungen der Formel (XVII), wenn man Aminosäuren der Formel (XVIII) 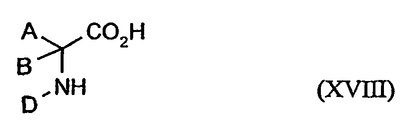
in welcher
- A, B und D: die oben angegebenen Bedeutungen haben,

mit substituierten Hetarylessigsäurederivaten der Formel (XVI) 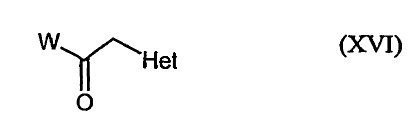
in welcher
- W und Het: die oben angegebenen Bedeutungen haben,

beispielsweise nach Schotten-Baumann acyliert (Organikum, VEB Deutscher Verlag der Wissenschaften, Berlin 1977, S. 505).

Die Verbindungen der Formel (XVI) sind teilweise neu. Sie lassen sich nach im Prinzip bekannten Verfahren darstellen (s. z.B. H. Henecka, Houben-Weyl, Methoden der Organischen Chemie, Bd. 8, S. 467-469 (1952)).

Man erhält die Verbindungen der Formel (XVI) beispielsweise, indem man substituierte Hetarylessigsäuren der Formel (XIX) 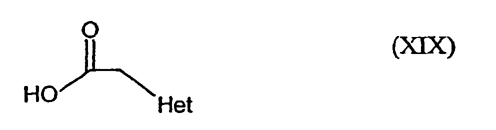
in welcher
- X und Y: die oben angegebene Bedeutung haben,

mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Hetarylessigsäuren der Formel (XIX) sind teilweise käuflich, teilweise bekannt oder lassen sich nach im Prinzip bekannten Verfahren herstellen
a) Thiazolylessigsäuren: C.S. Rooney et al. J. Med. Chem. 26, 700-714 (1983); EP-A-368 592; M.S. Malamas et al. J. Med. Chem. 39, 237-246 (1996); J. L. Collins et al. J. Med. Chem. 41, 5037-5054 (1998); NL-A-66 14 130;
b) Oxazolylessigsäuren: K. Meguro et al., Chem. Pharm. Bull, 34, 2840-2851 (1986), M. Sehi et al., Chem. Pharm. Bull. 36, 4435-4440 (1988), Malmas et al., J. Med. Chem. 39, 237-245 (1996), EP 0 177 353 A2, WO 87/03 807, WO 95/18 125, B. Hulin, J. Med. Chem. 35, 1853-1864 (1992), EP 0 389 699 A 1.

Die Verbindungen der Formel (XV) und (XVIII) sind teilweise käuflich und/oder bekannt und/oder lassen sich nach bekannten Verfahren darstellen (siehe z.B. Compagnon, Miocque Ann. Chim. (Paris) [14]5, S. 11-22, 23-27 (1970)).

Die substituierten cyclischen Aminocarbonsäuren der Formel (XVIIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 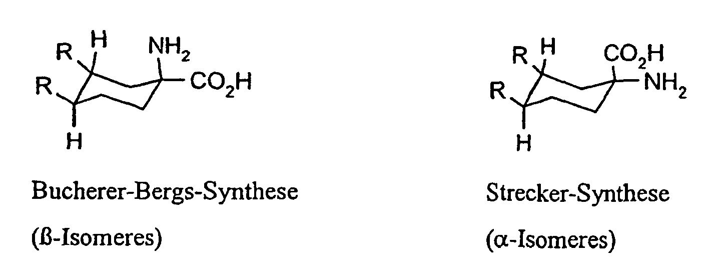
(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 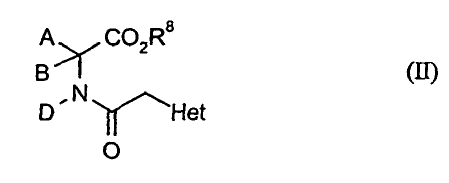
in welcher
- A, B, D, Het und R⁸: die oben angegebenen Bedeutungen haben,

herstellen, wenn man Aminonitrile der Formel (XX) 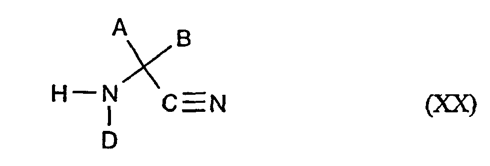
in welcher
- A, B und D: die oben angegebenen Bedeutungen haben,

mit substituierten Hetarylessigsäurederivaten der Formel (XVI) 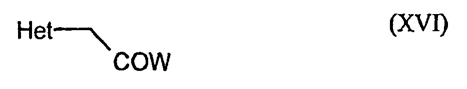
in welcher
- W und Het: die oben angegebenen Bedeutungen haben,

zu Verbindungen der Formel (XXI) 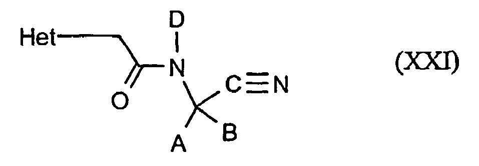
in welcher
- A, B, D und Het: die oben angegebenen Bedeutungen haben,

umsetzt,
und diese anschließend einer sauren Alkoholyse unterwirft.

Die Verbindungen der Formel (XXI) sind ebenfalls neu.

Die bei dem erfindungsgemäßen Verfahren (B) als Ausgangstoffe benötigten Verbindungen der Formel (III) 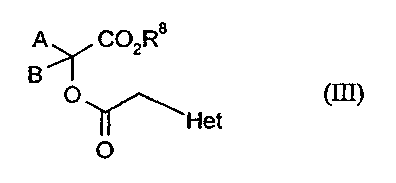
in welcher
- A, B, Het und R⁸: die oben angegebenen Bedeutungen haben,

sind neu abgeschen von der Verbindung welche au WO-A-9932464 bekannt ist.

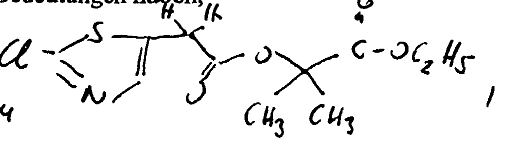

Sie lassen sich nach im Prinzip bekannten Methoden herstellen.

So erhält man die Verbindungen der Formel (III) beispielsweise, wenn man
2-Hydroxycarbonsäureester der Formel (XXII) 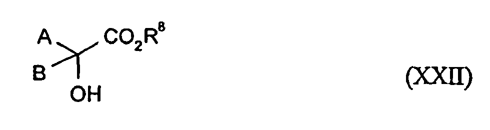
in welcher
- A, B und R⁸: die oben angegebenen Bedeutungen haben,

mit substituierten Hetarylessigsäurederivaten der Formel (XVI) 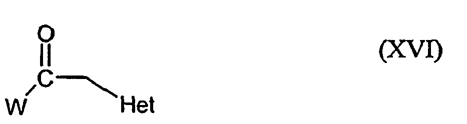
in welcher
- W und Het: die oben angegebenen Bedeutungen haben,

acyliert (Chem. Reviews 52, 237-416 (1953).

Weiterhin erhält man Verbindungen der Formel (III), wenn man
substituierte Hetarylessigsäuren der Formel (XIX) 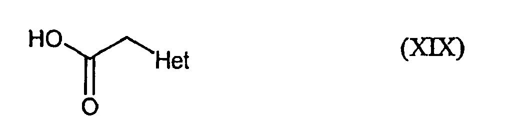
in welcher
- Het: die oben angegebenen Bedeutungen haben,

mit α-Halogencarbonsäureestern der Formel (XXIII) 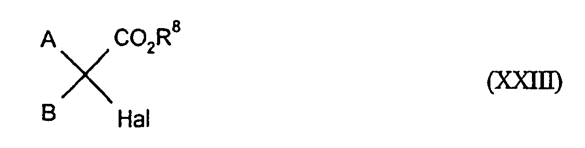
in welcher
- A, B und R⁸: die oben angegebenen Bedeutungen haben und
- Hal: für Chlor oder Brom steht,

alkyliert.

Die bei dem obigen Verfahren (C) als Ausgangsstoffe benötigten Verbindungen der Formel (IV) 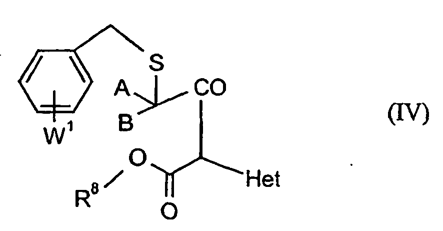
in welcher
- A, B, W¹, Het und R⁸: die oben angegebenen Bedeutungen haben, und
- W¹: für Wasserstoff, Halogen, Alkyl (bevorzugt C₁-C₆-Alkyl) oder Alkoxy (bevorzugt C₁-C₆-Alkoxy) steht,

sind neu.

Sie lassen sich nach im Prinzip bekannten Methoden herstellen.

Man erhält die Verbindungen der Formel (IV) beispielsweise, wenn man

Verbindungen der Formel (XXIV) 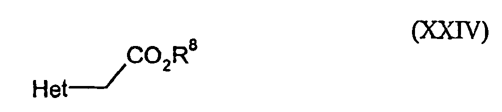
in welcher
- Het und R⁸: die oben angegebenen Bedeutungen haben,

mit 2-Benzylthio-carbonsäurehalogeniden der Formel (XXV) 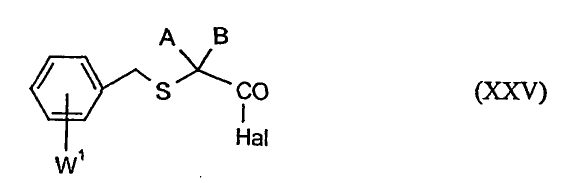
in welcher
- A, B und W¹: die oben angegebenen Bedeutungen haben und
- Hal: für Halogen (insbesondere Chlor oder Brom) steht,

in Gegenwart von starken Basen acyliert (siehe z.B. M.S. Chambers, E.J. Thomas, D.J. Williams, J. Chem. Soc. Chem. Commun., (1987), 1228).

Die Verbindungen der Formel (XXIV) sind teilweise käuflich, teilweise bekannt oder lassen sich nach bekannten Verfahren herstellen:
a) Thiazolylessigsäureester: C.S. Rooney et al., J. Med. Chem. 26, 700-714 (1983), EP-A-368 592), M.S. Malmas et al., J. Med. Chem. 39, 237-246 (1996); J. C. Collins et al., J. Med. Chem. 41, 5037-5054 (1998); NL-A-6 614 130.
b) Oxazolylessigsäuren: K. Meguro et al., Chem. Pharm. Bull, 34, 2840-2851 (1986), M. Sehi et al., Chem. Pharm. Bull. 36, 4435-4440 (1988), Malmas et al., J. Med. Chem. 39, 237-245 (1996), EP 0 177 353 A2, WO 87/03 807, WO 95/18 125, B. Hulin, J. Med. Chem. 35, 1853-1864 (1992), EP 0 389 699 A1.

Die zur Durchführung der erfindungsgemäßen Verfahren (D), (E), (F), (G), (H), (I) und (J) außerdem als Ausgangsstoffe benötigten Säurehalogenide der Formel (V), Carbonsäureanhydride der Formel (VI), Chlorameisensäureester oder Chlorameisensäurethioester der Formel (VII), Chlormonothioameisensäureester oder Chlordithioameisensäureester der Formel (VIII), Sulfonsäurechloride der Formel (IX), Phosphorverbindungen der Formel (X) und Metallhydroxide, Metallalkoxide oder Amine der Formel (XI) und (XII) und Isocyanate der Formel (XIII) und Carbamidsäurechloride der Formel (XIV) sind allgemein bekannte Verbindungen der Organischen bzw. Anorganischen Chemie.

Die Verbindungen der Formeln (XV), (XVIII), (XX), (XXII), (XXIII) und, (XXV), sind teilweise käuflich, teilweise bekannt und/oder lassen sich nach im Prinzip bekannten Methoden herstellen.

Das Verfahren (A) ist dadurch gekennzeichnet, dass man Verbindungen der Formel (II), in welcher A, B, D, Het und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, Het und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formel (IV) in welcher A, B, Het und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im allgemeinen arbeitet man bei Temperaturen zwischen -30°C und 250°C, vorzugsweise zwischen 0°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Das Verfahren (D-α) ist dadurch gekennzeichnet, dass man Verbindungen der Formeln (I-1-A-a) bis (I-3-B-a) jeweils mit Carbonsäurehalogeniden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (D-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (D-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (D-α) werden die Ausgangsstoffe der Formeln (I-1-A-a) bis (I-3-B-a) und das Carbonsäurehalogenid der Formel (V) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (D-β) ist dadurch gekennzeichnet, dass man Verbindungen der Formeln (I-1-A-a) bis (I-3-B-a) mit Carbonsäureanhydriden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (D-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (D-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (D-β) werden die Ausgangsstoffe der Formeln (I-1-A-a) bis (I-3-B-a) und das Carbonsäureanhydrid der Formel (VI) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, dass man Verbindungen der Formeln (I-1-A-a) bis (I-3-B-a) jeweils mit Chlorameisensäureestern oder Chlorameisensäurethiolestern der Formel (VII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan.

Die Reaktionstemperaturen können bei der Durchführung des erfindungsgemäßen Verfahrens (E) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formeln (I-1-A-a) bis (I-3-B-a) und der entsprechende Chlorameisensäureester bzw. Chlorameisensäurethiolester der Formel (VII) im allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (F) ist dadurch gekennzeichnet, dass man Verbindungen der Formeln (I-1-A-a) bis (I-3-B-a) jeweils mit Verbindungen der Formel (VIII) in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formeln (I-1-A-a) bis (I-3-B-a) ca. 1 Mol Chlormonothioameisensäureester bzw. Chlordithioameisensäureester der Formel (VIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Nitrile, Ester, Ether, Amide, Sulfone, Sulfoxide, aber auch Halogenalkane.

Vorzugsweise werden Acetonitril, Essigsäureethylester, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-A-a) bis (I-3-B-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-A-a) bis (I-3-B-a) jeweils mit Sulfonsäurechloriden der Formel (IX) gegebenenfalls in Gegenwart eines Verdünnungsmittels und gegebenenfalls in Gegenwart eines Säurebindemittels umsetzt.

Beim Herstellungsverfahren (G) setzt man pro Mol Ausgangsverbindung der Formel (I-1-A-a) bis (I-3-B-a) ca. 1 Mol Sulfonsäurechlorid der Formel (IX) bei -20 bis 150°C, vorzugsweise bei 20 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe wie Methylenchlorid.

Vorzugsweise werden Acetonitril, Essigsäureethylester, Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-A-a) bis (I-3-B-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (H) ist dadurch gekennzeichnet, dass man Verbindungen der Formeln (I-1-A-a) bis (I-3-B-a) jeweils mit Phosphorverbindungen der Formel (X) gegebenenfalls in Gegenwart eines Verdünnungsmittels und gegebenenfalls in Gegenwart eines Säurebindemittels umsetzt.

Beim Herstellungsverfahren (H) setzt man zum Erhalt von Verbindungen der Formeln (I-1-A-e) bis (I-3-B-e) auf 1 Mol der Verbindungen (I-1-A-a) bis (1-3-B-a), 1 bis 2, vorzugsweise 1 bis 1,3 Mol der Phosphorverbindung der Formel (X) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (I) ist dadurch gekennzeichnet, dass man Verbindungen der Formeln (I-1-A-a) bis (I-3-B-a) mit Metallhydroxiden bzw. Metallalkoxiden der Formel (XI) oder Aminen der Formel (3üI), gegebenenfalls in Gegenwart eines Verdünnungsmittels, umsetzt.

Als Verdünnungsmittel können bei dem erfindungsgemäßen Verfahren (I) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (I) wird im allgemeinen unter Normaldruck durchgeführt.

Die Reaktionstemperaturen liegen im allgemeinen zwischen -20°C und 100°C, vorzugsweise zwischen 0°C und 50°C.

Das erfindungsgemäße Verfahren (J) ist dadurch gekennzeichnet, dass man Verbindungen der Formeln (I-1-A-a) bis (I-3-B-a) jeweils (J-α) mit Verbindungen der Formel (XIII) gegebenenfalls in Gegenwart eines Verdünnungsmittels und gegebenenfalls in Gegenwart eines Katalysators oder (J-β) mit Verbindungen der Formel (XIV) gegebenenfalls in Gegenwart eines Verdünnungsmittels und gegebenenfalls in Gegenwart eines Säurebindemittels umsetzt.

Bei Herstellungsverfahren (J-α) setzt man pro Mol Ausgangsverbindung der Formeln (I-1-A-a) bis (I-3-B-a) ca. 1 Mol Isocyanat der Formel (XIII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (J-β) setzt man pro Mol Ausgangsverbindung der Formeln (I-1-A-a) bis (I-3-B-a) ca. 1 Mol Carbamidsäurechlorid der Formel (XIV) bei - 20 bis 150°C, vorzugsweise bei 0 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe.

Vorzugsweise werden Acetonitril, Essigsäureethylester, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-A-a) bis (I-3-B-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofinannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link®⁻ (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage:
z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin, Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen, Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon, (E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin,
Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, BromophosA, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon,
Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dergleichen zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-FungizidGemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dergleichen zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dergleichen eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethem wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozide und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-n-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

### Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

### Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;

### Molluskizide wie

Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind.* **1985,** *37,* 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel I-1-A-a-1

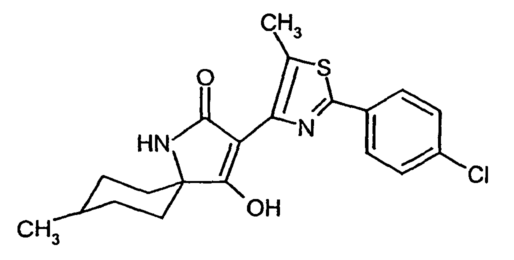

3,70 g (0,031 Mol) Kalium-tert.-butylat werden in 26 ml wasserfreiem DMF bei 0°C vorgelegt und 6 g der Verbindung gemäß Beispiel II-1 in 12 ml wasserfreiem DMF zugegeben. Man rührt 1 h bei 20°C.

Die Reaktionslösung gibt man in 200 ml Eiswasser und säuert bei 0-10°C an. Anschließend saugt man den Niederschlag ab und kristallisiert um.

Ausbeute: 4,66 g (=̂ 83 % der Theorie), Fp. > 260°C.

DMF (Dimethylformamid)

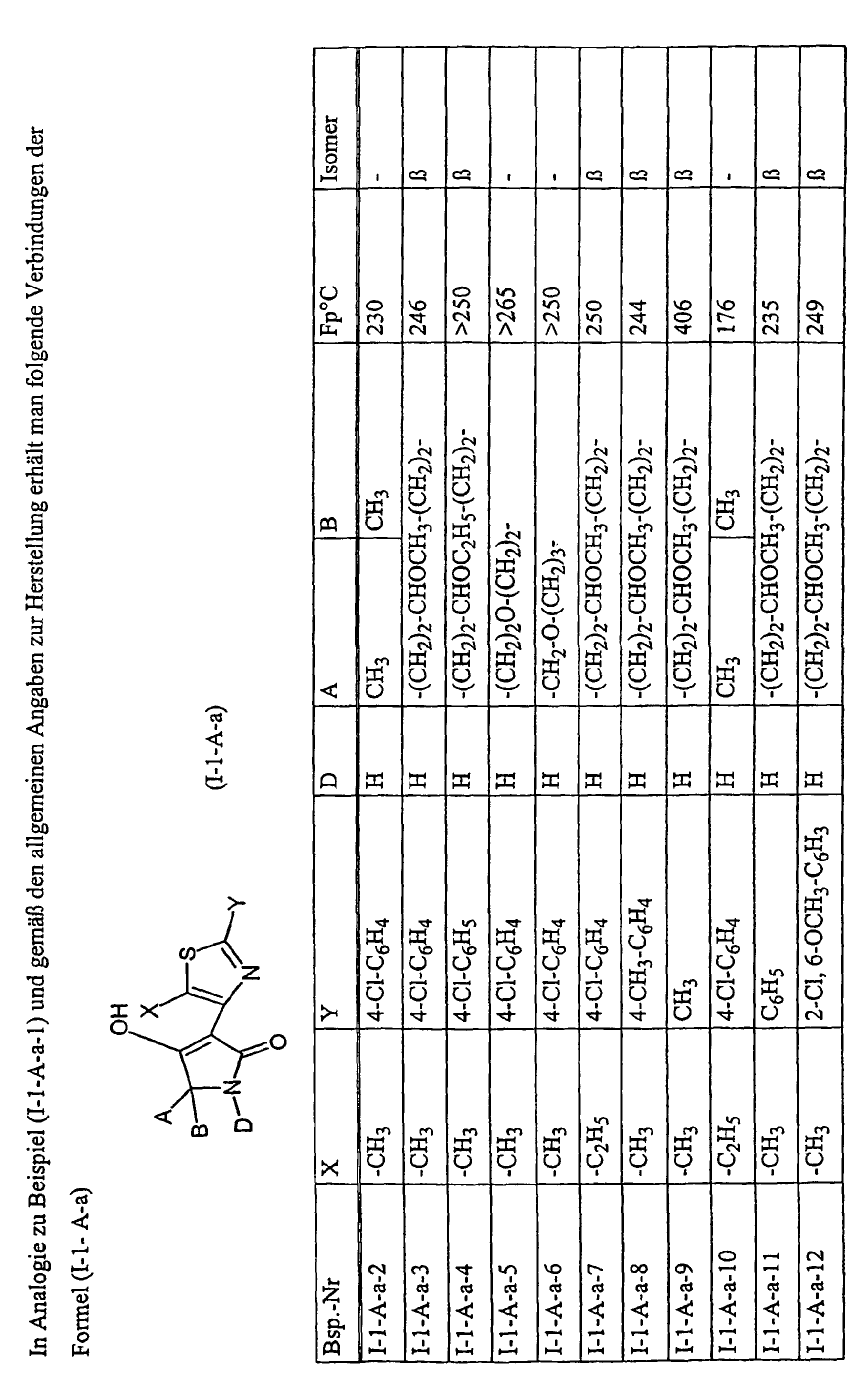

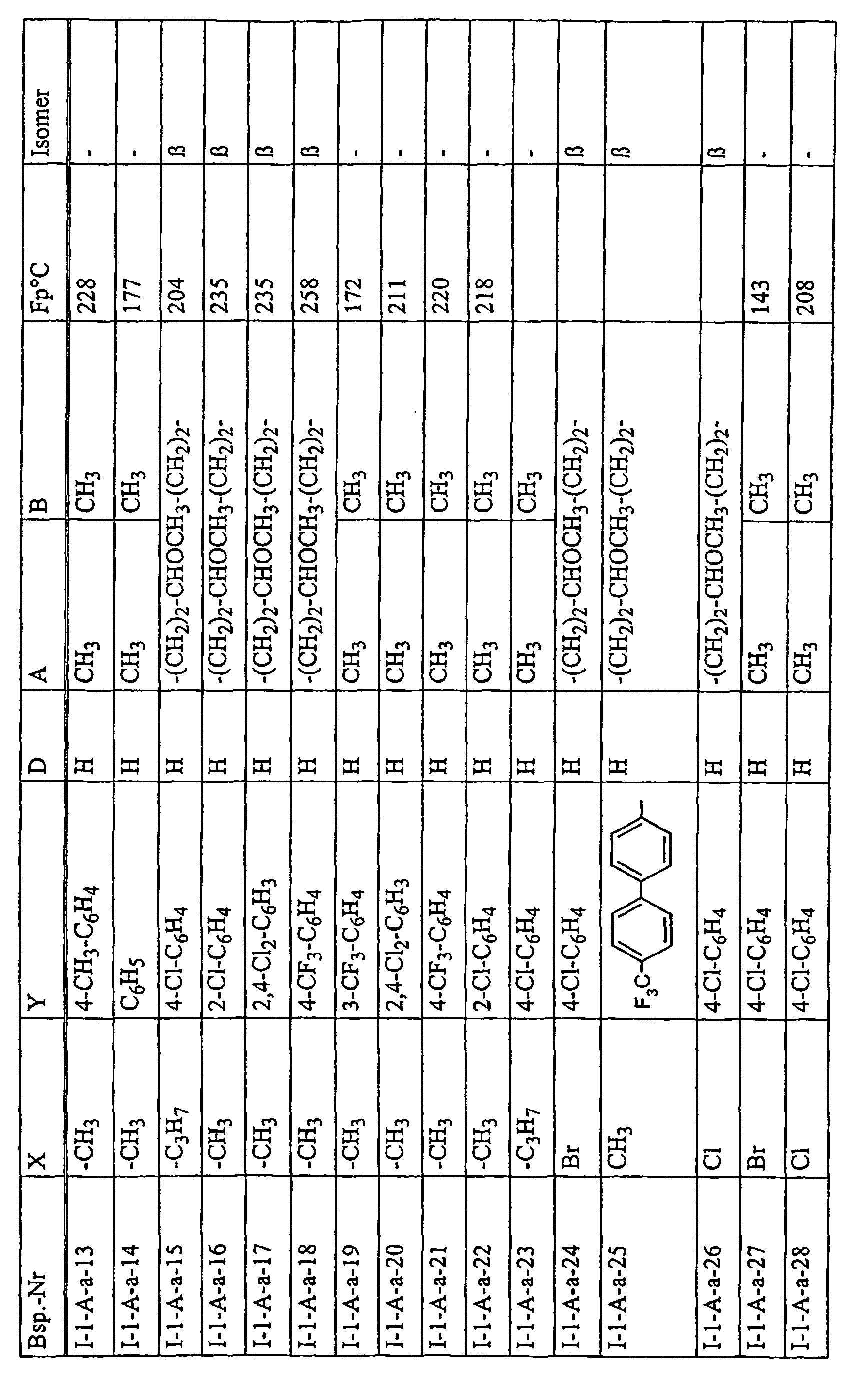

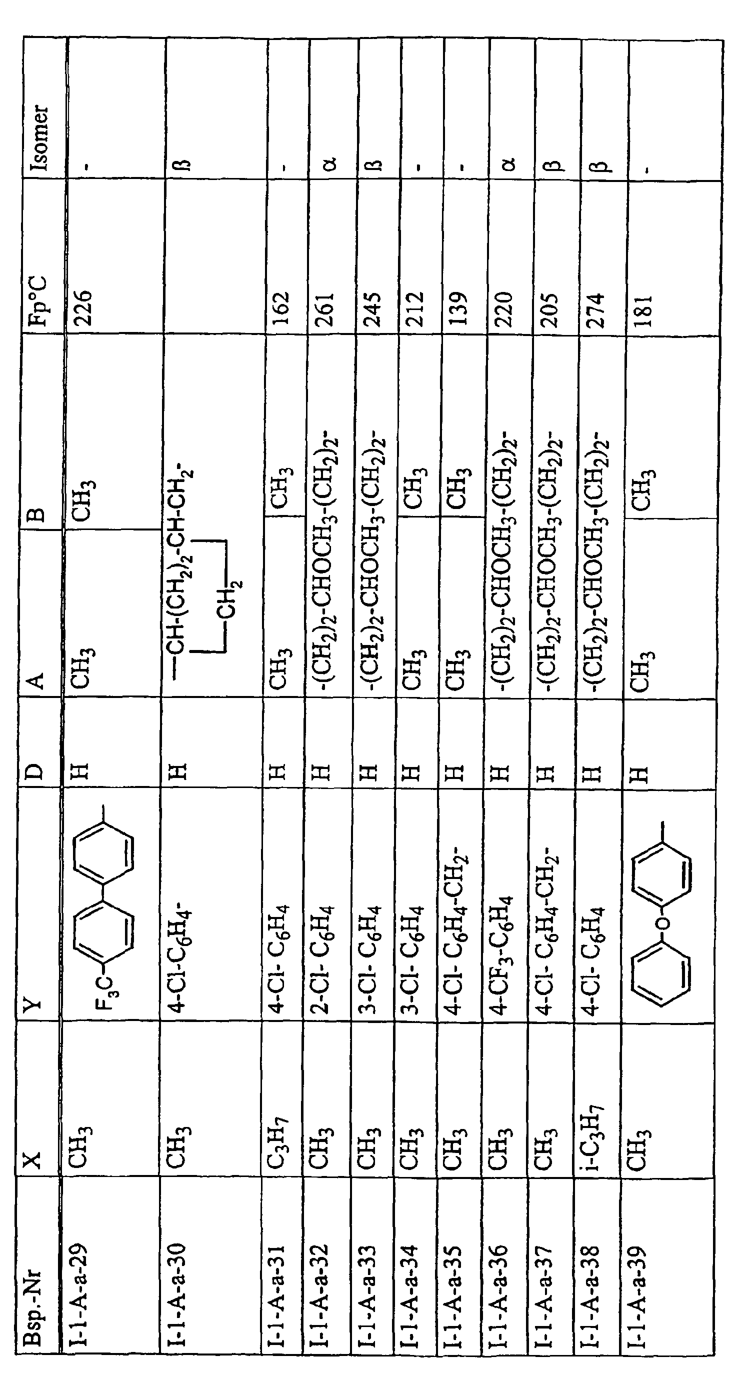

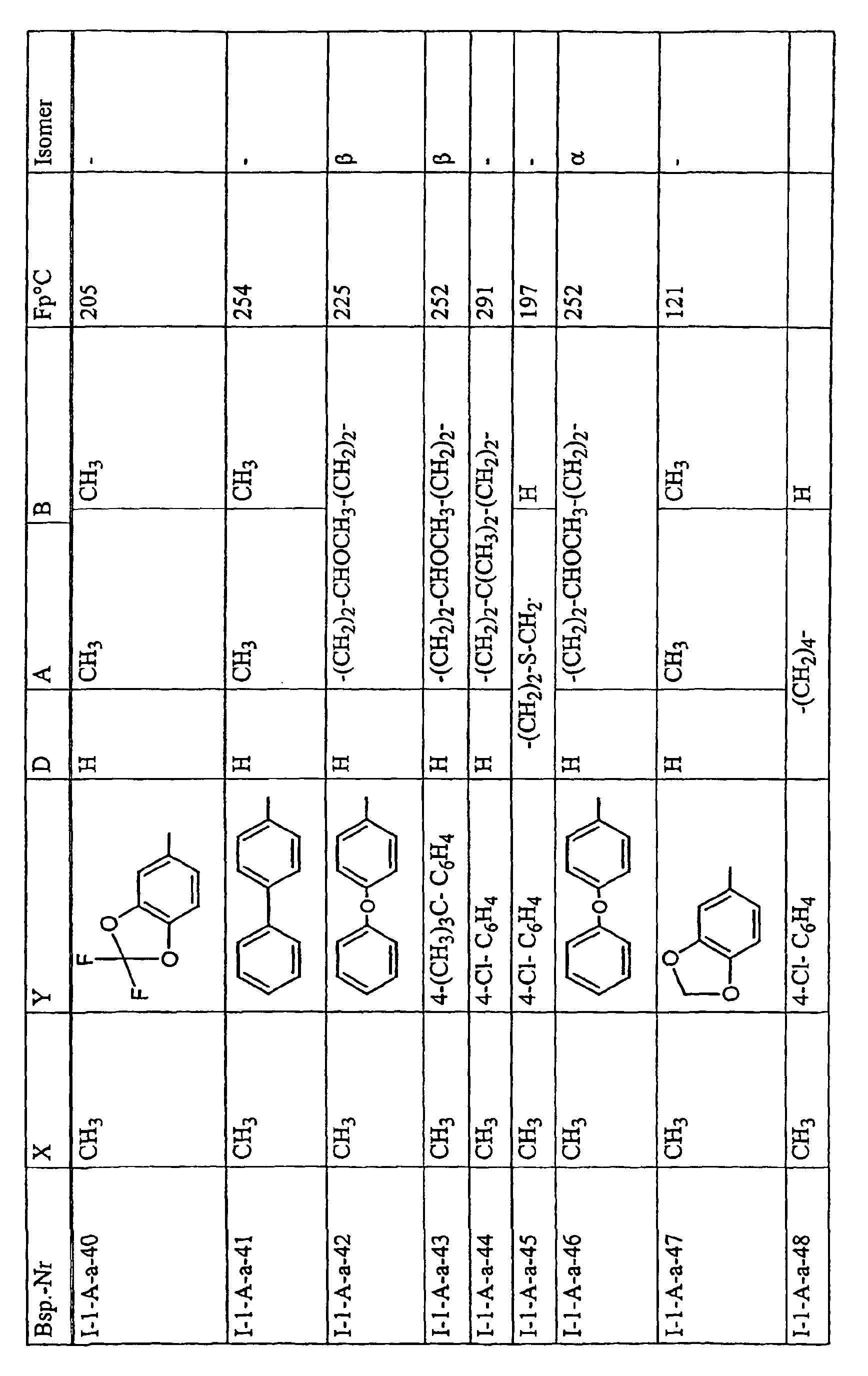

### Beispiel I-1-A-b-1

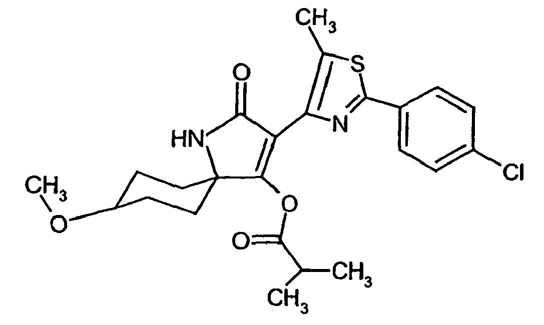

0,81 g der Verbindung gemäß Beispiel I-1-a-2 werden in 10 ml wasserfreiem Essigsäureethylester vorgelegt und mit 0,3 ml (2,2 mmol) Triethylamin unter Rückfluss erhitzt. Anschließend gibt man 0,25 ml (0,0022 Mol) Isobuttersäurechlorid in 5 ml wasserfreiem Essigsäureethylester hinzu und erhitzt unter Rückfluss.

Nach beendeter Reaktion (DC-Kontrolle) wird das Lösungsmittel abdestilliert und der Rückstand in Dichlormethan aufgenommen, zweimal mit 10 ml 0,5 N NaOH gewaschen, getrocknet und einrotiert. Der Rückstand wird säulenchromatographisch an Kieselgel gereinigt (Dichlormethan/Essigsäureethylester, 3:1).
Ausbeute: 0,2 g (=̂ 21 % d.Th.), Fp 208°C.

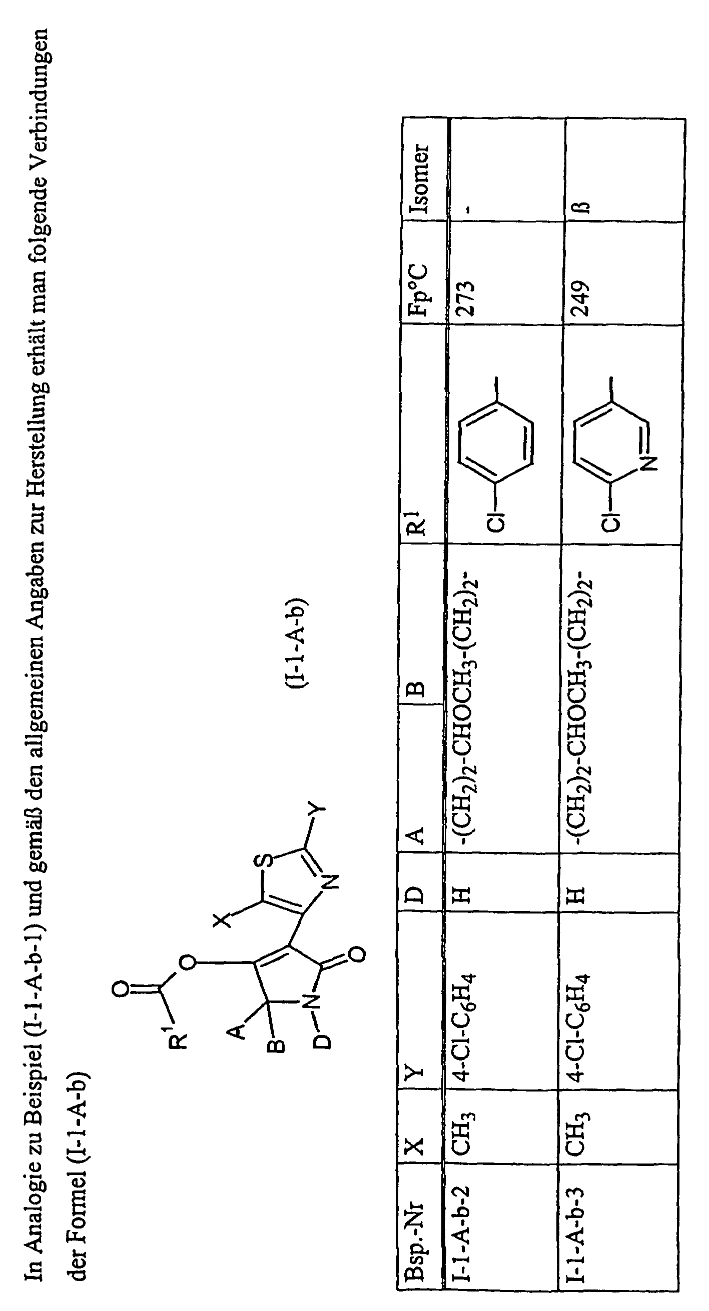

### Beispiel I-1-A-c-1

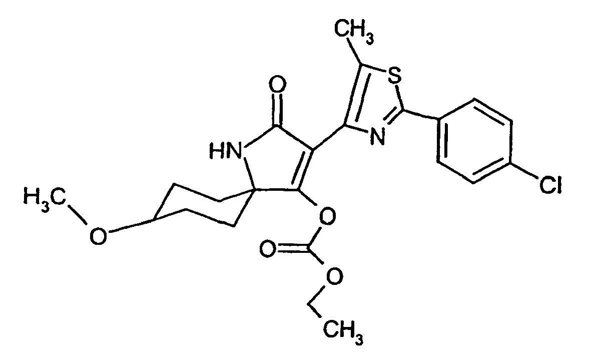

0,69 g der Verbindung gemäß Beispiel I-1-a-2 werden in 10 ml wasserfreiem Dichlormethan vorgelegt und bei 10°C mit 0,24 ml (1,7 mmol) Triethylamin versetzt.

Anschließend gibt man 0,17 ml (1,7 mmol) Chlorameisensäure-ethylester in 1 ml wasserfreiem Dichlormethan hinzu und rührt bei Raumtemperatur.

Nach beendeter Reaktion (DC-Kontrolle) wird das Lösungsmittel abdestilliert und der Rückstand in Dichlormethan aufgenommen, zweimal mit 10 ml 0,5 N NaOH gewaschen, getrocknet und einrotiert. Der Rückstand wird säulenchromatographisch an Kieselgel gereinigt (Dichlormethan/Essigsäureethylester, 3:1).

Ausbeute: 0,58 g (=̂ 71 % d.Th), Fp. 211°C.

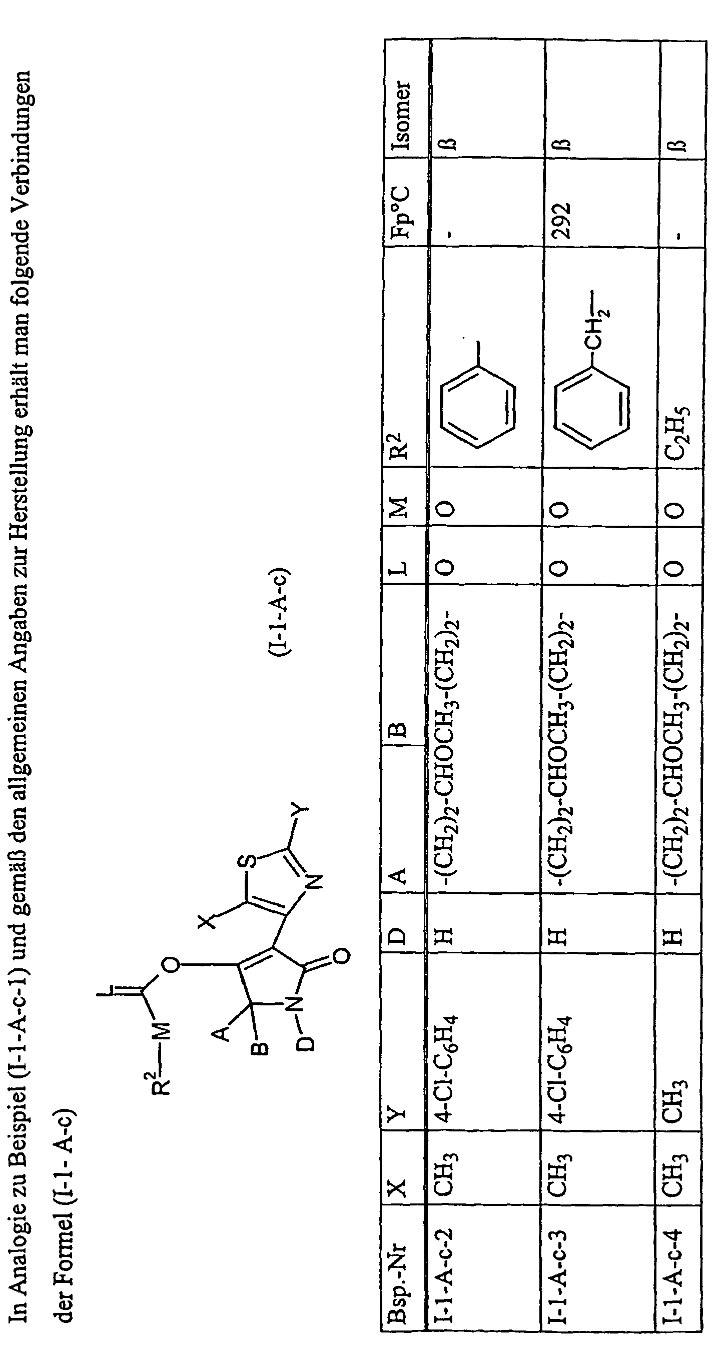

### Beispiel II-A-1

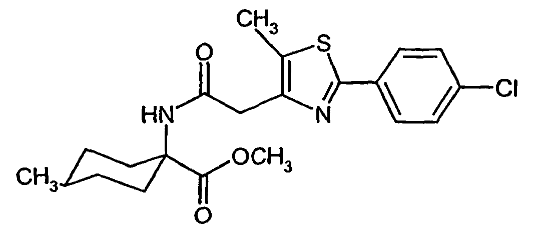

Zu 4,61 g 4-Methyl-1-amino-cyclohexan-1-carbonsäuremethylester-hydrochlorid in 80 ml wasserfreiem Tetrahydrofuran gibt man 6,6 ml Triethylamin und rührt 3 Min. 5,3 g 4-[2-(4-Chlorphenyl)-5-methyl]-thiazolyl-essigsäure werden zugegeben. Man rührt weitere 15 Min. bei Raumtemperatur. Anschließend gibt man 4,4 ml Triethylamin zu und tropft sofort 1,12 ml Phosphoroxychlorid zu, dass die Lösung mässig siedet. Man erhitzt 30 Min. unter Rückfluss.

Die Reaktionslösung wird in 200 ml Eiswasser gegeben, mit Dichlormethan extrahiert und getrocknet. Das Lösungsmittel wird abdestilliert und der Rückstand umkristallisiert.

Ausbeute: 6,19 g (=̂ 73 % d. Th.), Fp: 150°C.

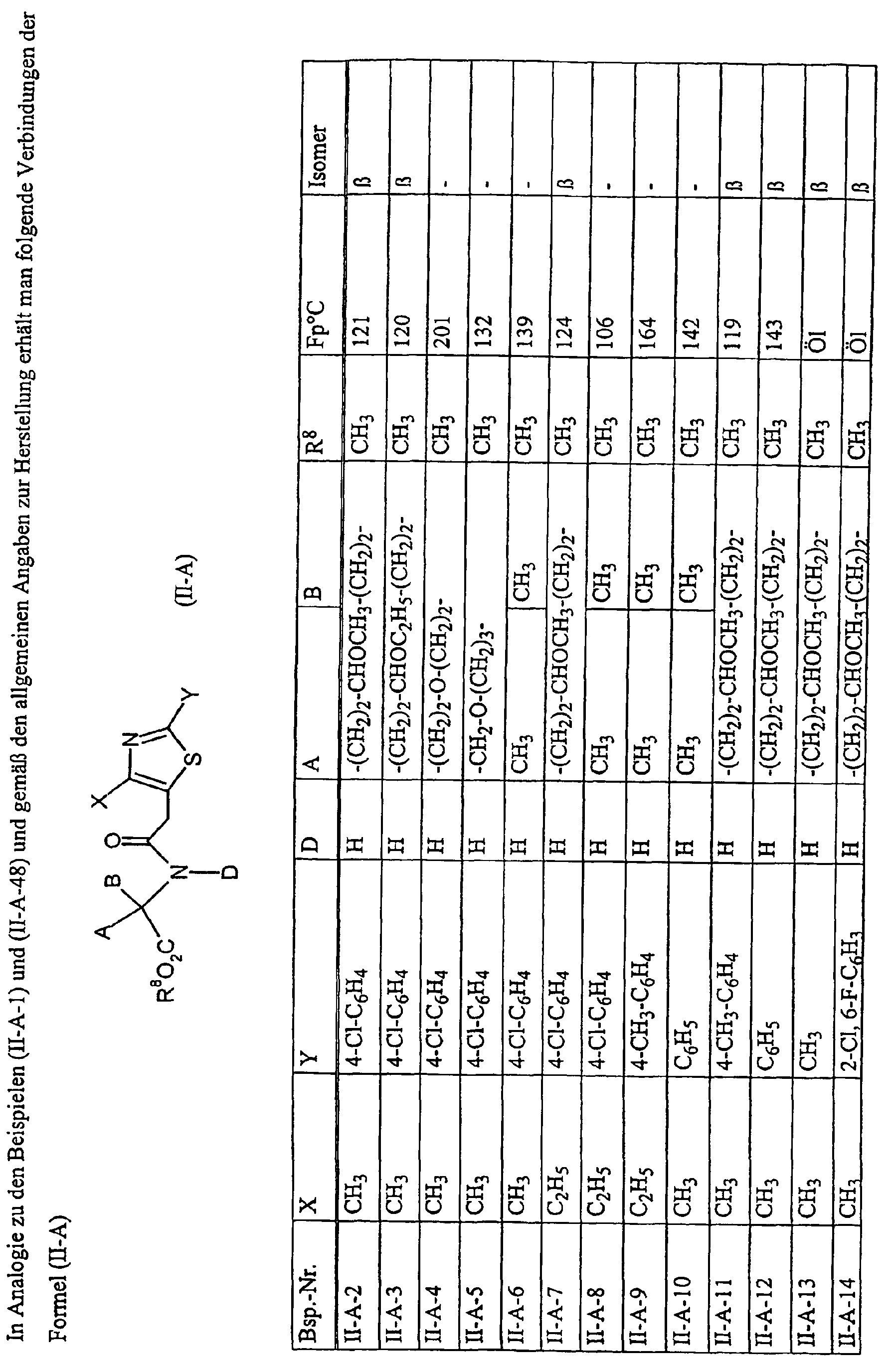

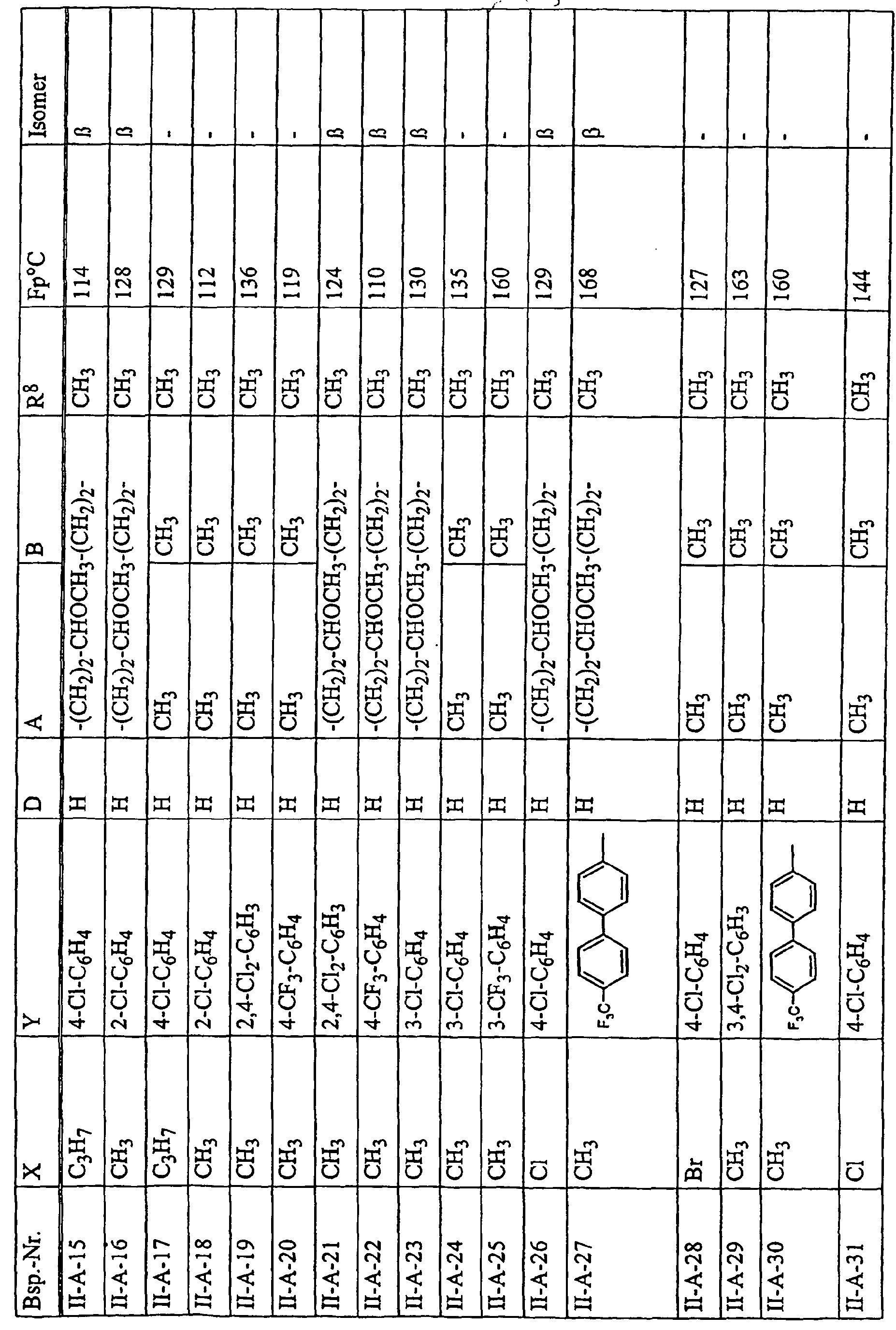

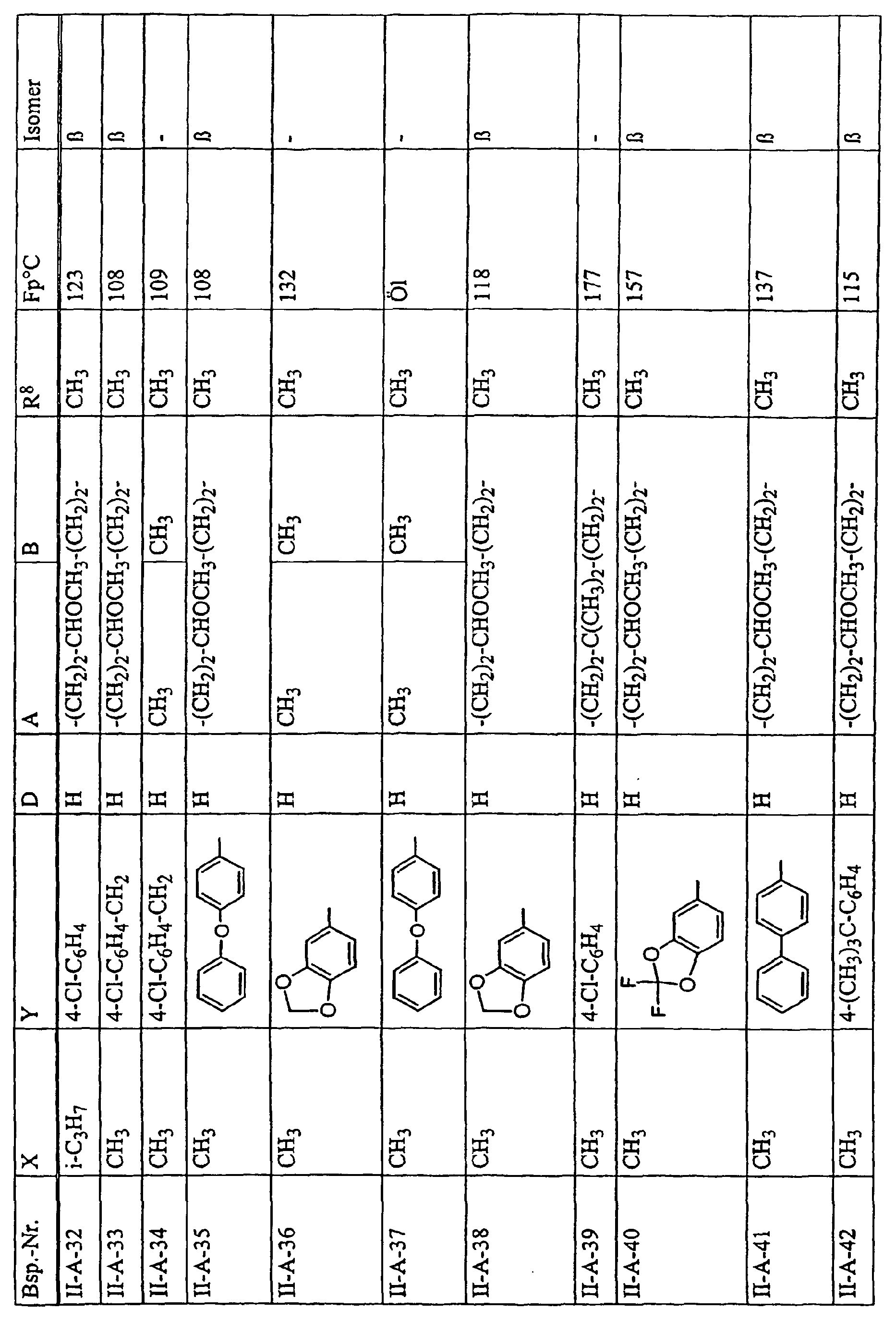

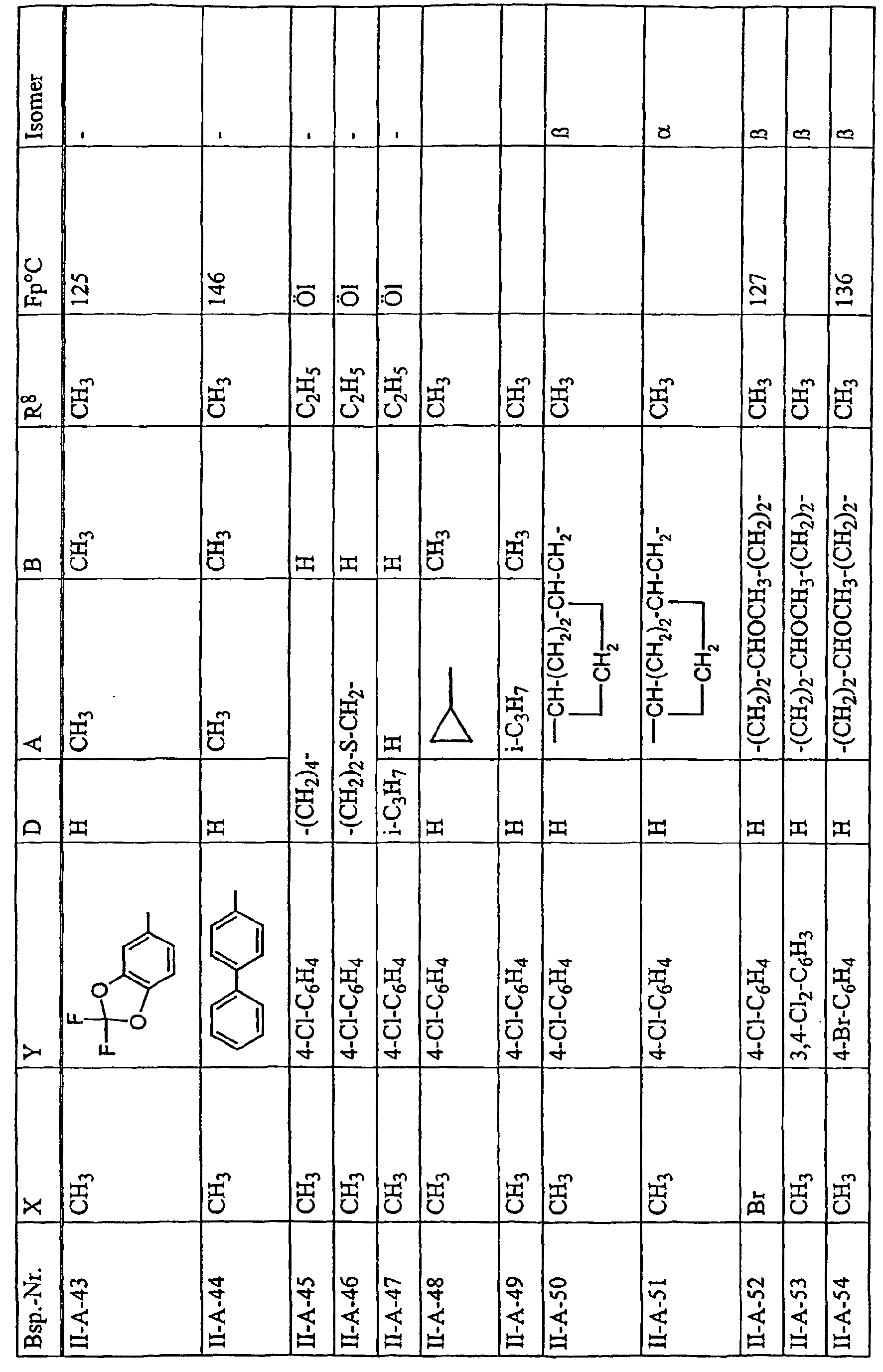

### Beispiel-Nr. II-A-48

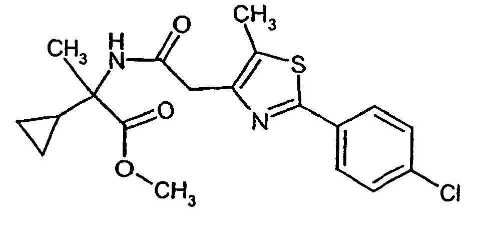

7,4 g (0,075 mol) konzentrierte Schwefelsäure werden vorgelegt und 5,37 g der Verbindung gemäß Herstellungsbeispiel XXI-A-1 in 30 ml Methylenchlorid bei einer Innentemperatur von 30 bis 40°C zugetropft. Anschließend wird 2 Stunden bei 30 bis 40°C nachgerührt. Man tropft 11 ml absolutes Methanol zu, dass sich eine Innentemperatur von 40°C einstellt. Es wird 6 Stunden bei 40 bis 70°C weitergerührt.

Die Reaktionslösung wird auf 0,08 kg Eis gegeben. Man extrahiert mit Dichlormethan, wäscht mit NaHCO₃-Lösung, trocknet und destilliert das Lösungsmittel ab. Anschließend wird der Rückstand säulenchromatographisch über Kieselgel (Dichlormethan/Essigsäureethylester, 5:1) gereinigt.

Man erhält 0.42 g (=̂ 7 % der Theorie), Fp. 131°C.

### Beispiel XXI-A-1

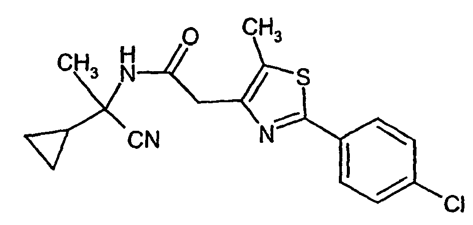

3,7 g 2-Amino-2-cyclopropyl-propionitril in 60 ml absolutem Tetrahydrofuran und 4,92 ml Triethylamin werden 5 min gerührt. Dann gibt man 8,03 g 5-Methyl-2-(4-chlor-phenyl)-thiazolylessigsäure zu und rührt 15 min bei Raumtemperatur. Es werden 6,6 ml Triethylamin zugegeben und sofort 1,68 ml Phosphoroxychlorid so zugetropft, dass die Lösung mäßig siedet.

Man rührt 30 min unter Rückfluss. Das Lösungsmittel wird anschließend abdestilliert und der Rückstand säulenchromatographisch an Kieselgel (n-Hexan/Essigsäureethylester, 2:1) gereinigt.

Ausbeute: 5,37 g (49 % d. Th.), Fp. 114°C.

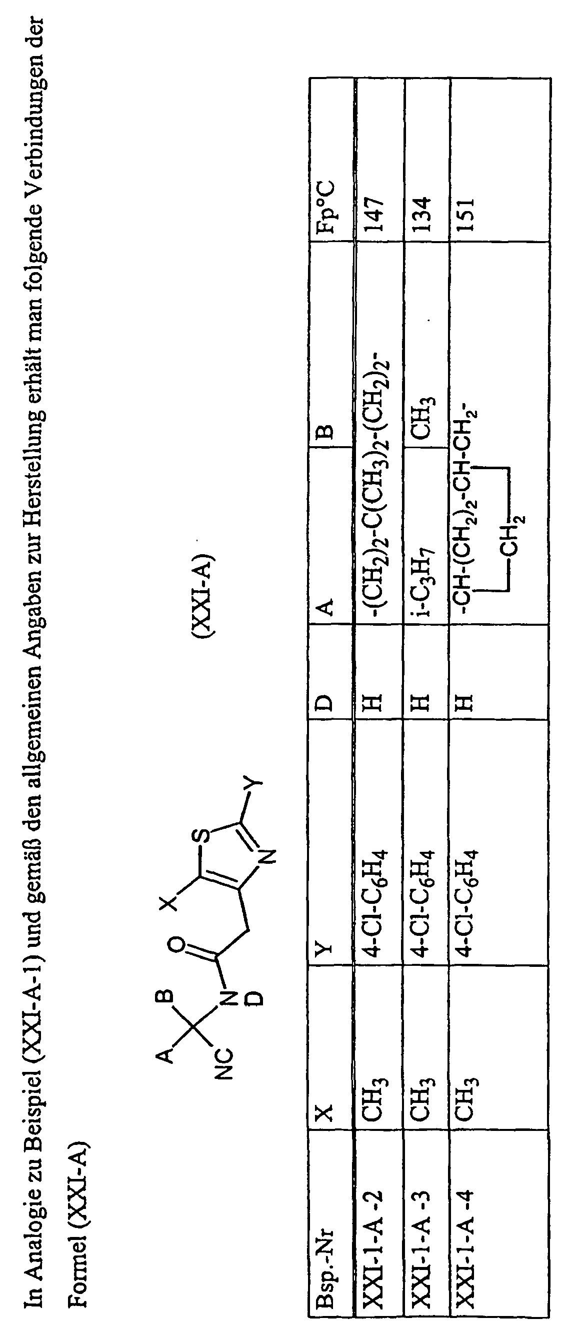

### Beispiel I-2-A-a-1

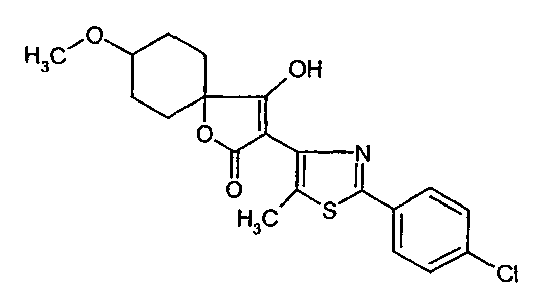

Zu 2,48 g (5,49 mmol) der Verbindung gemäß Beispiel III-1 in 10 ml wasserfreiem DMF gibt man unter Eiskühlung 6,6 ml 1 M Kalium-tert.-butylat-Lösung und rührt bei Raumtemperatur über Nacht. Das Lösungsmittel wird abdestilliert, der Rückstand in Wasser gelöst, langsam mit konz. HCl angesäuert, abgesaugt und getrocknet. Ausbeute: 2 g (=̂ 89 % d.Th.), Fp. 117-120°C.

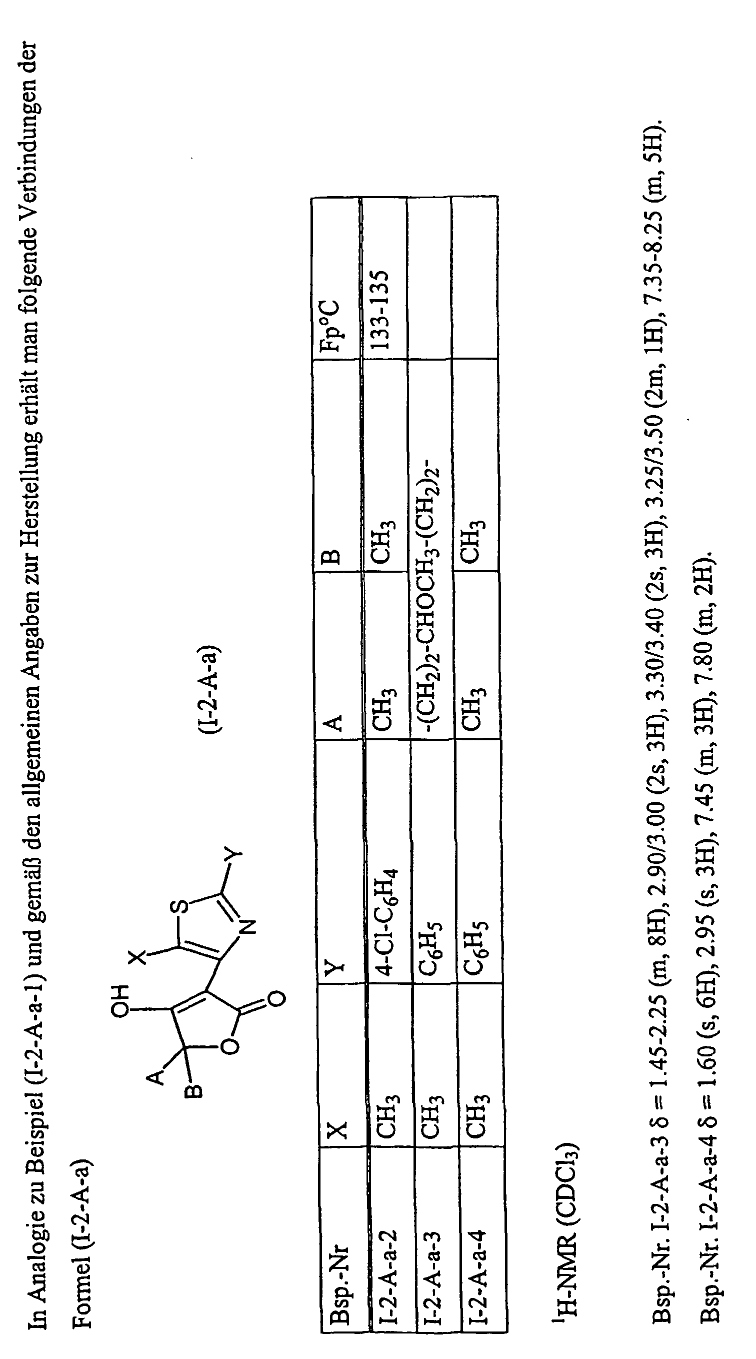

### Beispiel I-2-A-b-1

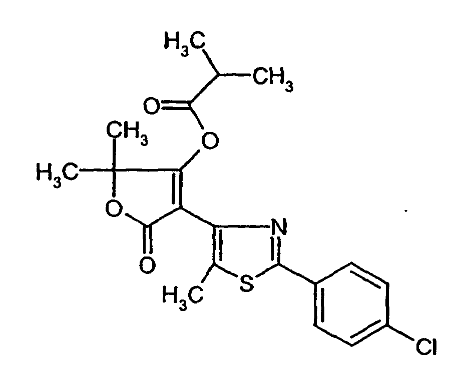

Zu 0,7 g (2,1 mmol) der Verbindung gemäß Herstellungsbeispiel III-A-2 in absolutem Dichlormethan und 0,232 g (2,3 mmol) Triethylamin gibt man bei 0 bis 10°C 0,244 g (2,3 mmol) Isobuttersäurechlorid und rührt bei Raumtemperatur 8 Stunden. Die Reaktionslösung wird mit 10 % Zitronensäure und 10 % NaOH-Lösung gewaschen. Die organische Phase wird abgetrennt, getrocknet und eingeengt.

Es wird säulenchromatographisch gereinigt über Kieselgel (Dichlormethan/Essigsäureethylester, 20/1).

Ausbeute: 0,1 g =̂ 11,8 % der Theorie, Fp. 95-98°C.

### Beispiel I-2-A-c-1

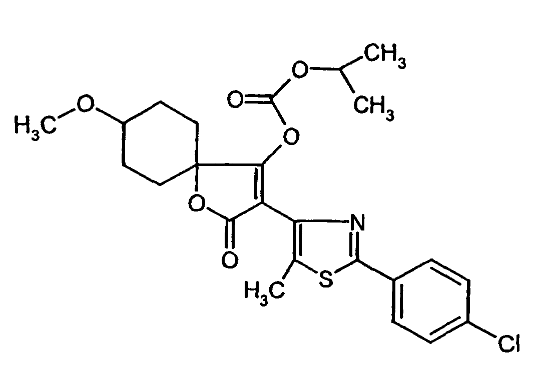

Zu 0,75 g (1,85 mmol) gemäß Beispiel I-2-A-1 in absolutem Dichlormethan und 0,206 g (2 mmol) Triethylamin gibt man bei 0 bis 10°C Chlorameisensäureisopropylester (0,249 g, 2 mmol, 1 M in Toluol). Man rührt 8 Stunden bei Raumtemperatur. Man wäscht mit 10 % Zitronensäurelösung und mit 10 %iger NaOH-Lösung, trennt die organische Phase ab, trocknet und destilliert das Lösungsmittel ab.

Es wird säulenchromatographisch gereinigt über Kieselgel (Dichlormethan/Essigsäureethylester, 20/1).

Ausbeute: 0,37 g =̂ 40 % der Theorie, Fp. 140-145°C.

### Beispiel III-A-1

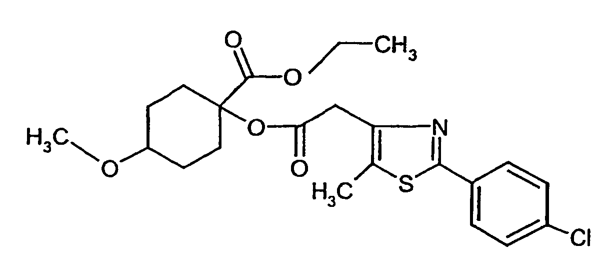

1,11 g (5,49 mmol) 4-Methoxy-1-hydroxy-cyclohexan-1-carbonsäureethylester und 1,57 g (5,49 mmol) der Verbindung gemäß Beispiel XVI-1 werden 6 h bei 140°C verrührt, abgekühlt und das HCl-Gas mit Argon ausgeblasen.

Ausbeute: 2,4 g (=̂ 41,62 % d.Th.), LogP (sauer) 4,54 (HPLC; Acetonitril).

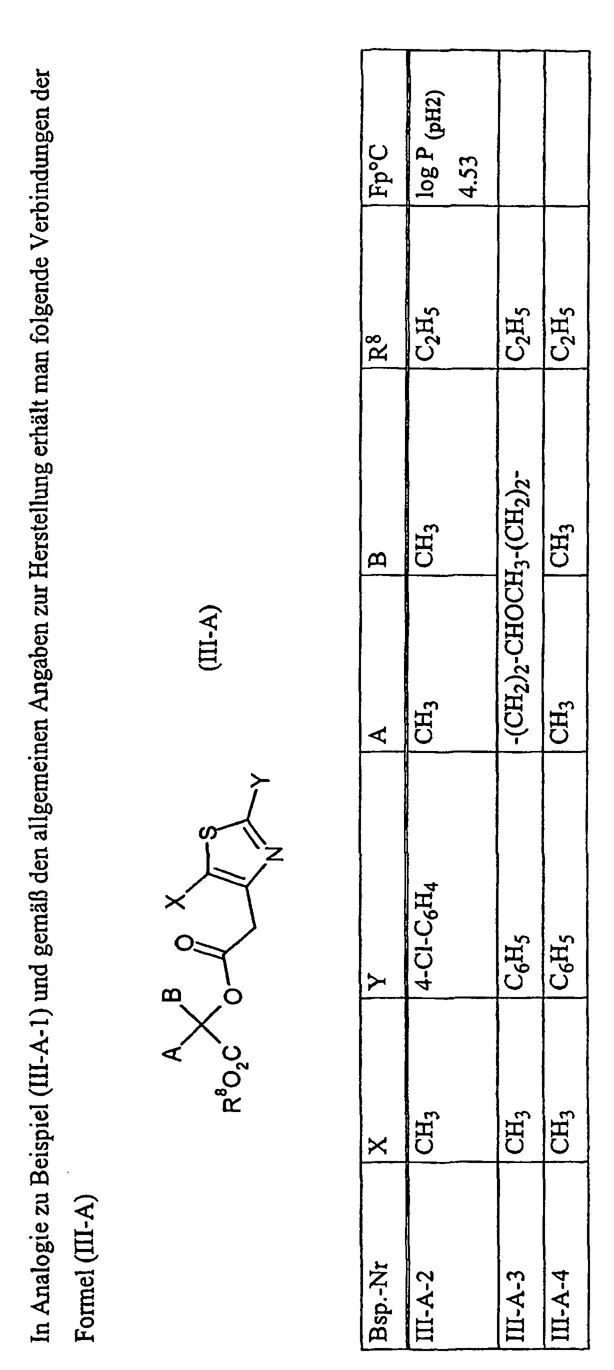

### Beispiel I-3-A-a-1

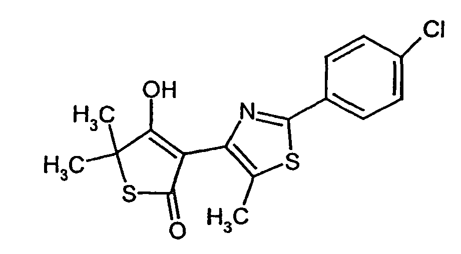

15 g (20 mmol) der Verbindung gemäß Beispiel (IV-A-1) wird in 22 ml Trifluoressigsäure (TFA) und 51 ml Toluol vorgelegt und über Nacht unter Rückfluss erhitzt. Das Lösungsmittel und die TFA werden abdestilliert, der Rückstand in 200 ml Wasser und 200 ml Methyl-tert.-butylether aufgenommen. Die organische Phase wird alkalisch extrahiert und die wässrige Phase angesäuert, mit Dichlormethan extrahiert, getrocknet und das Lösungsmittel abdestilliert.

Es wird säulenchromatographisch über Kieselgel (Dichlormethan/Essigsäureethylester, 10:1 → 5:1) gereinigt.

Ausbeute: 1,6 g (23 % d. Th.), Fp. 132-135°C.

### Beispiel I-3-A-c-1

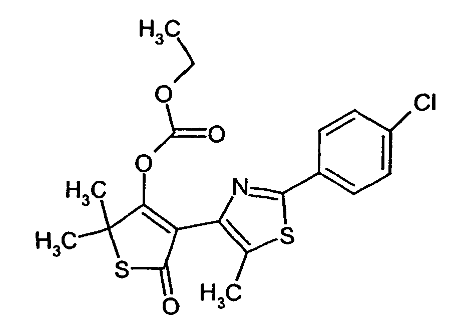

500 mg (1,4 mmol) der Verbindung gemäß Beispiel (I-3-A-a-1) werden in 5 ml absolutem Dichlormethan vorgelegt, mit 0,29 ml (2,1 mmol, 1,5 eq) Triethylamin versetzt und unter Eiskühlung 0,2 g (1,8 mmol, 1,3 eq) Chlorameisensäureethylester zugegeben.

Es wird 2 Stunden bei Raumtemperatur gerührt. Man wäscht mit 10 %iger Citronensäure, extrahiert mit Dichlormethan, wäscht mit 1 N NaOH, extrahiert mit Dichlormethan, trocknet und destilliert das Lösungsmittel ab.

Ausbeute: 0,55 g (93 % d. Th.), Öl.

¹H-NMR (d₆-DMSO, 400 MHz): δ = 0,93 (t, 3H, CH₃-CH₂), 1,75 (s, 6H, 2 CH₃-C), 2,37 (s, 3H, CH₃-C arom.), 3,98 (q, 2H, CH₃-CH₂), 7,55 (d, 2H, 2 CH arom.), 7,87 (d, 2H, 2 CH arom.) ppm.

### Beispiel IV-A-1

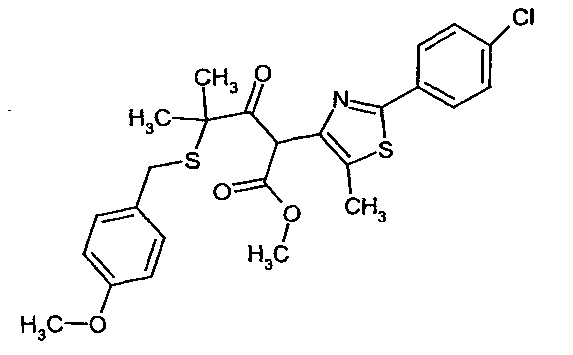

Es wird 4,8 g (20,0 mmol) 2-(4-Methoxy-benzylthio)-2-methyl-propionsäure in 40 ml wasserfreiem Toluol und 1 Tropfen DMF vorgelegt und 7,2 g (60 mmol, 4 ml) Thionylchlorid zugesetzt. Man rührt 5 min bei Raumtemperatur, anschließend bei 100°C, bis die Gasentwicklung beendet ist. Das Lösungsmittel wird abdestilliert und der Rückstand in 10 ml wasserfreien Tetrahydrofuran aufgenommen (Lösung A).

17.1 ml Lithium-düsopropylamid in 40 ml wasserfreiem Tetrahydrofuran werden bei 0°C vorgelegt und 10,1 g (32,7 mmol) 4-[5-Methyl-2-(4-chlor-phenyl)]-thiazolylessigsäuremethylester in 10 ml wasserfreiem Tetrahydrofuran bei 0°C zugetropft und 30 min nachgerührt. Anschließend tropft man Lösung A bei 0°C zu und rührt 1 h bei Raumtemperatur nach. Anschließend versetzt man die Reaktionsmischung mit 100 ml MTB-Ether und einigen Tropfen Wasser und wäscht 2 x mit je 100 ml 10 %iger NH₄Cl-Lösung, die organische Phase wird getrocknet und das Lösungsmittel abdestilliert. Das Rohprodukt wird ohne weitere Reinigung umgesetzt.

Ausbeute: 15,2 g (100 % d. Th.).

¹H-NMR (DMSO, 400 MHz): δ = 1,43 (s, 3H, CH₃-C aliph.), 1,50 (s, 3H, CH₃-C aliph.), 2,40 (s, 3H, CH₃-C heteroarom.), 3,31 (s, 2H, CH₂), 3,61 (s, 3H, CH₃-OCO), 3,82 (s, 3H, CH₃-O) ppm.

### Beispiel XVI-1

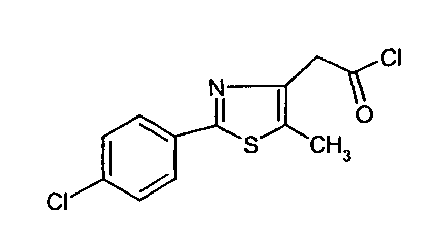

3 g (11,21 mmol) 4-[2-(4-Chlorphenyl)-5-methyl]-thiazolyl-essigsäure werden in 50ml absolutem Dichlormethan vorgelegt. Unter Eiskühlung werden 2,13 g (16,81 mmol) Oxalsäurechlorid langsam zugetropft, über Nacht bei Raumtemperatur gerührt und anschließend 2 h unter Rückfluss erhitzt und das Lösungsmittel abdestilliert.

Ausbeute: 3,19 g (96 % d.Th.).

### Beispiel I-1-B-a-1

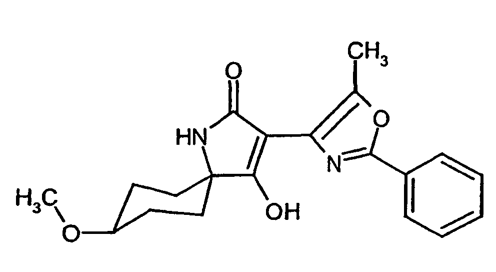

Zu 2,41 g (0,020 mol) Kalium-tert.-butylat in 17 ml wasserfreiem Dimethylformamid (DMF) werden bei 20°C 3,59 g der Verbindung gemäß Herstellungsbeispiel II-B-1 in 7 ml wasserfreiem DMF zugetropft und bei 40°C 1 Stunde gerührt.

Die Reaktionslösung wird in 100 ml Eiswasser eingerührt und bei 0 bis 10°C mit Salzsäure angesäuert. Der Niederschlag wird abgesaugt und mit Methylenchlorid/Aceton 5:1 an Kieselgel chromatographiert.

Ausbeute: 1,2 g (36 % d. Th.), Fp >240°C

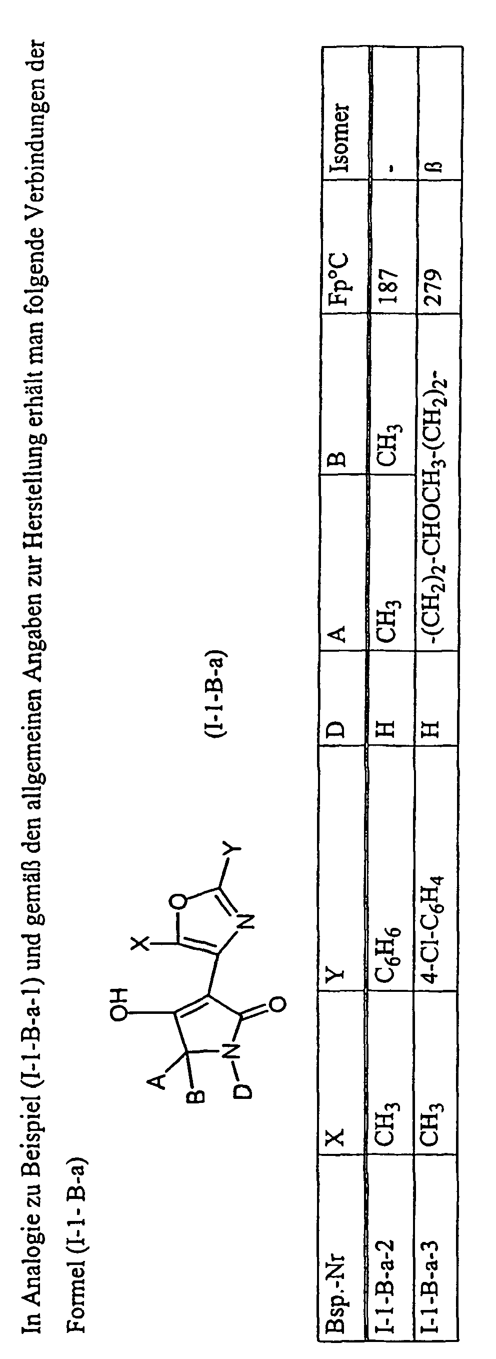

### Beispiel II-B-1

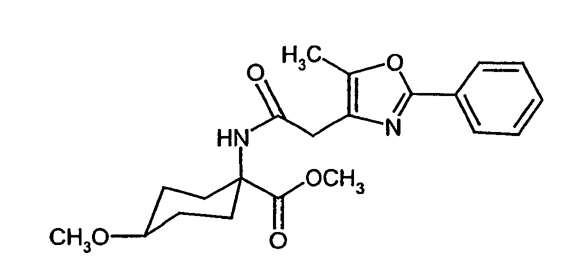

4,56 g (20 mmol) 1-Amino-4-methoxy-cyclohexancarbonsäuremethylester-hydrochlorid werden in 60 ml absolutem THF vorgelegt, mit 6 ml Triethylamin versetzt und 5 min gerührt. Man gibt 3,27 g 4-(2-Phenyl-5-methyl)-oxazolyl-essigsäure zu und rührt bei Raumtemperatur 15 min. Es werden 3,3 ml Triethylamin zugegeben und 0,84 ml Phosphoroxychlorid so zugetropft, dass die Lösung mäßig siedet.

Man rührt 30 min unter Rückfluss.

Das Lösungsmittel wird abdestilliert und der Niederschlag säulenchromatographisch über Kieselgel (Dichlormethan/Essigsäureethylester, 3:1) gereinigt.

Ausbeute: 3,59 g (61 % d. Th.), Fp 125°C.

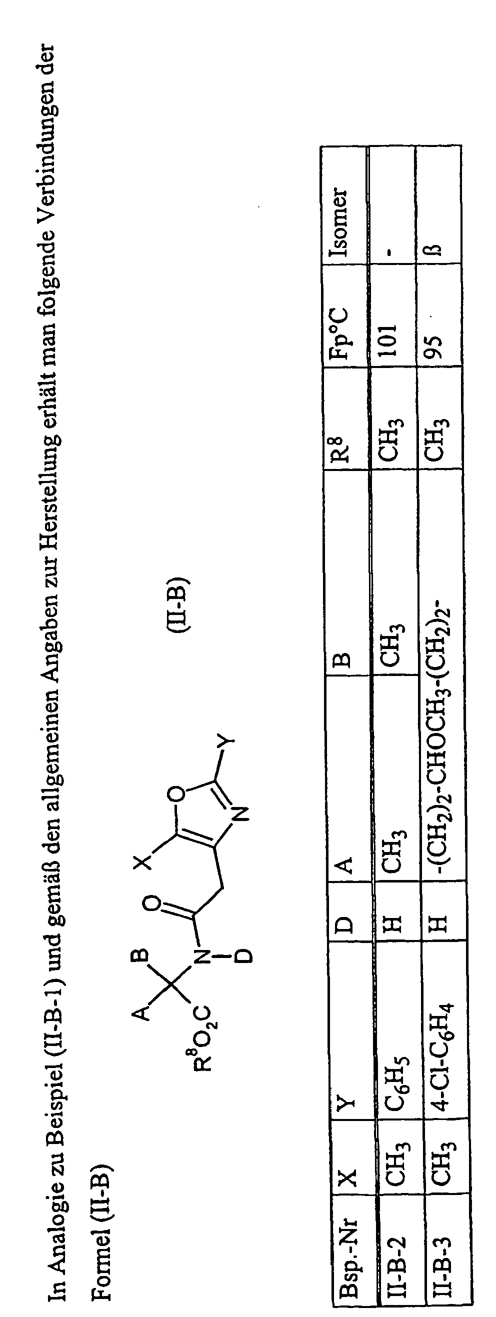

### Beispiel I-2-B-a-1

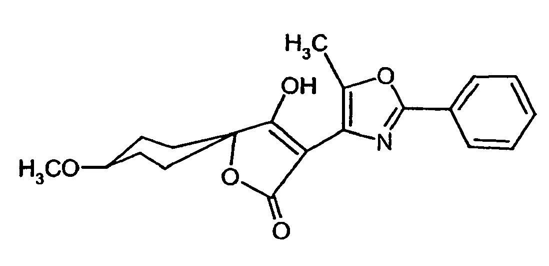

1,01 g (5 mmol) 4-Methoxy-1-Hydroxy-cyclohexan-1-carbonsäureethylester und 1,095 g (5 mmol) 2-Phenyl-4-methyl-isoxazol-3-acetylchlorid wurden ohne Lösungsmittel über Nacht bei 140°C erwärmt und anschließend kurz im Vakuum entgast. Das erhaltene Rohprodukt wurde in 10 ml DMF gelöst. 6 ml 1M Lösung von KOtBu in DMF in 10 min zudosiert, über Nacht bei Raumtemperatur gerührt, zur Trockene einrotiert, der Rückstand in Wasser gelöst, 1 x mit Essigester extrahiert um Verunreinigungen zu entfernen, 10 ml 1N Salzsäure langsam zudosiert (bis sauer) und das ausgefallene Produkt abgesaugt. Die weitere Reinigung erfolgte durch präparative HPLC (Acetonitril/Wasser an RP-18):

Ausbeute: 0,33 g (19 % d. Th.), Fp. 65°C.

¹H-NMR MHz (CDCl₃) : δ = 1,60-2,30 (m, 8H), 2,85 (s, 3H), 3,30/3,40 (2s, 3H), 3,30/3,60 (2 m, 1H), 7,40-8,00 (m, 5H).

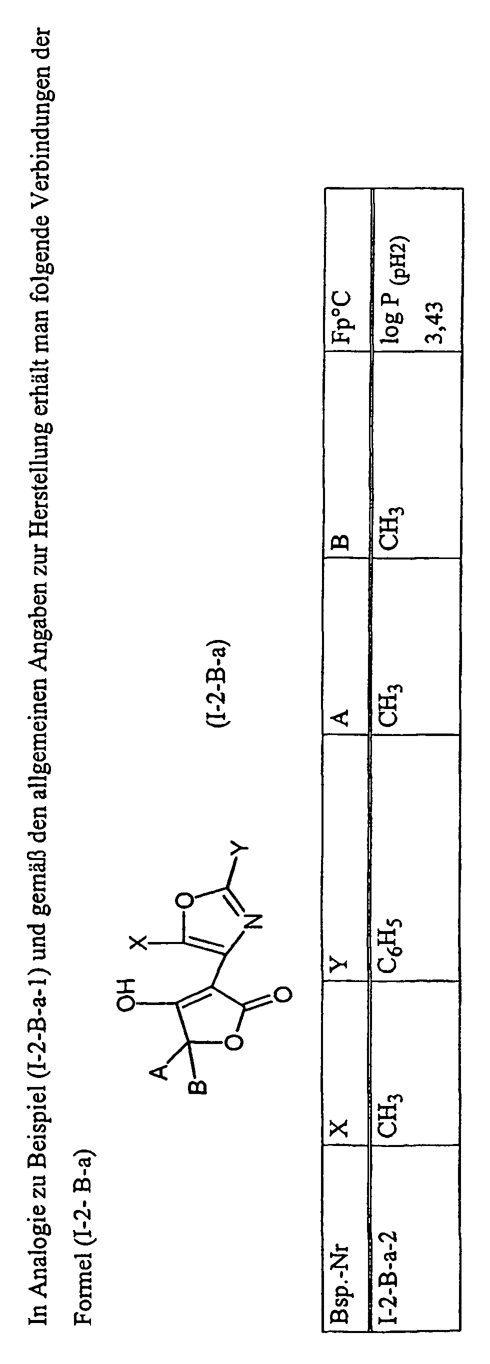

### Anwendungsbeispiele:

### Beispiel A

### Meloidogyne-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100%, dass keine Gallen gefunden wurden; 0% bedeutet, dass die Zahl der Gallen an den behandelten Pflanzen der der unbehandelten Kontrolle entspricht.

Bei diesem Test zeigen z. B. die folgenden Verbindungen der Herstellungsbeispiele gute Wirksamkeit: I-2-a-1 und I-2-a-2.

### Beispiel B

### Myzus-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Blattläuse abgetötet wurden; 0% bedeutet, dass keine Blattläuse abgetötet wurden.

Bei diesem Test zeigen z. B. die folgenden Verbindungen der Herstellungsbeispiele gute Wirksamkeit: I-2-a-1, I-1-a-5, I-1-a-6 und I-1-a-8.

### Beispiel C

### Phaedon-Larven-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100%, dass alle Käferlarven abgetötet wurden; 0% bedeutet, dass keine Käferlarven abgetötet wurden.

Bei diesem Test zeigen z. B. die folgenden Verbindungen der Herstellungsbeispiele gute Wirksamkeit: I-2-a-1, I-2-a-2, I-1-a-3, I-1-a-4, I-1-a-5, I-1-a-6, I-1-a-2, I-1-a-1, I-1-c-1 und I-1-a-8.

### Beispiel D

### Spodoptera frugiperda-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.
Bei diesem Test zeigen z. B. die folgenden Verbindungen der Herstellungsbeispiele gute Wirksamkeit: I-2-a-1, I-1-a-4, I-1-a-5, I-1-a-6, I-1-a-2, I-1-a-1 und I-1-c-1.

### Beispiel E

### Tetranychus-Test (OP-resistent/Tauchbehandlung)

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100%, dass alle Spinnmilben abgetötet wurden; 0% bedeutet, dass keine Spinnmilben abgetötet wurden.

Bei diesem Test zeigen z. B. die folgenden Verbindungen der Herstellungsbeispiele gute Wirksamkeit: I-2-a-2, I-1-a-4, I-1-a-5 und I-1-a-6.

### Beispiel F

### Post-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

### Beispiel G

### Pre-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

| pre-emergence | g ai/ha | Zuckerrüben | Raps | Alopecurus | Avena fatua | Lolium | Setaria | Matricaria |
|---|---|---|---|---|---|---|---|---|
| Bsp. I-2-a-1 | 125 | 0 | 0 | 90 | 100 | 100 | 100 | 100 |

| post- emergence | g ai/ha | Raps | Alopecurus | Avena fatua | Echinochloa | Setaria | Amaranthus | Chenopodium |
|---|---|---|---|---|---|---|---|---|
| Bsp. I-2-a-1 | 250 | 20 | 80 | 95 | 100 | 100 | 70 | 70 |

| pre-emergence | g ai/ha | Zuckerrüben | Raps | Avena fatua | Bromus | Lolium | Setaria |
|---|---|---|---|---|---|---|---|
| Bsp.I-2-a-2 | 250 | 0 | 0 | 90 | 90 | 100 | 100 |

| post- emergence | g ai/ha | Zuckerrüben | Soja | Avena fatua | Digitaria | Echinochloa | Setaria |
|---|---|---|---|---|---|---|---|
| Bsp.I-2-a-2 | 250 | 0 | 0 | 90 | 90 | 100 | 95 |

| post-emergence | g ai/ha | Alopecurus | Echinochloa | Amaranthus |
|---|---|---|---|---|
| Bsp. I-1-a-5 | 250 | 80 | 100 | 90 |

| post-emergence | g ai/ha | Zuckerrüben | Alopecurus | Avena fatua | Digitaria | Echinochloa |
|---|---|---|---|---|---|---|
| Bsp. I-1-a-6 | 125 | 0 | 90 | 90 | 95 | 90 |

| post-emergence | g ai/ha | Alopecurus | Echinochloa | Setaria | Amaranthus |
|---|---|---|---|---|---|
| Bsp.I-1-a-2 | 250 | 70 | 90 | 95 | 90 |

| post-emergence | g ai/ha | Alopecurus | Avena fatua | Echinochloa | Amaranthus | Sinapis |
|---|---|---|---|---|---|---|
| Bsp. I-1-a-1 | 250 | 80 | 80 | 95 | 70 | 80 |

| post-emergence | g ai/ha | Reis | Avena fatua | Digitaria | Echinochloa | Stellaria |
|---|---|---|---|---|---|---|
| Bsp. I-1-a-3 | 250 | 10 | 100 | 100 | 100 | 90 |

| post-emergence | g ai/ha | Alopecurus | Avena fatua | Echinochloa | Setaria |
|---|---|---|---|---|---|
| Bsp. I-1-c-1 | 250 | 80 | 95 | 95 | 90 |

| post-emergence | g ai/ha | Alopecurus | Avena fatua | Echinochloa | Setaria | Abutilon | Amaranthus |
|---|---|---|---|---|---|---|---|
| Bsp. I-1-a-4 | 250 | 95 | 100 | 100 | 100 | 80 | 95 |
| Bsp. I-1-b-1 | 250 | 95 | 100 | 100 | 100 | 80 | 80 |

### Beispiel H

### Grenzkonzentrations-Test / Bodeninsekten - Behandlung transgener Pflanzen

### Testinsekt: Diabrotica balteata - Larven im Boden

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel I

### Heliothis virescens - Test - Behandlung transgener Pflanzen

| | |
|---|---|
| Lösungsmittel: | 7 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 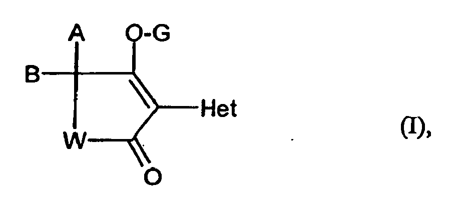
in welcher
W für N-D ⁽¹⁾, Sauerstoff ⁽²⁾ oder Schwefel ⁽³⁾ steht,
Het für jeweils gegebenenfalls substituiertes Thiazolyl, Oxazolyl oder Pyrazolyl steht,
A für Wasserstoff, jeweils gegebenenfalls durch Halogen substituiertes Alkyl, Alkenyl, Alkoxyalkyl, Polyalkoxyalkyl, Alkylthioalkyl, gesättigtes oder ungesättigtes, gegebenenfalls substituiertes Cycloalkyl, in welchem gegebenenfalls mindestens ein Ringatom durch ein Hetero-atom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
D für Wasserstoff oder einen gegebenenfalls substituierten Rest aus der Reihe Alkyl, Alkenyl, Alkinyl, Alkoxyalkyl, Polyalkoxyalkyl, Alkylthioalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen,
G für Wasserstoff (a) oder für eine der Gruppen 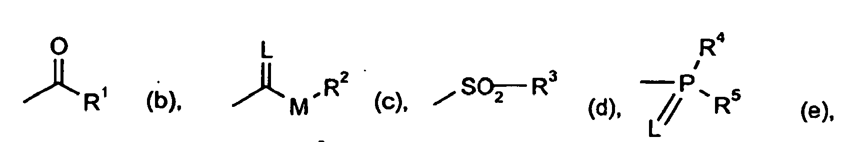 E (f) oder 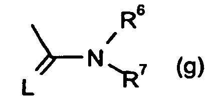
steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder
gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³ für Alkyl, Halogenalkyl oder für jeweils gegebenenfalls substituiertes Phenyl oder Benzyl steht,
R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio und für jeweils gegebenenfalls substituiertes Phenyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

2. Verbindungen der Formel (I) gemäß Anspruch 1, in welcher
Het für 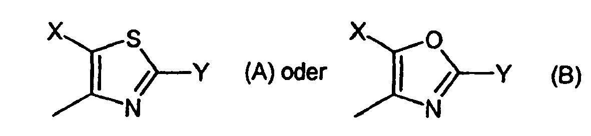 steht,
X für Wasserstoff, Halogen, C₁-C₆-Alkyl, C₁-C₄-Halogenalkyl, C₁-C₆-Alkoxy, C₃-C₆-Alkenyloxy, Nitro oder Cyano steht,
Y für Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Alkylsulfoxinyl oder für die Gruppe 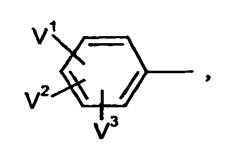
oder im Falle von Het = Thiazolyl ((I-1-A) bis (I-3-A)) auch für 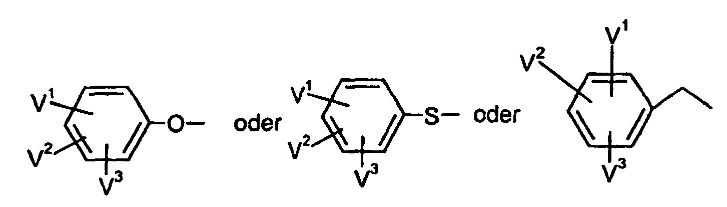 steht,
worin
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano, Phenoxy oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
V¹ und V² zusammen gemeinsam mit den Kohlenstoffatomen, an die sie gebunden sind für einen gegebenenfalls durch C₁-C₄-Alkyl oder Halogen substituierten gesättigten oder ungesättigten 5- oder 6-gliedrigen Cyclus stehen, in welchem gegebenenfalls ein bis drei Kohlenstoffatome durch Sauerstoff, Schwefel oder Stickstoff ersetzt sein können,
W für N-D ⁽¹⁾, Sauerstoff⁽²⁾ oder Schwefel ⁽³⁾ steht,
A für Wasserstoff oder jeweils gegebenenfalls durch Fluor oder Chlor substituiertes C₁-C₁₂-Alkyl, C₃-C₈-Alkenyl, C₁-C₆-Alkoxy-C₁-C₆-alkyl, Poly-C₁-C₆-alkoxy-C₁-C₆-alkyl, C₁-C₁₀-Alkylthio-C₁-C₆-alkyl, gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl, Hetaryl mit 5 bis 6 Ringatomen oder C₆- oder C₁₀-Aryl-C₁-C₆-alkyl steht,
B für Wasserstoff, C₁-C₆-Alkyl oder C₁-C₄-Alkoxy-C₁-C₄-alkyl steht, oder
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₄-Halogenalkyl, C₁-C₆-Alkoxy, C₁-C₆-Alkylthio, Halogen oder Phenyl substituiert sind oder
A, B und das Kohlenstoffatom, an das sie gebunden sind, für C₅-C₆-Cycloalkyl stehen, welches durch eine gegebenenfalls ein Sauerstoff- oder Schwefelatom enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis sechsgliedrigen Ring bildet der gegebenenfalls einfach bis vierfach durch C₁-C₄-Alkyl substituiert sein kann, oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen oder Cyano substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆- Halogenalkoxy, Cyano oder Nitro substituiertes Phenyl, Hetaryl mit 5 oder 6 Ringatomen, Phenyl-C₁-C₆-alkyl oder Hetaryl-C₁-C₆-alkyl mit 5 oder 6 Ringatomen stehen, oder
A und D gemeinsam für jeweils gegebenenfalls substituiertes C₃-C₆-Alkandiyl oder C₃-C₆-Alkendiyl stehen, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1-A) bis (I-1-B) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 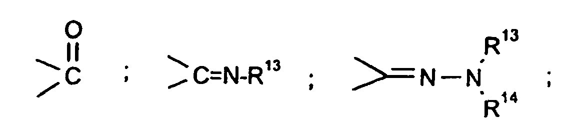 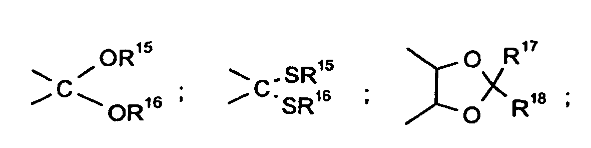 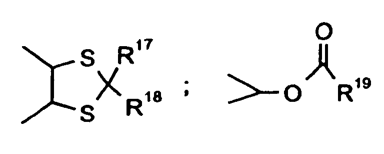
oder 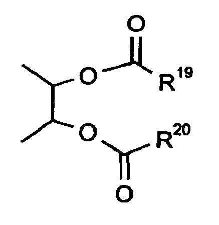
enthalten ist,
G für Wasserstoff (a) oder für eine der Gruppen 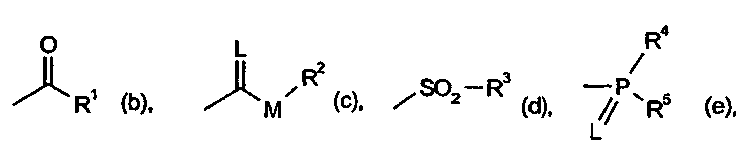 E (f) oder 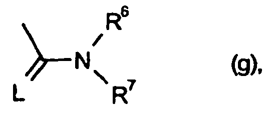 steht
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkyl-thio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen, C₁-C₆-Alkyl, C₁-C₂-Halogenalkyl oder C₁-C₄-Alkoxy substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl, für gegebenenfalls durch Halogen, C₁-C₆-Alkyl oder C₁-C₆-Alkoxy substituiertes C₃-C₈-Cycloalkyl in welchem gegebenenfalls ein Ringatom durch Sauerstoff ersetzt ist, oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
R¹³ für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy steht,
R¹⁴ für Wasserstoff oder C₁-C₈-Alkyl steht oder
R¹³ und R¹⁴ gemeinsam für C₄-C₆-Alkandiyl stehen,
R¹⁵ und R¹⁶ gleich oder verschieden sind und für C₁-C₆-Alkyl stehen oder
R¹⁵ und R¹⁶ gemeinsam für einen C₂-C₄-Alkandiylrest stehen, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
R¹⁷ und R¹⁸ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
R¹⁷ und R¹⁸ gemeinsam mit dem Kohlenstoffatom, an das sie gebunden sind, für eine Carbonylgruppe oder für gegebenenfalls durch Halogen, C₁-C₄-Alkyl oder C₁-C₄-Alkoxy substituiertes C₅- C₇-Cycloalkyl stehen, in dem gegebenenfalls eine Methylengruppe durch Sauerstoff oder Schwefel ersetzt ist,
R¹⁹ und R²⁰ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

3. Verbindungen der Formel (I) gemäß Anspruch 1, in welcher
Het für 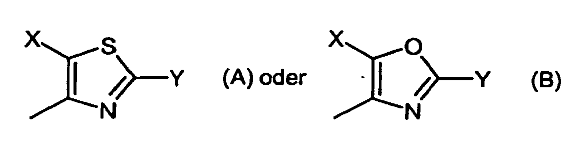 steht,
X für Wasserstoff, Chlor, Brom, C₁-C₄-Alkyl, C₁-C₂-Halogenalkyl, Nitro oder Cyano steht,
Y für Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfonyl oder für die Gruppe 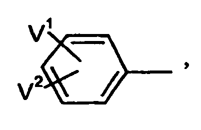
oder im Falle von Het = Thiazolyl ((I-1-A) bis (I-3-A)) auch für 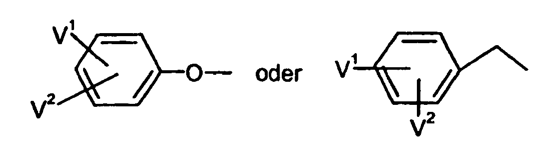 steht,
worin
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano, Phenoxy oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio steht,
V² für Wasserstoff, Fluor, Chlor, Brom, C₁-C₄-Alkyl, C₁-C₄-Alkoxy, C₁-C₂-Halogenalkyl oder C₁-C₂-Halogenalkoxy steht, oder
V¹ und V² zusammen gemeinsam mit den Kohlenstoffatomen, an die sie gebunden sind für einen gegebenenfalls durch Fluor oder Methyl substituierten 5- oder 6-gliedrigen Cyclus stehen, in welchem gegebenenfalls ein bis zwei Kohlenstoffatome durch Sauerstoff ersetzt sein können,
W für N-D ⁽¹⁾, Sauerstoff ⁽²⁾ oder Schwefel ⁽³⁾ steht,
A für Wasserstoff, jeweils gegebenenfalls durch Fluor substituiertes C₁-C₁₀-Alkyl, C₁-C₄-Alkoxy-C₁-C₄-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Phenyl-C₁-C₂-alkyl steht,
B für Wasserstoff oder C₁-C₄-Alkyl steht, oder
A, B und das Kohlenstoffatom an das sie gebunden sind, für gesättigtes C₅-C₇-Cycloalkyl stehen, worin gegebenenfalls ein Ringglied durch Sauerstoff oder Schwefel ersetzt ist und welches gegebenenfalls einfach oder zweifach durch C₁-C₆-Alkyl, C₅-C₆-Cycloalkyl, C₁-C₂-Halogenalkyl, C₁-C₆-Alkoxy, Fluor, Chlor oder Phenyl substituiert ist oder
A, B und das Kohlenstoffatom, an das sie gebunden sind, für C₅-C₆-Cycloalkyl stehen, welches durch eine gegebenenfalls ein Sauerstoff- oder Schwefelatom enthaltende Alkylendiyl- oder durch eine Alkylendioxyl- oder durch eine Alkylendithioyl-Grruppe substituiert ist, die mit dem Kohlenstoffatom, an das sie gebunden ist, einen weiteren fünf- oder sechsgliedrigen Ring bildet der gegebenenfalls einfach bis dreifach durch C₁-C₃-Alkyl substituiert sein kann, oder
A, B und das Kohlenstoffatom, an das sie gebunden sind, für C₅-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl, C₂-C₄-Alkendiyl, worin gegebenenfalls eine Methylengruppe durch Sauerstoff oder Schwefel ersetzt ist oder Butadiendiyl stehen,
D für Wasserstoff, für jeweils gegebenenfalls durch Fluor substituiertes C₁-C₁₀-Alkyl, C₃-C₆-Alkenyl, C₁-C₆-Alkoxy-C₂-C₄-alkyl oder C₁-C₆-Alkylthio-C₂-C₄-alkyl, für gegebenenfalls durch Fluor, C₁-C₄-Alkyl, C₁-C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₄-Alkyl, C₁-C₂-Halogenalkyl, C₁- C₄-Alkoxy oder C₁-C₂-Halogenalkoxy substituiertes Phenyl, oder Phenyl-C₁-C₄-alkyl steht, oder
A und D gemeinsam für gegebenenfalls substituiertes C₃-C₅-Alkandiyl stehen, in welchem gegebenenfalls eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
A und D gemeinsam mit den Atomen, an die sie gebunden sind, für eine der Gruppen AD-1 bis AD-10 stehen: 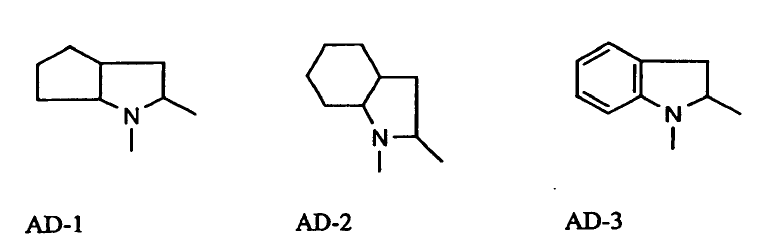 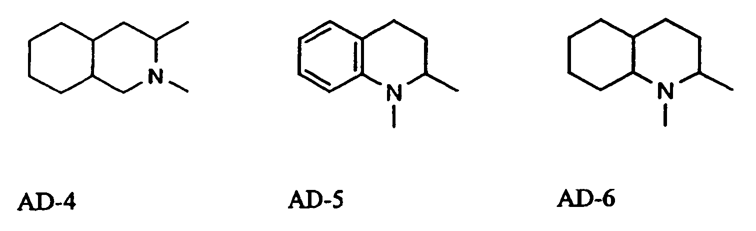 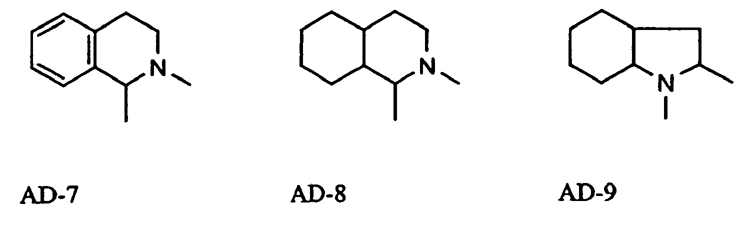 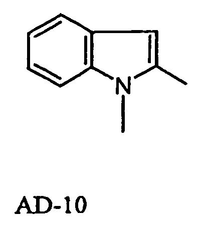
G für Wasserstoff (a) oder für eine der Gruppen 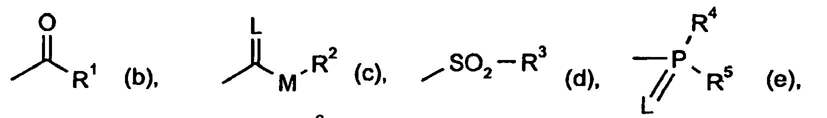 E (f) oder 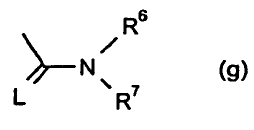 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom, C₁-C₄-Alkyl, Trifluormethyl oder C₁-C₂-Alkoxy substituiertes Pyrazolyl, Thiazolyl, Pyridyl, Pyrimidyl, Furanyl oder Thienyl steht,
R² für jeweils gegebenenfalls durch Fluor substituiertes C₁-C₁₆-Alkyl, C₂-C₁₆-Alkenyl oder C₁-C₆-Alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₂-Halogenalkyl oder C₁-C₂-Halogenalkoxy substituiertes Phenyl oder Benzyl steht,
R³ für gegebenenfalls durch Fluor substituiertes C₁-C₆-Alkyl oder für gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio steht,
R⁵ für C₁-C₆-Alkoxy oder C₁-C₆-Alkylthio steht,
R⁶ für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl steht,
R⁷ für Wasserstoff, C₁-C₆-Alkyl, C₃-C₆-Alkenyl steht, oder
R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
Het für 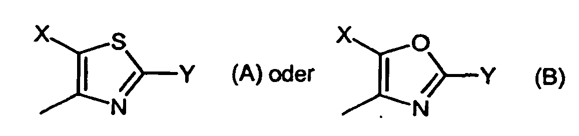 steht,
X für Wasserstoff, Chlor, Brom, Methyl, Ethyl, n-Propyl, iso-Propyl, n-Butyl oder iso-Butyl steht,
Y für Methyl, Ethyl, n-Propyl, i-Propyl, n-Butyl, i-Butyl, tert.-Butyl, Trifluormethyl oder für die Gruppe 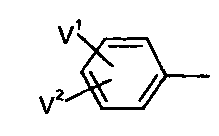
oder im Falle von Het = Thiazolyl ((I-1-A) bis (I-3-A)) auch für 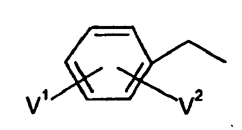 steht,
worin
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano, Phenoxy oder gegebenenfalls einfach oder zweifach durch Fluor, Chlor, Methyl, Methoxy, Trifluormethyl oder Trifluormethoxy substituiertes Phenyl steht,
V² für Wasserstoff, Fluor, Chlor, Methyl, Ethyl, n-Propyl, iso-Propyl, Methoxy, Ethoxy, Trifluormethyl oder Trifluormethoxy steht, oder
V¹ und V² zusammen für -O-CH₂-O-, -O-CF₂-O- oder -O-CF₂-CF₂-O-stehen,
W für N-D ⁽¹⁾, Sauerstoff⁽²⁾ oder Schwefel ⁽³⁾ steht,
A für Wasserstoff, für jeweils gegebenenfalls durch Fluor substituiertes C₁-C₈-Alkyl oder C₁-C₄-Alkoxy-C₁-C₂-alkyl, gegebenenfalls durch Fluor, Methyl, Ethyl oder Methoxy substituiertes C₃-C₆-Cycloalkyl steht, in welchem gegebenenfalls ein Ringglied durch Sauerstoff oder Schwefel ersetzt ist oder für jeweils gegebenenfalls durch Fluor, Chlor, Brom, Methyl, Ethyl, n-Propyl, iso-Propyl, tert.-Butyl, Methoxy, Ethoxy, Trifluormethyl, Trifluormethoxy, Cyano oder Nitro substituiertes Phenyl oder Benzyl,
B für Wasserstoff, Methyl oder Ethyl steht, oder
A, B und das Kohlenstoffatom an das sie gebunden sind, für gesättigtes C₅-C₆-Cycloalkyl stehen, in welchem gegebenenfalls ein Ringglied durch Sauerstoff oder Schwefel ersetzt ist und welches gegebenenfalls einfach oder zweifach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy oder iso-Butoxy substituiert ist oder
A, B und das Kohlenstoffatom, an das sie gebunden sind, für C₆-Cycloalkyl stehen, welches durch eine gegebenenfalls durch Methyl oder Ethyl einfach bis zweifach substituierte Alkylendioxyl-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
D für Wasserstoff, für jeweils gegebenenfalls durch Fluor substituiertes C₁-C₆-Alkyl, C₃-C₄-Alkenyl, C₁-C₄-Alkoxy-C₂-C₃-alkyl, C₁-C₄-Alkylthio-C₂-C₃-alkyl oder C₃-C₆-Cycloalkyl, in welchem gegebenenfalls eine Methylengruppe durch Sauerstoff oder Schwefel ersetzt ist oder für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl oder Benzyl steht,
oder
A und D gemeinsam für gegebenenfalls substituiertes C₃-C₄-Alkandiyl stehen, worin gegebenenfalls ein Kohlenstoffatom durch Sauerstoff oder Schwefel ersetzt ist und welches gegebenenfalls durch Methyl substituiert ist oder
A und D (im Fall der Verbindungen der Formel (I-1)) gemeinsam mit den Atomen, an die sie gebunden sind, für eine der folgenden Gruppen AD stehen: 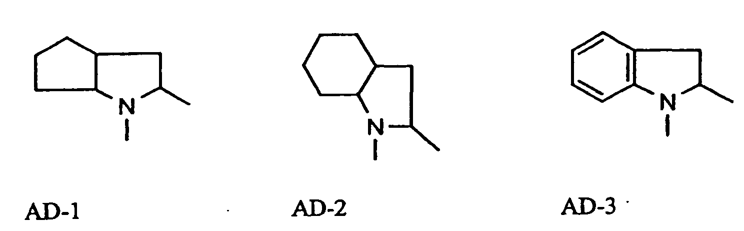 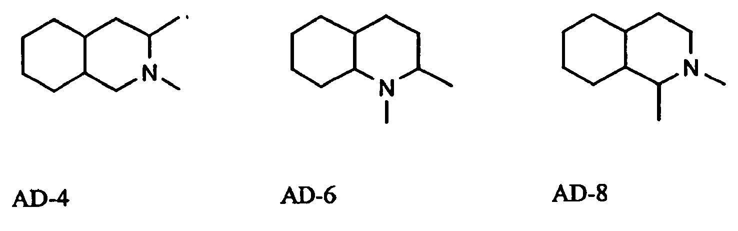 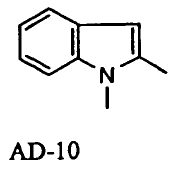
G für Wasserstoff (a) oder für eine der Gruppen 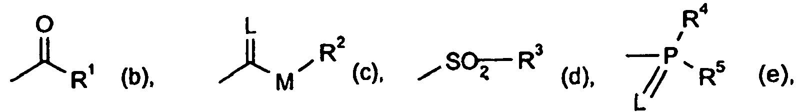 E (f) oder 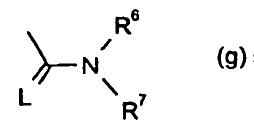 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor substituiertes C₁-C₁₄-Alkyl, C₂-C₁₄-Alkenyl, C₁-C₄-Alkoxy-C₁-C₂-alkyl, C₁-C₄-Alkylthio-C₁-C₂-alkyl oder jeweils gegebenenfalls durch Fluor, Chlor, Methyl, Ethyl oder Methoxy substituiertes Cyclopropyl, Cyclopentyl oder Cyclohexyl,
für gegebenenfalls einfach bis zweifach durch Fluor, Chlor, Brom, Cyano, Nitro, Methyl, Ethyl, tert.-Butyl, Methoxy, Ethoxy, Trifluormethyl oder Trifluormethoxy substituiertes Phenyl,
für jeweils gegebenenfalls einfach durch Fluor, Chlor, Brom oder Methyl substituiertes Thienyl oder Pyridyl steht,
R² für jeweils gegebenenfalls durch Fluor substituiertes C₁-C₈-Alkyl, C₂-C₈-Alkenyl oder C₁-C₄-Alkoxy-C₂-C₃-alkyl,
für gegebenenfalls durch Fluor, Chlor, Methyl, Ethyl, n-Propyl, iso-Propyl oder Methoxy substituiertes Cyclohexyl,
oder für jeweils gegebenenfalls durch Fluor, Chlor, Cyano, Nitro, Methyl, Ethyl, Methoxy, Trifluormethyl oder Trifluormethoxy substituiertes Phenyl oder Benzyl steht,
R³ für Methyl, Ethyl, n-Propyl oder gegebenenfalls durch Fluor, Chlor, Brom, Methyl, tert.-Butyl, Methoxy, Trifluormethyl, Trifluormethoxy, Cyano oder Nitro einfach substituiertes Phenyl steht,
R⁴ für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio steht,
R⁵ für Methoxy, Ethoxy, Methylthio oder Ethylthio steht,
R⁶ für C₁-C₄-Alkyl, C₃-C₆-Cycloalkyl, C₁-C₄-Alkoxy, C₃-C₄-Allcenyl, C₁-C₄-Alkoxy-C₁-C₄-alkyl, für gegebenenfalls durch Fluor, Chlor, Brom, Trifluormethyl, Methyl oder Methoxy substituiertes Phenyl, für gegebenenfalls durch Fluor, Chlor, Brom, Methyl, Trifluormethyl oder Methoxy substituiertes Benzyl steht,
R⁷ für Wasserstoff, Methyl, Ethyl, Propyl oder Allyl steht, oder
R⁶ und R⁷ zusammen für einen C₅-C₆-Alkylenrest stehen, in welchem gegebenenfalls eine Methylengruppe durch Sauerstoff oder Schwefel ersetzt ist.

5. Verbindungen der Formel (I) gemäß Anspruch 1, in welcher
Het für 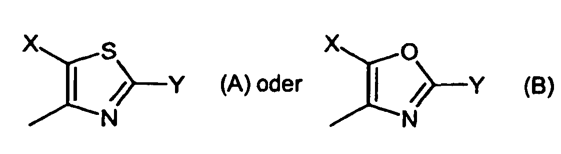 steht,
X für Wasserstoff, Chlor, Brom, Methyl, Ethyl, n-Propyl, oder iso-Propyl steht,
Y für Methyl, Ethyl, n-Propyl, i-Propyl, n-Butyl, i-Butyl, tert.-Butyl, Trifluormethyl oder für die Gruppe 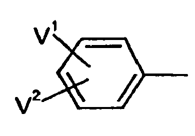
oder im Fall von Het = Thiazolyl ((I-1-A) bis (I-3-A)) auch für 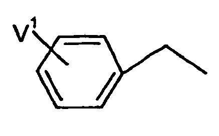 steht,
worin
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Phenoxy, für gegebenenfalls durch Chlor oder Trifluormethyl substituiertes Phenyl steht,
V² für Wasserstoff, Fluor, Chlor, Methyl, Ethyl, Methoxy oder Trifluormethyl steht, oder
V¹ und V² zusammen für -O-CH₂-O- oder -O-CF₂-O- stehen,
W für N-D ⁽¹⁾, Sauerstoff ⁽²⁾ oder Schwefel ⁽³⁾ steht,
A für Wasserstoff oder C₁-C₄-Alkyl oder Cyclopropyl steht,
B für Wasserstoff oder Methyl steht, oder
A, B und das Kohlenstoffatom, an das sie gebunden sind, für gesättigtes C₅-C₆-Cycloalkyl, in welchem gegebenenfalls ein Ringglied durch Sauerstoff oder Schwefel ersetzt ist und welches gegebenenfalls einfach oder zweifach durch Methyl, Ethyl, Propyl, Isopropyl, Trifluormethyl, Methoxy, Ethoxy, Propoxy, Butoxy oder iso-Butoxy substituiert ist oder für C₅-C₆-Cycloalkyl stehen, worin zwei nicht direkt benachbarte Kohlenstoffatome einen weiteren fünfgliedrigen Ring bilden,
D für Wasserstoff steht,
D auch für i-Propyl steht,
A und D zusammen mit den Atomen, an die sie gebunden sind, für Cyclohexyl stehen, in welchem ein Ringatom durch Schwefel ersetzt sein kann,
G für Wasserstoff (a) oder im Fall von Het = Thiazolyl (I-1-A) bis (1-3-A) für eine der Gruppen 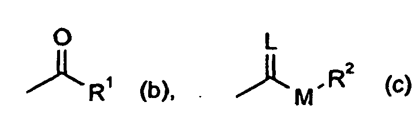 steht,
in welchen
L für Sauerstoff steht und
M für Sauerstoff steht,
R¹ für jeweils gegebenenfalls durch Fluor oder Chlor substituiertes C₁-C₄-Alkyl, C₂-C₄-Alkenyl, C₁-C₄-Alkoxy-C₁-C₂-alkyl, C₁-C₄- Alkylthio-C₁-C₂-alkyl oder gegebenenfalls durch Fluor, Chlor, Methyl, Ethyl oder Methoxy substituiertes Cyclopropyl oder Cyclohexyl,
für gegebenenfalls einfach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl,
für jeweils gegebenenfalls einfach durch Chlor oder Methyl substituiertes Thienyl oder Pyridyl steht,
R² für jeweils gegebenenfalls durch Fluor substituiertes C₁-C₄-Alkyl, C₂-C₄-Alkenyl oder C₁-C₄-Alkoxy-C₂-C₃-alkyl,
für gegebenenfalls durch Methyl, Ethyl, n-Propyl, iso-Propyl oder Methoxy substituiertes Cyclohexyl,
oder für jeweils gegebenenfalls einfach durch Fluor, Chlor, Cyano, Nitro, Methyl, Ethyl, Methoxy, Trifluormethyl oder Trifluormethoxy substituiertes Phenyl oder Benzyl steht.

6. Verbindungen der Formel (I) gemäß Anspruch 1, in welcher
Het für 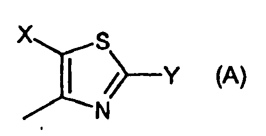 steht,
X für Methyl, Ethyl, n-Propyl, i-Propyl, Chlor oder Brom steht,
Y für Methyl, Ethyl, n-Propyl oder i-Propyl oder für die Gruppen 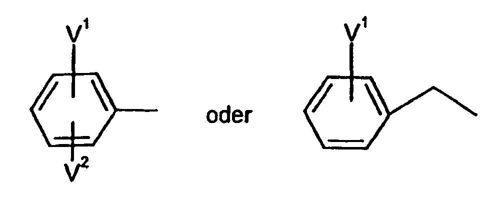 steht,
worin
V¹ für Wasserstoff, Brom, Chlor, Methyl, Trifluormethyl, Phenoxy oder t-Butyl oder für gegebenenfalls durch Chlor oder Trifluormethyl substituiertes Phenyl steht,
V² für Wasserstoff, Chlor, Fluor oder Methoxy steht oder
V¹ und V² zusammen für -O-CH₂-O- oder -O-CF₂-O- stehen,
W für N-D ⁽¹⁾, Sauerstoff ⁽²⁾ oder Schwefel ⁽³⁾ steht,
D für Wasserstoff oder i-Propyl steht,
A für Methyl, Ethyl, n- oder i-Propyl oder Cyclopropyl oder Wasserstoff steht,
B für Wasserstoff oder Methyl steht, oder
A und B und das Kohlenstoffatom, an das sie gebunden sind, für Cyclohexyl, in welchem gegebenenfalls ein Ringatom durch Sauerstoff ersetzt ist und welches gegebenenfalls einfach oder zweifach durch Methyl, Ethyl, Methoxy oder Ethoxy substituiert ist oder für Cyclohexyl stehen, worin zwei nicht direkt benachbarte Kohlenstoffatome einen weiteren 5-gliedrigen C-Ring bilden,
A und D zusammen mit den Atomen, an die sie gebunden sind, für Cyclohexyl stehen, in welchem ein Ringatom durch Schwefel ersetzt sein kann,
G für Wasserstoff (a) oder für eine der Gruppen 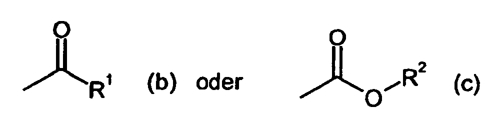 steht,
worin
R¹ für Methyl, Ethyl, n-Propyl oder i-Propyl oder für jeweils gegebenenfalls durch Chlor substituiertes Phenyl oder Pyridyl steht,
R² für Methyl, Ethyl, Phenyl, Benzyl, n- oder i-Propyl stehen.

7. Verbindungen der Formel (I) gemäß Anspruch 1, in welcher
Het für 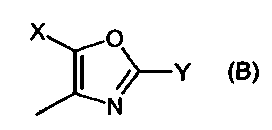 steht,
X für Methyl oder Ethyl steht,
Y für gegebenenfalls durch Chlor substituiertes Phenyl steht,
A für Methyl, Ethyl, n- oder i-Propyl steht,
B für Methyl steht, oder
A und B und das Kohlenstoffatom, an das sie gebunden sind, gemeinsam für Cyclohexyl stehen, welches gegebenenfalls durch Methoxy oder Methyl substituiert ist,
W für N-D oder Sauerstoff steht,
D für Wasserstoff steht,
G für Wasserstoff steht.

8. Verfahren zur Herstellung von Verbindungen der Formel (I) gemäß Anspruch 1, **dadurch gekennzeichnet, dass** man
(A) Verbindungen der Formel (I-1-A-a) bis (I-1-Ba) 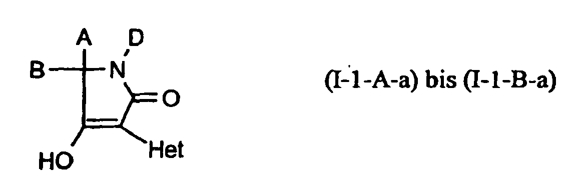
in welcher
A, B, D und Het die oben angegebenen Bedeutungen haben,
erhält, wenn man
Verbindungen der Formel (II) 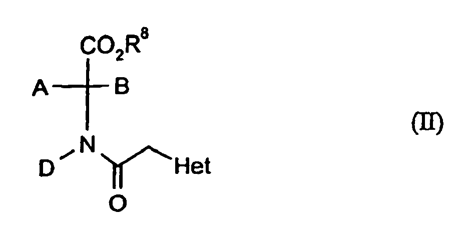
in welcher
A, B, D und Het die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-A-a) bis (I-2-B-a) 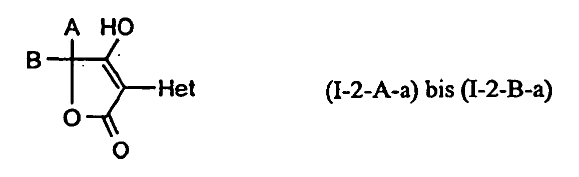
in welcher
A, B und Het die oben angegebenen Bedeutungen haben,
erhält, wenn man
Verbindungen der Formel (III) 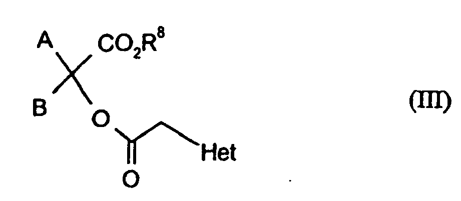
in welcher
A, B, Het und R⁸ die oben angegebenen Bedeutungen haben,
in Gegenwart eines Verdünnungsmittels und in Gegenwart einer Base intramolekular kondensiert,
(C) Verbindungen der Formel (I-3-A-a) bis (I-3-B-a) 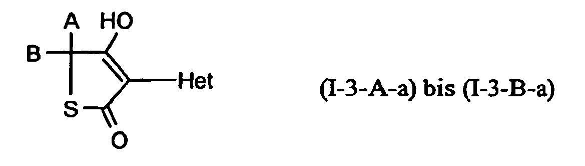
in welcher
A, B und Het die oben angegebenen Bedeutungen haben,
erhält, wenn man
Verbindungen der Formel (IV) 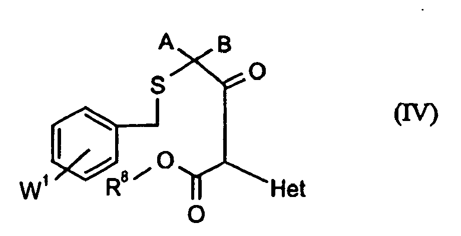
in welcher
A, B, Het und R⁸ die oben angegebenen Bedeutungen haben und
W¹ für Wasserstoff, Halogen, Alkyl oder Alkoxy steht,
gegebenenfalls in Gegenwart eines Verdünnungsmittels und in Gegenwart einer Säure intramolekular cyclisiert,
und gegebenenfalls die so erhaltenen Verbindungen der Formeln (I-1-A-a) bis (I-3-B-a), in welcher
A, B, D und Het jeweils die oben angegebenen Bedeutungen haben,
jeweils
(D) 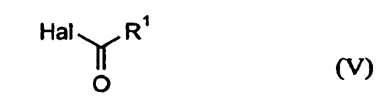
(α) mit Säurehalogeniden der Formel (V)in welcher
R¹ die oben angegebene Bedeutung hat und
Hal für Halogen steht
oder
(β) mit Carbonsäureanhydriden der Formel (VI)
R¹-CO-O-CO-R¹ (VI)
in welcher
R¹ die oben angegebene Bedeutung hat,
gegebenenfalls in Gegenwart eines Verdünnungsmittels und gegebenenfalls in Gegenwart eines Säurebindemittels umsetzt;
(E) mit Chlorameisensäureestem oder Chlorameisensäurethioestern der Formel (VII)
R²-M-CO-Cl (VII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(F) mit Chlormonothioameisensäureestern oder Chlordithioameisensäureestern der Formel (VIII) 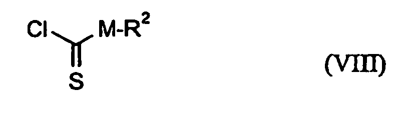
in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(G) mit Sulfonsäurechloriden der Formel (IX)
R³-SO₂-Cl (IX)
in welcher
R³ die oben angegebene Bedeutung hat,
gegebenenfalls in Gegenwart eines Verdünnungsmittels und gegebenenfalls in Gegenwart eines Säurebindemittels umsetzt,
(H) mit Phosphorverbindungen der Formel (X) 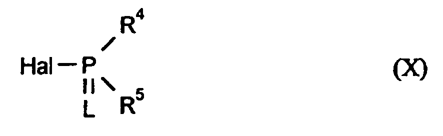
in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(I) mit Metallverbindungen oder Aminen der Formeln (XI) oder (XII)
Me(OR¹⁰)ₜ (XI) 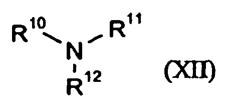
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(J)
(α) mit Isocyanaten oder Isothiocyanaten der Formel (XIII)
R⁶-N=C=L (XIII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XIV) 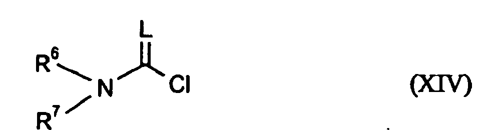
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

9. Verbindungen der Formel (II) 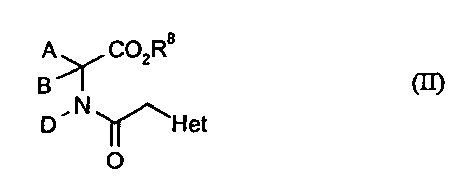
in welcher
A, B, D, Het und R⁸ die oben angegebenen Bedeutungen haben,
bitte einfügen S.197 a

10. Verbindungen der Formel (XVII) 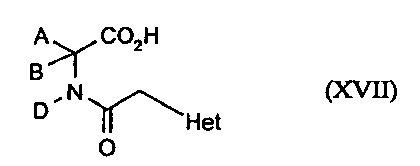
in welcher
A, B, D und Het die oben angegebenen Bedeutungen haben.

11. Verbindungen der Formel (XXI) 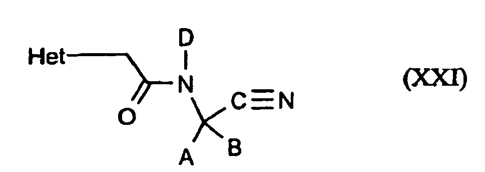
in welcher
A, B, D und Het die oben angegebenen Bedeutungen haben.
ausgenommen die Verbindungen

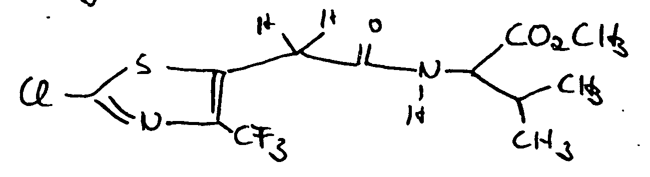

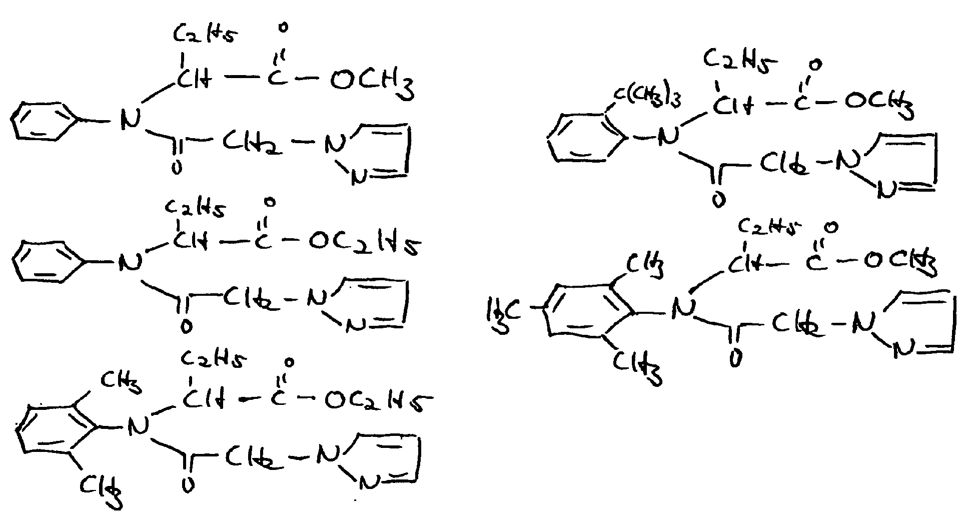

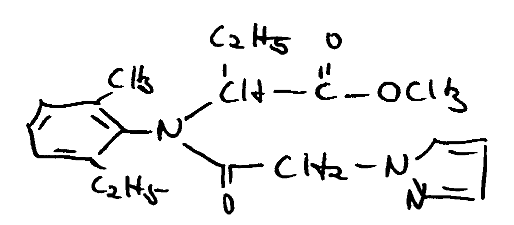

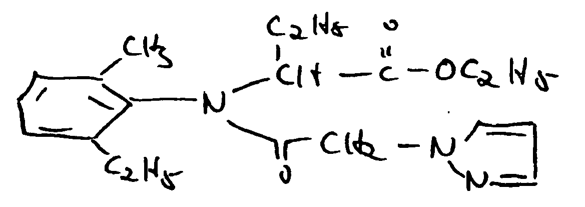

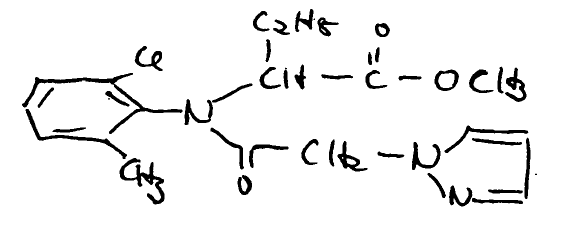

12. Verbindungen der Formel (III) 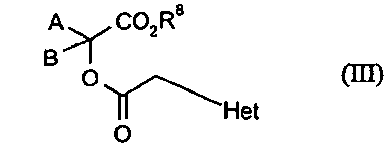
in welcher
A, B, Het und R⁸ die oben angegebenen Bedeutungen haben,
bitte einfügen S.198a

13. Verbindungen der Formel (IV) 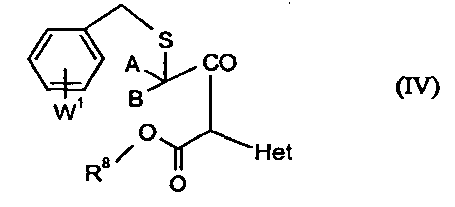
in welcher
A, B, W¹, Het und R⁸ die oben angegebenen Bedeutungen haben, und
W¹ für Wasserstoff, Halogen, Alkyl oder Alkoxy steht.

14. Schädlingsbekämpfungsmittel und Unkrautbekämpfungsmittel, **gekennzeichnet durch** einen Gehalt an mindestens einer Verbindung der Formel (I) gemäß Anspruch 1.

15. Verwendung von Verbindungen der Formel (I) gemäß Anspruch 1 zur Bekämpfung von Schädlingen und Unkräutern.
ausgenommen die Verbindung

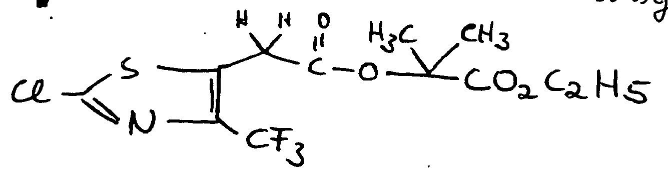

16. Verfahren zur Bekämpfung von Schädlingen und Unkräutern, **dadurch gekennzeichnet, dass** man Verbindungen der Formel (I) gemäß Anspruch 1 auf Schädlinge und/oder ihren Lebensraum einwirken lässt oder auf Unkräuter und/oder ihren Lebensraum einwirken lässt.

17. Verfahren zur Herstellung von Schädlingsbekämpfungsmitteln und Unkrautbekämpfungsmitteln, **dadurch gekennzeichnet, dass** man Verbindungen der Formel (I) gemäß Anspruch 1 mit Streckmitteln und/oder oberflächenaktiven Mitteln vermischt.

18. Verwendung von Verbindungen der Formel (I) gemäß Anspruch 1 zur Herstellung von Schädlingsbekämpfungsmitteln und Unkrautbekämpfungsmitteln.

## Claims

1. Compounds of the formula (I) 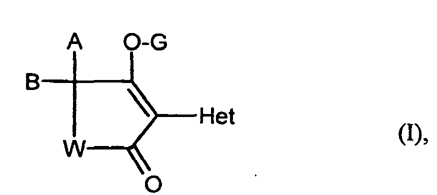
in which
W represents N-D ⁽¹⁾, oxygen ⁽²⁾ or sulphur ⁽³⁾,
Het represents in each case optionally substituted thiazolyl, oxazolyl or pyrazolyl,
A represents hydrogen, in each case optionally halogen-substituted alkyl, alkenyl, alkoxyalkyl, polyalkoxyalkyl, alkylthioalkyl, saturated or unsaturated optionally substituted cycloalkyl in which optionally at least one ring atom is replaced by a heteroatom, or in each case optionally halogen-, alkyl-, halogenoalkyl, alkoxy-, halogenoalkoxy-, cyano- or nitro-substituted aryl, arylalkyl or hetaryl,
B represents hydrogen, alkyl or alkoxyalkyl, or
A and B together with the carbon atom to which they are attached represent a saturated or unsaturated unsubstituted or substituted cycle which optionally contains at least one heteroatom,
D represents hydrogen or represents an optionally substituted radical from the group consisting of alkyl, alkenyl, alkinyl, alkoxyalkyl, polyalkoxyalkyl, alkylthioalkyl, saturated or unsaturated cycloalkyl in which optionally one or more ring members are replaced by heteroatoms, arylalkyl, aryl, hetarylalkyl or hetaryl or
A and D together with the atoms to which they are attached represent a saturated or unsaturated cycle which is unsubstituted or substituted in the A,D moiety and optionally contains at least one heteroatom,
G represents hydrogen (a) or represents one of the groups 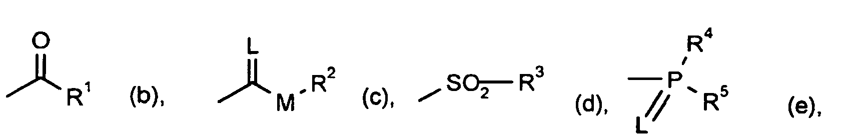 E(f) or 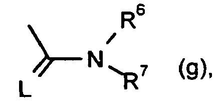
in which
E represents a metal ion equivalent or an ammonium ion,
L represents oxygen or sulphur,
M represents oxygen or sulphur,
R¹ represents in each case optionally halogen-substituted alkyl, alkenyl, alkoxyalkyl, alkylthioalkyl, polyalkoxyalkyl or optionally halogen-, alkyl- or alkoxy-substituted cycloalkyl which may be interrupted by at least one heteroatom, in each case optionally substituted phenyl, phenylalkyl, hetaryl, phenoxyalkyl or hetaryloxyalkyl,
R² represents in each case optionally halogen-substituted alkyl, alkenyl, alkoxyalkyl, polyalkoxyalkyl or represents in each case optionally substituted cycloalkyl, phenyl or benzyl,
R³ represents alkyl, halogenoalkyl or represents in each case optionally substituted phenyl or benzyl,
R⁴ and R⁵ independently of one another each represent in each case optionally halogen-substituted alkyl, alkoxy, alkylamino, dialkylamino, alkylthio, alkenylthio, cycloalkylthio and represents in each case optionally substituted phenyl, benzyl, phenoxy or phenylthio,
R⁶ and R⁷ independently of one another each represent hydrogen, in each case optionally halogen-substituted alkyl, cycloalkyl, alkenyl, alkoxy, alkoxyalkyl, represent optionally substituted phenyl, represent optionally substituted benzyl, or together with the N atom to which they are attached represent a cycle which is optionally interrupted by oxygen or sulphur.

2. Compounds of the formula (I) according to Claim 1 in which
Het represents 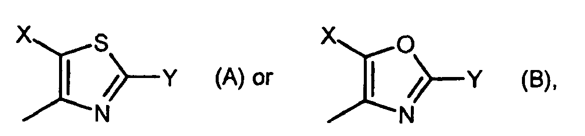
X represents hydrogen, halogen, C₁-C₆-alkyl, C₁-C₄-halogenoalkyl, C₁-C₆-alkoxy, C₃-C₆-alkenyloxy, nitro or cyano,
Y represents halogen, C₁-C₆-alkyl, C₁-C₆-halogenoalkyl, C₁-C₆-alkoxy, C₁-C₆-halogenoalkoxy, C₁-C₆-alkylthio, C₁-C₆-alkylsulphonyl, C₁-C₆-alkylsulphoxinyl or represents the group 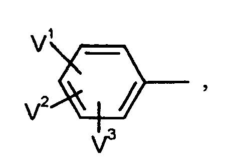
or in the case of Het = thiazolyl ((I-1-A) to (I-3-A)) also represents 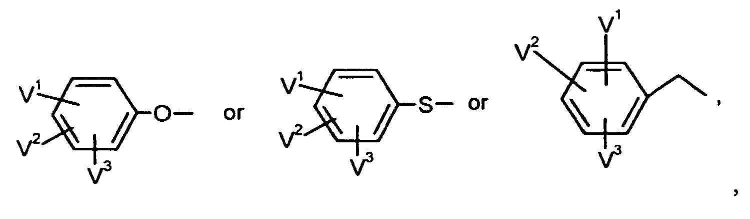
in which
V¹ represents hydrogen, halogen, C₁-C₁₂-alkyl, C₁-C₆-alkoxy, C₁-C₆-alkylthio, C₁-C₆-alkylsulphinyl, C₁-C₆-alkylsulphonyl, C₁-C₄-halogenoalkyl, C₁-C₄-halogenoalkoxy, nitro, cyano, phenoxy or represents phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-halogenoalkyl, C₁-C₄-halogenoalkoxy, nitro or cyano,
V² and V³ independently of one another each represent hydrogen, halogen, C₁-C₆-alkyl, C₁-C₆-alkoxy, C₁-C₄-halogenoalkyl or C₁-C₄-halogenoalkoxy,
V¹ and V² together with the carbon atoms to which they are attached represent an optionally C₁-C₄-alkyl- or halogen-substituted saturated or unsaturated 5- or 6-membered cycle in which optionally one to three carbon atoms may be replaced by oxygen, sulphur or nitrogen,
W represents N-D ⁽¹⁾, oxygen ⁽²⁾ or sulphur ⁽³⁾,
A represents hydrogen or in each case optionally fluorine- or chlorine- substituted C₁-C₁₂-alkyl, C₃-C₈-alkenyl, C₁-C₆-alkoxy-C₁-C₆-alkyl, poly-C₁-C₆-alkoxy-C₁-C₆-alkyl, C₁-C₁₀-alkylthio-C₁-C₆-alkyl, optionally fluorine-, chlorine-, C₁-C₆-alkyl- or C₁-C₆-alkoxy- substituted C₃-C₈-cycloalkyl in which optionally one or two not directly adjacent ring members are replaced by oxygen and/or sulphur or represents in each case optionally halogen-, C₁-C₆-alkyl-, C₁-C₆-halogenoalkyl-, C₁-C₆-alkoxy-, C₁-C₆-halogenoalkoxy-, cyano- or nitro-substituted C₆- or C₁₀-aryl, hetaryl having 5 or 6 ring atoms or C₆- or C₁₀-aryl-C₁-C₆-alkyl,
B represents hydrogen, C ₁-C₆-alkyl or C ₁-C₄-alkoxy-C₁-C₄-alkyl, or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₆-alkyl, C₃-C₈-cycloalkyl, C₁-C₄-halogenoalkyl, C₁-C₆-alkoxy, C₁-C₆-alkylthio, halogen or phenyl or
A, B and the carbon atom to which they are attached represent C₅-C₆-cycloalkyl which is substituted by an alkylenediyl group which optionally contains an oxygen or sulphur atom, or by an alkylenedioxyl or by an alkylenedithioyl group which, together with the carbon atom to which it is attached, forms a further five- or six-membered ring which may optionally be mono- to tetrasubstituted by C₁-C₄-alkyl, or
A, B and the carbon atom to which they are attached represent C₃-C₈-cycloalkyl or C₅-C₈-cycloalkenyl in which two substituents together with the carbon atoms to which they are attached represent in each case optionally C₁-C₆-alkyl-, C₁-C₆-alkoxy- or halogen-substituted C₂-C₆-alkanediyl, C₂-C₆-alkenediyl or C₄-C₆-alkanedienediyl in which optionally one methylene group is replaced by oxygen or sulphur,
D represents hydrogen, in each case optionally halogen- or cyano-substituted C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl, C₁-C₁₀-alkylthio-C₂-C₈-alkyl, optionally halogen-, C₁-C₄-alkyl-, C₁-C₄-alkoxy- or C₁-C₄-halogenoalkyl-substituted C₃-C₈-cycloalkyl in which optionally one ring member is replaced by oxygen or sulphur or in each case optionally halogen-, C₁-C₆-alkyl-, C₁-C₆-halogenoalkyl, C₁-C₆-alkoxy-, C₁-C₆-halogenoalkoxy-, cyano- or nitro-substituted phenyl, hetaryl having 5 or 6 ring atoms, phenyl-C₁-C₆-alkyl or hetaryl-C₁-C₆-alkyl having 5 or 6 ring atoms, or
A and D together represent in each case optionally substituted C₃-C₆-alkanediyl or C₃-C₆-alkenediyl in which optionally one methylene group is replaced by a carbonyl group, oxygen or sulphur, possible substituents being in each case:
halogen, hydroxyl, mercapto or in each case optionally halogen-substituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl grouping, C₃-C₆-alkenediyl grouping or a butadienyl grouping which is optionally substituted by C₁-C₆-alkyl or in which optionally two adjacent substituents together with the carbon atoms to which they are attached form a further saturated or unsaturated cycle having 5 or 6 ring atoms (in the case of the compound of the formula (I-1-A) to (I-1-B), A and D together with the atoms to which they are attached in this case represent, for example, the groups AD-1 to AD-10 mentioned further below) which may contain oxygen or sulphur, or which optionally contains one of the groups below 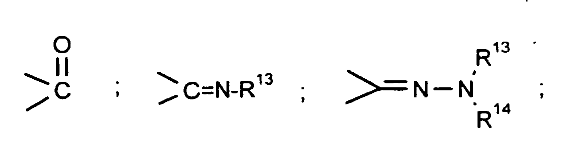 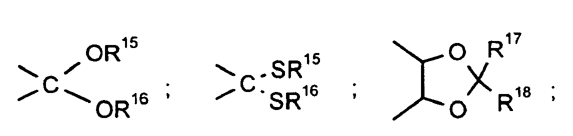 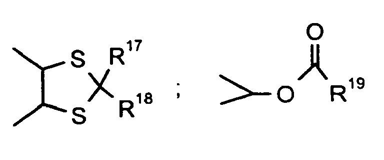 or 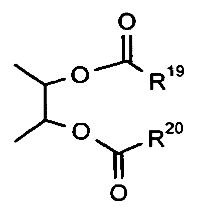
G represents hydrogen (a) or represents one of the groups 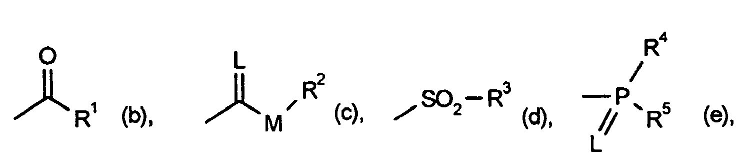 E (f) or 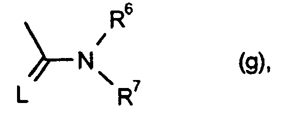
in which
E represents a metal ion equivalent or an ammonium ion,
L represents oxygen or sulphur and
M represents oxygen or sulphur,
R¹ represents in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur,
represents optionally halogen-, cyano-, nitro-, C₁-C₆-alkyl, C₁-C₆-alkoxy-, C₁-C₆-halogenoalkyl-, C₁-C₆-halogenoalkoxy-, C₁-C₆-alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆-halogenoalkyl- or C₁-C₆-halogenoalkoxy-substituted phenyl-C₁-C₆-alkyl,
represents optionally halogen-, C₁-C₆-alkyl-, C₁-C₂-halogenoalkyl- or C₁-C₄-alkoxy-substituted 5- or 6-membered hetaryl,
represents optionally halogen- or C₁-C₆-alkyl-substituted phenoxy-C₁-C₆-alkyl or
represents optionally halogen-, amino- or C₁-C₆-alkyl-substituted 5- or 6-membered hetaryloxy-C₁-C₆-alkyl,
R² represents in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl,
represents optionally halogen-, C₁-C₆-alkyl or C₁-C₆-alkoxy- substituted C₃-C₈-cycloalkyl in which optionally one ring atom is replaced by oxygen, or
represents in each case optionally halogen-, cyano-, nitro-, C₁-C₆-alkyl-, C₁-C₆-alkoxy-, C₁-C₆-halogenoalkyl- or C₁-C₆-halogenoalkoxy-substituted phenyl or benzyl,
R³ represents optionally halogen-substituted C₁-C₈-alkyl or represents in each case optionally halogen-, C₁-C₆-alkyl-, C₁-C₆-alkoxy-, C₁-C₄-halogenoalkyl-, C₁-C₄-halogenoalkoxy-, cyano- or nitro- substituted phenyl or benzyl,
R⁴ and R⁵ independently of one another each represent in each case optionally halogen-substituted C₁-C₈-alkyl, C₁-C₈-alkoxy, C₁-C₈-alkylamino, di-(C₁-C₈-alkyl)amino, C₁-C₈-alkylthio, C₂-C₈-alkenylthio, C₃-C₇-cycloalkylthio or represent in each case optionally halogen-, nitro-, cyano-, C₁-C₄-Alkoxy-, C₁-C₄-halogenoalkoxy-, C₁-C₄-alkylthio-, C₁-C₄-halogenoalkylthio-, C₁-C₄-alkyl- or C₁-C₄-halogenoalkyl-substituted phenyl, phenoxy or phenylthio,
R⁶ and R⁷ independently of one another each represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈-alkyl, represent optionally halogen-, C₁-C₈-halogenoalkyl-, C₁-C₈-alkyl- or C₁-C₈-alkoxy-substituted phenyl, optionally halogen-, C₁-C₈-alkyl-, C₁-C₈-halogenoalkyl- or C₁-C₈-alkoxy- substituted benzyl or together represent an optionally C₁-C₄-alkyl- substituted C₃-C₆-alkylene radical in which optionally one carbon atom is replaced by oxygen or sulphur,
R¹³ represents hydrogen, represents in each case optionally halogen- substituted C₁-C₈-alkyl or C₁-C₈-alkoxy, represents optionally halogen-, C₁-C₄-alkyl- or C₁-C₄-alkoxy-substituted C₃-C₈-cycloalkyl, in which optionally one methylene group is replaced by oxygen or sulphur, or represents in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenyl-C₁-C₄-alkyl or phenyl-C₁-C₄-alkoxy,
R¹⁴ represents hydrogen or C₁-C₈-alkyl or
R¹³ and R¹⁴ together represent C₄-C₆-alkanediyl,
R¹⁵ and R¹⁶ are identical or different and each represent C₁-C₆-alkyl or
R¹⁵ and R¹⁶ together represent a C₂-C₄-alkanediyl radical which is optionally substituted by C₁-C₆-alkyl, C₁-C₆-halogenoalkyl or by optionally halogen-, C₁-C₆-alkyl-, C₁-C₄-halogenoalkyl-, C₁-C₆-alkoxy-, C₁-C₄-halogenoalkoxy-, nitro- or cyano-substituted phenyl,
R¹⁷ and R¹⁸ independently of one another each represent hydrogen, represent optionally halogen-substituted C₁-C₈-alkyl or represent optionally halogen-, C₁-C₆-alkyl-, C₁-C₆-alkoxy-, C₁-C₄-halogeno-alkyl-, C₁-C₄-halogenoalkoxy-, nitro- or cyano-substituted phenyl or
R¹⁷ and R¹⁸ together with the carbon atom to which they are attached represent a carbonyl group or represent optionally halogen-, C₁-C₄-alkyl or C₁-C₄-alkoxy-substituted C₅-C₇-cycloalkyl in which optionally one methylene group is replaced by oxygen or sulphur,
R¹⁹ and R²⁰ independently of one another each represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl)amino or di-(C₃-C₁₀-alkenyl)amino.

3. Compounds of the formula (I) according to Claim 1 in which
Het represents 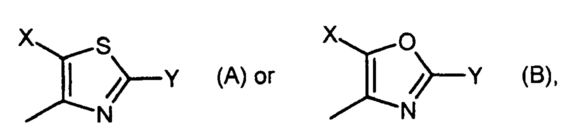
X represents hydrogen, chlorine, bromine, C₁-C₄-alkyl, C₁-C₂-halogenoalkyl, nitro or cyano,
Y represents chlorine, bromine, C₁-C₄-alkyl, C₁-C₄-halogenoalkyl, C₁-C₄-alkoxy, C₁-C₄-halogenoalkoxy, C₁-C₄-alkylthio, C₁-C₄-alkylsulphonyl or represents the group 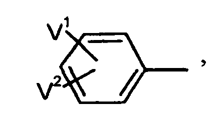
or in the case of Het = thiazolyl ((I-1-A) to (I-3-A)) also represents 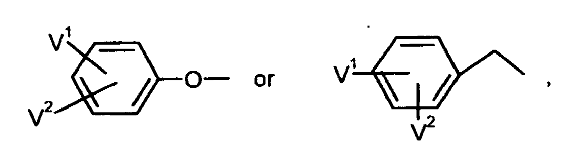
in which
V¹ represents hydrogen, fluorine, chlorine, bromine, C₁-C₆-alkyl, C₁-C₄-alkoxy, C₁-C₂-halogenoalkyl, C₁-C₂-halogenoalkoxy, nitro, cyano, phenoxy or represents phenyl, phenoxy, phenoxy-C₁-C₂-alkyl, phenyl-C₁-C₂-alkoxy, phenylthio-C₁-C₂-alkyl or phenyl-C₁-C₂-alkylthio, each of which is optionally mono- or disubstituted by fluorine, chlorine, bromine, C₁-C₄-alkyl, C₁-C₄-alkoxy, C₁-C₂-halogenoalkyl, C₁-C₂-halogenoalkoxy, nitro or cyano,
V² represents hydrogen, fluorine, chlorine, bromine, C₁-C₄-alkyl, C₁-C₄-alkoxy, C₁-C₂-halogenoalkyl or C₁-C₂-halogenoalkoxy, or
V¹ and V² together with the carbon atoms to which they are attached represent an optionally fluorine- or methyl-substituted 5- or 6-membered cycle in which optionally one or two carbon atoms may be replaced by oxygen,
W represents N-D⁽¹⁾, oxygen⁽²⁾ or sulphur ⁽³⁾,
A represents hydrogen, in each case optionally fluorine-substituted C₁-C₁₀-alkyl, C₁-C₄-alkoxy-C₁-C₄-alkyl, optionally fluorine-, chlorine-, C₁-C₄-alkyl- or C₁-C₄-alkoxy-substituted C₃-C₇-cycloalkyl in which optionally one ring member is replaced by oxygen or sulphur or in each case optionally fluorine-, chlorine-, bromine-, C₁-C₄-alkyl-, C₁-C₄-halogenoalkyl-, C₁-C₄-alkoxy- or C₁-C₄-halogenoalkoxy-substituted phenyl or phenyl-C₁-C₂-alkyl,
B represents hydrogen or C₁-C₄-alkyl, or
A, B and the carbon atom to which they are attached represent saturated C₅-C₇-cycloalkyl in which optionally one ring member is replaced by oxygen or sulphur and which is optionally mono- or disubstituted by C₁-C₆-alkyl, C₅-C₆-cycloalkyl, C₁-C₂-halogenoalkyl, C₁-C₆-alkoxy, fluorine, chlorine or phenyl or
A, B and the carbon atom to which they are attached represent C₅-C₆-cycloalkyl which is substituted by an alkylenediyl group which optionally contains an oxygen or sulphur atom or by an alkylenedioxyl or by an alkylenedithioyl group which together with the carbon atom to which it is attached forms a further five- or six-membered ring which may optionally be mono- to trisubstituted by C₁-C₃-alkyl, or
A, B and the carbon atom to which they are attached represent C₅-C₆-cycloalkyl or C₅-C₆-cycloalkenyl in which two substituents together with the carbon atoms to which they are attached represent in each case optionally C₁-C₅-alkyl-, C₁-C₅-alkoxy-, fluorine-, chlorine- or bromine-substituted C₂-C₄-alkanediyl, C₂-C₄-alkenediyl in which optionally one methylene group is replaced by oxygen or sulphur or represent butadienediyl,
D represents hydrogen, represents in each case optionally fluorine- substituted C₁-C₁₀-alkyl, C₃-C₆-alkenyl, C₁-C₆-alkoxy-C₂-C₄-alkyl or C₁-C₆-alkylthio-C₂-C₄-alkyl, represents optionally fluorine-, C₁-C₄-alkyl, C₁-C₄-alkoxy or C₁-C₂-halogenoalkyl-substituted C₃-C₇-cycloalkyl, in which optionally one methylene group is replaced by oxygen or sulphur or represents in each case optionally fluorine-, chlorine-, bromine-, C₁-C₄-alkyl-, C₁-C₂-halogenoalkyl-, C₁-C₄-alkoxy- or C₁-C₂-halogenoalkoxy-substituted phenyl or phenyl-C₁-C₄-alkyl, or
A and D together represent optionally substituted C₃-C₅-alkanediyl in which optionally one methylene group may be replaced by a carbonyl group, oxygen or sulphur, possible substituents being hydroxyl, C₁-C₆-alkyl or C₁-C₄-alkoxy, or
A and D together with the atoms to which they are attached represent one of the groups AD-1 to AD-10: 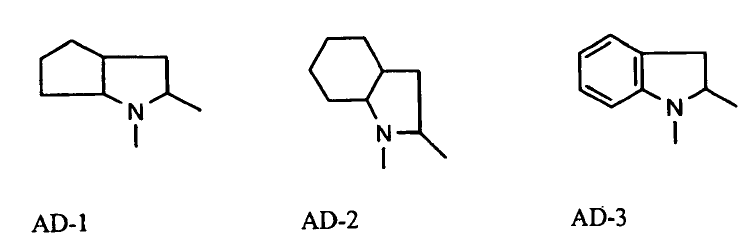 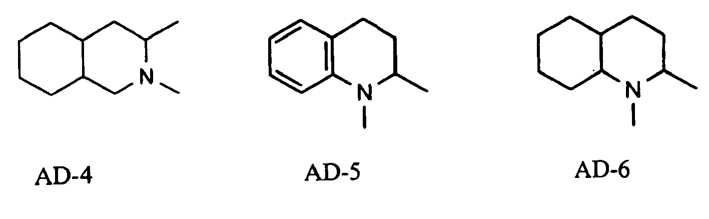 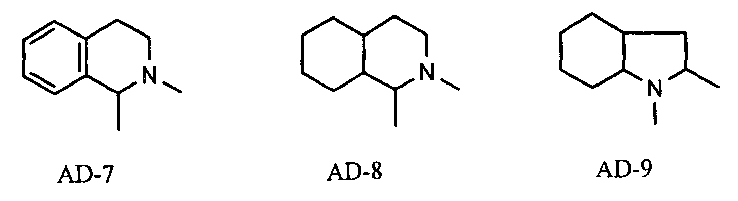 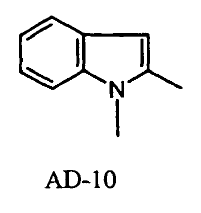
G represents hydrogen (a) or represents one of the groups 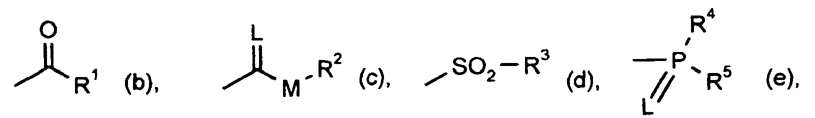 E (f) or 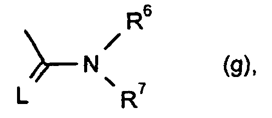
in which
E represents a metal ion equivalent or an ammonium ion,
L represents oxygen or sulphur and
M represents oxygen or sulphur,
R¹ represents in each case optionally fluorine- or chlorine-substituted C₁-C₁₆-alkyl, C₂-C₁₆-alkenyl, C₁-C₆-alkoxy-C₁-C₄-alkyl, C₁-C₆-alkylthio-C₁-C₄-alkyl or optionally fluorine-, chlorine-, C₁-C₅-alkyl- or C₁-C₅-alkoxy-substituted C₃-C₇-cycloalkyl in which optionally one or two not directly adjacent ring members are replaced by oxygen and/or sulphur,
represents optionally fluorine-, chlorine-, bromine-, cyano-, nitro-, C₁-C₄-alkyl-, C₁-C₄-alkoxy-, C₁-C₃-halogenoalkyl-, C₁-C₃-halogenoalkoxy-, C₁-C₄-alkylthio- or C₁-C₄-alkylsulphonyl-substituted phenyl,
represents optionally fluorine-, chlorine-, bromine-, C₁-C₄-alkyl-, C₁-C₄-alkoxy-, C₁-C₃-halogenoalkyl- or C₁-C₃-halogenoalkoxy- substituted phenyl-C₁-C₄-alkyl,
represents in each case optionally fluorine-, chlorine-, bromine-, C₁-C₄-alkyl-, trifluoromethyl- or C₁-C₂-alkoxy-substituted pyrazolyl, thiazolyl, pyridyl, pyrimidyl, furanyl or thienyl,
R² represents in each case optionally fluorine-substituted C₁-C₁₆-alkyl, C₂-C₁₆-alkenyl or C₁-C₆-alkoxy-C₂-C₆-alkyl,
represents optionally fluorine-, chlorine-, C₁-C₄-alkyl- or C₁-C₄-alkoxy-substituted C₃-C₇-cycloalkyl or
represents in each case optionally fluorine-, chlorine-, bromine-, cyano-, nitro-, C₁-C₄-alkyl-, C₁-C₃-alkoxy-, C₁-C₂-halogenoalkyl- or C₁-C₂-halogenoalkoxy-substituted phenyl or benzyl,
R³ represents optionally fluorine-substituted C₁-C₆-alkyl or represents optionally fluorine-, chlorine-, bromine-, C₁-C₄-alkyl-, C₁-C₄-alkoxy-, C₁-C₃-halogenoalkyl-, C₁-C₃-halogenoalkoxy-, cyano- or nitro-substituted phenyl,
R⁴ represents C₁-C₆-alkyl, C₁-C₆-alkoxy, C₁-C₆-alkylamino, di-(C₁-C₆-alkyl)amino, C₁-C₆-alkylthio, C₃-C₄-alkenylthio, C₃-C₆-cycloalkylthio or represents in each case optionally fluorine-, chlorine-, bromine-, nitro-, cyano-, C₁-C₃-alkoxy-, C₁-C₃-halogenoalkoxy-, C₁-C₃-alkylthio-, C₁-C₃-halogenoalkylthio-, C₁-C₃-alkyl- or C₁-C₃-halogenoalkyl-substituted phenyl, phenoxy or phenylthio,
R⁵ represents C₁-C₆-alkoxy or C₁-C₆-alkylthio,
R⁶ represents C₁-C₆-alkyl, C₃-C₆-cycloalkyl, C₁-C₆-alkoxy, C₃-C₆-alkenyl, C₁-C₆-alkoxy-C₁-C₆-alkyl, represents optionally fluorine-, chlorine-, bromine-, C₁-C₃-halogenoalkyl-, C₁-C₄-alkyl- or C₁-C₄-alkoxy-substituted phenyl, represents optionally fluorine-, chlorine-, bromine-, C₁-C₄-alkyl-, C₁-C₃-halogenoalkyl- or C₁-C₄-alkoxy-substituted benzyl,
R⁷ represents hydrogen, C₁-C₆-alkyl, C₃-C₆-alkenyl, or
R⁶ and R⁷ together represent an optionally methyl- or ethyl-substituted C₄-C₅-alkylene radical in which optionally one methylene group is replaced by oxygen or sulphur.

4. Compounds of the formula (I) according to Claim 1 in which
Het represents 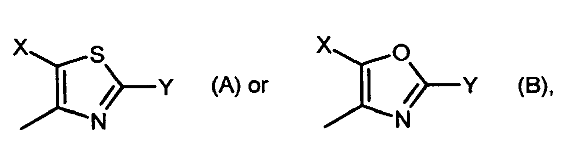
X represents hydrogen, chlorine, bromine, methyl, ethyl, n-propyl, iso-propyl, n-butyl or iso-butyl,
Y represents methyl, ethyl, n-propyl, i-propyl, n-butyl, i-butyl, tert-butyl, trifluoromethyl or represents the group 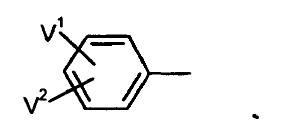
or in the case of Het = thiazolyl ((I-1-A) to (I-3-A)) also represents 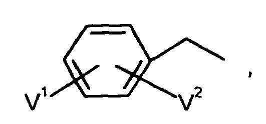
in which
V¹ represents hydrogen, fluorine, chlorine, bromine, methyl, ethyl, n-propyl, iso-propyl, n-butyl, iso-butyl, tert-butyl, methoxy, ethoxy, n-propoxy, iso-propoxy, trifluoromethyl, trifluoromethoxy, nitro, cyano, phenoxy or phenyl which is optionally mono- or disubstituted by fluorine, chlorine, methyl, methoxy, trifluoromethyl or trifluoromethoxy,
V² represents hydrogen, fluorine, chlorine, methyl, ethyl, n-propyl, iso-propyl, methoxy, ethoxy, trifluoromethyl or trifluoromethoxy, or
V¹ and V² together represent -O-CH₂-O-, -O-CF₂-O- or -O-CF₂-CF₂-O-,
W represents N-D⁽¹⁾, oxygen⁽²⁾ or sulphur ⁽³⁾,
A represents hydrogen, represents in each case optionally fluorine-substituted C₁-C₈-alkyl or C₁-C₄-alkoxy-C₁-C₂-alkyl, optionally fluorine-, methyl-, ethyl- or methoxy-substituted C₃-C₆-cycloalkyl in which optionally one ring member is replaced by oxygen or sulphur or represents in each case optionally fluorine-, chlorine-, bromine-, methyl-, ethyl-, n-propyl-, iso-propyl-, tert-butyl-, methoxy-, ethoxy-, trifluoromethyl-, trifluoromethoxy-, cyano- or nitro-substituted phenyl or benzyl,
B represents hydrogen, methyl or ethyl, or
A, B and the carbon atom to which they are attached represent saturated C₅-C₆-cycloalkyl in which optionally one ring member is replaced by oxygen or sulphur and which is optionally mono- or disubstituted by methyl, ethyl, propyl, isopropyl, butyl, isobutyl, sec-butyl, tert-butyl, trifluoromethyl, methoxy, ethoxy, propoxy, iso-propoxy, butoxy or iso-butoxy or
A, B and the carbon atom to which they are attached represent C₆-cycloalkyl which is substituted by an alkylenedioxyl group which is optionally mono- or disubstituted by methyl or ethyl and which together with the carbon atom to which it is attached forms a further five- or six-membered ring or
A, B and the carbon atom to which they are attached represent C₅-C₆-cycloalkyl or C₅-C₆-cycloalkenyl in which two substituents together with the carbon atoms to which they are attached represent C₂-C₄-alkanediyl or C₂-C₄-alkenediyl in which in each case optionally one methylene group is replaced by oxygen or sulphur, or represent butadienediyl,
D represents hydrogen, represents in each case optionally fluorine-substituted C₁-C₆-alkyl, C₃-C₄-alkenyl, C₁-C₄-alkoxy-C₂-C₃-alkyl, C₁-C₄-alkylthio-C₂-C₃-alkyl or C₃-C₆-cycloalkyl in which optionally one methylene group is replaced by oxygen or sulphur or represents in each case optionally fluorine-, chlorine-, methyl-, ethyl-, n-propyl-, iso-propyl-, methoxy-, ethoxy-, trifluoromethyl- or trifluoromethoxy-substituted phenyl or benzyl,
or
A and D together represent optionally substituted C₃-C₄-alkanediyl in which optionally one carbon atom is replaced by oxygen or sulphur and which is optionally substituted by methyl or
A and D (in the case of the compounds of the formula (I-1)) together with the atoms to which they are attached represent one of the groups AD below: 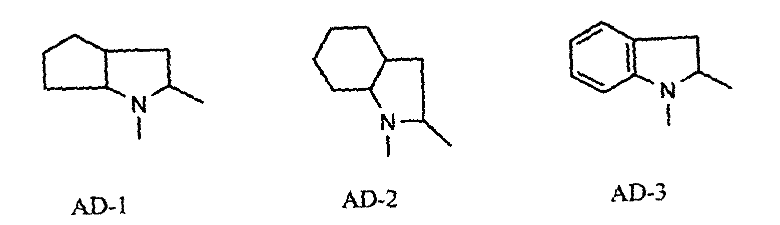 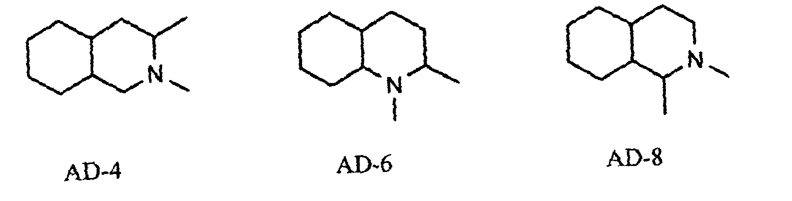 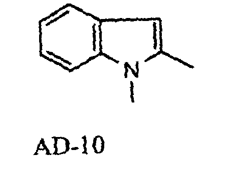
G represents hydrogen (a) or represents one of the groups 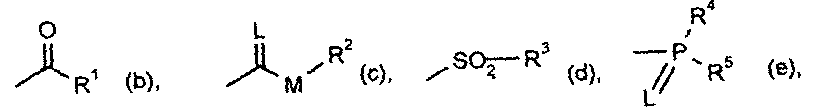 E (f) or 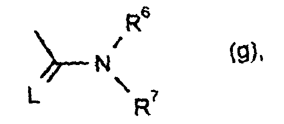
in which
E represents a metal ion equivalent or an ammonium ion,
L represents oxygen or sulphur and
M represents oxygen or sulphur,
R¹ represents in each case optionally fluorine-substituted C₁-C₁₄-alkyl, C₂-C₁₄-alkenyl, C₁-C₄-alkoxy-C₁-C₂-alkyl, C₁-C₄-alkylthio-C₁-C₂-alkyl or in each case optionally fluorine-, chlorine-, methyl-, ethyl- or methoxy-substituted cyclopropyl, cyclopentyl or cyclohexyl,
represents phenyl which is optionally mono- or disubstituted by fluorine, chlorine, bromine, cyano, nitro, methyl, ethyl, tert-butyl, methoxy, ethoxy, trifluoromethyl or trifluoromethoxy,
represents thienyl or pyridyl, each of which is optionally monosubstituted by fluorine, chlorine, bromine or methyl,
R² represents in each case optionally fluorine-substituted C₁-C₈-alkyl, C₂-C₈-alkenyl or C₁-C₄-alkoxy-C₂-C₃-alkyl,
represents optionally fluorine-, chlorine-, methyl-, ethyl-, n-propyl-, iso-propyl- or methoxy-substituted cyclohexyl,
or represents in each case optionally fluorine-, chlorine-, cyano-, nitro-, methyl-, ethyl-, methoxy-, trifluoromethyl- or trifluoromethoxy-substituted phenyl or benzyl,
R³ represents methyl, ethyl, n-propyl or represents phenyl which is optionally monosubstituted by fluorine, chlorine, bromine, methyl, tert-butyl, methoxy, trifluoromethyl, trifluoromethoxy, cyano or nitro,
R⁴ represents C₁-C₄-alkyl, C₁-C₄-alkoxy, C₁-C₄-alkylamino, di-(C₁-C₄-alkyl)amino, C₁-C₄-alkylthio or represents in each case optionally fluorine-, chlorine-, bromine-, nitro-, cyano-, C₁-C₂-alkoxy-, C₁-C₂-fluoroalkoxy-, C₁-C₂-alkylthio-, C₁-C₂-fluoroalkylthio- or C₁-C₃-alkyl-substituted phenyl, phenoxy or phenylthio,
R⁵ represents methoxy, ethoxy, methylthio or ethylthio,
R⁶ represents C₁-C₄-alkyl, C₃-C₆-cycloalkyl, C₁-C₄-alkoxy, C₃-C₄-alkenyl, C₁-C₄-alkoxy-C₁-C₄-alkyl, represents optionally fluorine-, chlorine-, bromine-, trifluoromethyl-, methyl- or methoxy- substituted phenyl, represents optionally fluorine-, chlorine-, bromine-, methyl-, trifluoromethyl- or methoxy-substituted benzyl,
R⁷ represents hydrogen, methyl, ethyl, propyl or allyl, or
R⁶ and R⁷ together represent a C₅-C₆-alkylene radical in which optionally one methylene group is replaced by oxygen or sulphur.

5. Compounds of the formula (I) according to Claim 1 in which
Het represents 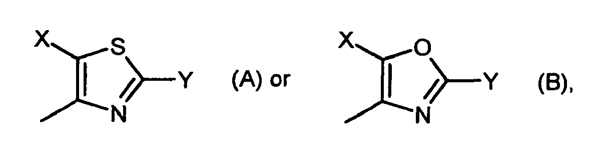
X represents hydrogen, chlorine, bromine, methyl, ethyl, n-propyl, or isopropyl,
Y represents methyl, ethyl, n-propyl, i-propyl, n-butyl, i-butyl, tert-butyl, trifluoromethyl or represents the group 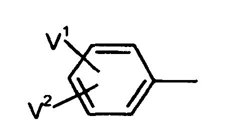
or in the case of Het = thiazolyl ((I-1-A) or (I-3-A)) also represents 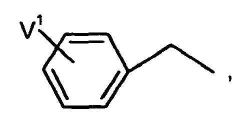
in which
V¹ represents hydrogen, fluorine, chlorine, bromine, methyl, ethyl, n-propyl, iso-propyl, n-butyl, iso-butyl, tert-butyl, methoxy, ethoxy, n-propoxy, iso-propoxy, trifluoromethyl, trifluoromethoxy, phenoxy, represents optionally chlorine- or trifluoromethyl-substituted phenyl,
V² represents hydrogen, fluorine, chlorine, methyl, ethyl, methoxy or trifluoromethyl, or
V¹ and V² together represent -O-CH₂-O- or -O-CF₂-O- ,
W represents N-D⁽¹⁾, oxygen⁽²⁾ or sulphur ⁽³⁾,
A represents hydrogen or C₁-C₄-alkyl or cyclopropyl,
B represents hydrogen or methyl, or
A, B and the carbon atom to which they are attached represent saturated C₅-C₆-cycloalkyl in which optionally one ring member is replaced by oxygen or sulphur, and which is optionally mono- or disubstituted by methyl, ethyl, propyl, isopropyl, trifluoromethyl, methoxy, ethoxy, propoxy, butoxy or iso-butoxy, or represents C₅-C₆-cycloalkyl in which two not directly adjacent carbon atoms form a further five-membered ring,
D represents hydrogen,
D also represents i-propyl,
A and D together with the atoms to which they are attached represent cyclohexyl in which one ring atom may be replaced by sulphur,
G represents hydrogen (a) or in the case of Het = thiazolyl ((I-1-A) to (I-3-A)) represents one of the groups 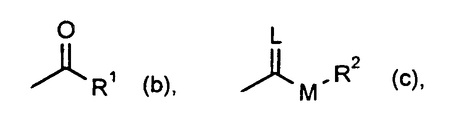
in which
L represents oxygen and
M represents oxygen,
R¹ represents in each case optionally fluorine- or chlorine-substituted C₁-C₄-alkyl, C₂-C₄-alkenyl, C₁-C₄-alkoxy-C₁-C₂-alkyl, C₁-C₄-alkylthio-C₁-C₂-alkyl or optionally fluorine-, chlorine-, methyl-, ethyl- or methoxy-substituted cyclopropyl or cyclohexyl,
represents phenyl which is optionally monosubstituted by fluorine, chlorine, bromine, cyano, nitro, methyl, ethyl, n-propyl, i-propyl, tert-butyl, methoxy, ethoxy, i-propoxy, tert-butoxy, trifluoromethyl or trifluoromethoxy,
represents thienyl or pyridyl, each of which is optionally monosubstituted by chlorine or methyl,
R² represents optionally in each case fluorine-substituted C₁-C₄-alkyl, C₂-C₄-alkenyl or C₁-C₄-alkoxy-C₂-C₃-alkyl,
represents optionally methyl-, ethyl-, n-propyl-, iso-propyl- or methoxy-substituted cyclohexyl,
or represents phenyl or benzyl, each of which is optionally monosubstituted by fluorine, chlorine, cyano, nitro, methyl, ethyl, methoxy, trifluoromethyl or trifluoromethoxy.

6. Compounds of the formula (I) according to Claim 1 in which
Het represents 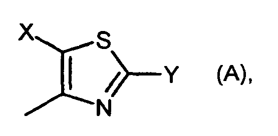
X represents methyl, ethyl, n-propyl, i-propyl, chlorine or bromine,
Y represents methyl, ethyl, n-propyl or i-propyl or represents the groups 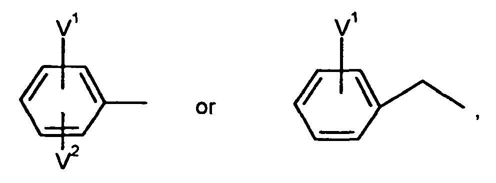
in which
V¹ represents hydrogen, bromine, chlorine, methyl, trifluoromethyl, phenoxy or t-butyl or represents optionally chlorine- or trifluoromethyl-substituted phenyl,
V² represents hydrogen, chlorine, fluorine or methoxy or
V¹ and V² together represent -O-CH₂-O- or -O-CF₂-O-,
W represents N-D⁽¹⁾, oxygen⁽²⁾ or sulphur ⁽³⁾,
D represents hydrogen or i-propyl,
A represents methyl, ethyl, n- or i-propyl or cyclopropyl or hydrogen,
B represents hydrogen or methyl, or
A and B and the carbon atom to which they are attached represent cyclohexyl in which optionally one ring atom is replaced by oxygen and which is optionally mono- or disubstituted by methyl, ethyl, methoxy or ethoxy, or represent cyclohexyl in which two not directly adjacent carbon atoms form a further 5-membered C ring,
A and D together with the atoms to which they are attached represent cyclohexyl in which one ring atom may be replaced by sulphur,
G represents hydrogen (a) or represents one of the groups 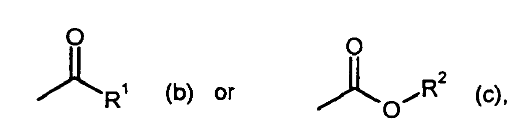
in which
R¹ represents methyl, ethyl, n-propyl or i-propyl or represents in each case optionally chlorine-substituted phenyl or pyridyl,
R² represents methyl, ethyl, phenyl, benzyl, n- or i-propyl.

7. Compounds of the formula (I) according to Claim 1 in which
Het represents 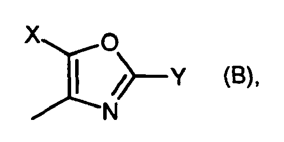
X represents methyl or ethyl,
Y represents optionally chlorine-substituted phenyl,
A represents methyl, ethyl, n- or i-propyl,
B represents methyl, or
A and B and the carbon atom to which they are attached together represent cyclohexyl which is optionally substituted by methoxy or methyl,
W represents N-D or oxygen,
D represents hydrogen,
G represents hydrogen.

8. Process for preparing compounds of the formula (I) according to Claim 1, **characterized in that**
(A) compounds of the formulae (I-1-A-a) to (I-1-Ba) 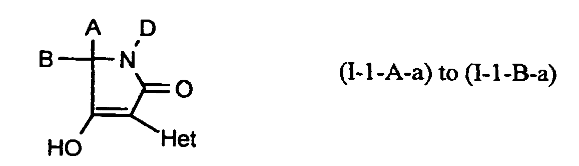
in which
A, B, D and Het are each as defined above
are obtained when
compounds of the formula (II) 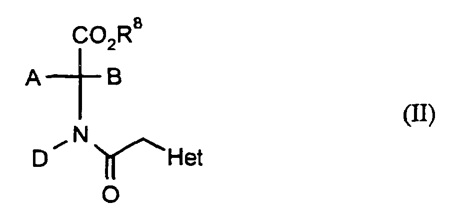
in which
A, B, D and Het are each as defined above
and
R⁸ represents alkyl
are condensed intramolecularly in the presence of a diluent and in the presence of a base,
(B) compounds of the formulae (I-2-A-a) to (I-2-B-a) 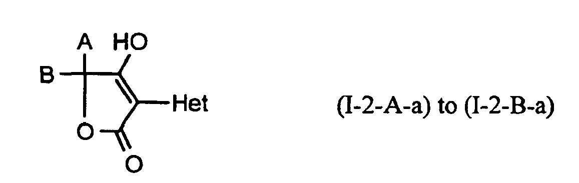
in which
A, B and Het are each as defined above
are obtained when
compounds of the formula (III) 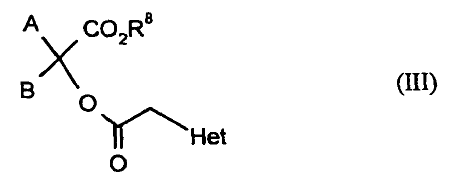
in which
A, B, Het and R⁸ are each as defined above
are condensed intramolecularly in the presence of a diluent and in the presence of a base,
(C) compounds of the formulae (I-3-A-a) to (I-3-B-a) 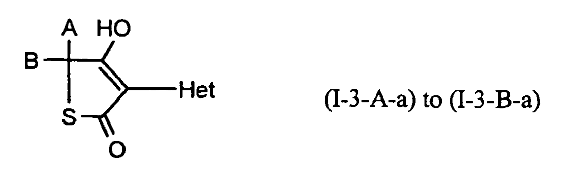
in which
A, B and Het are each as defined above,
are obtained when
compounds of the formula (IV) 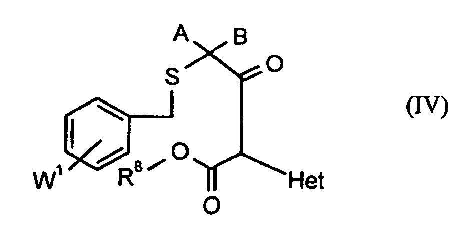
in which
A, B, Het and R⁸ are each as defined above and
W¹ represents hydrogen, halogen, alkyl or alkoxy,
are cyclized intramolecularly, if appropriate in the presence of a diluent and in the presence of an acid,
and, if appropriate, the resulting compounds of the formulae (I-1-A-a) to (I-3-B-a), in which
A, B, D and Het are each as defined above
are in each case reacted
(D)
(α) with acyl halides of the formula (V) 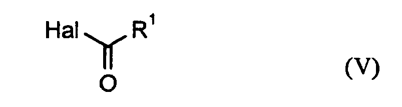
in which
R¹ is as defined above and
Hal represents halogen
or
(β) with carboxylic anhydrides of the formula (VI)
R¹-CO-O-CO-R¹ (VI)
in which
R¹ is as defined above,
if appropriate in the presence of a diluent and if appropriate in the presence of an acid binder;
(E) with chloroformic esters or chloroformic thioesters of the formula (VII)
R²-M-CO-Cl (VII)
in which
R² and M are each as defined above,
if appropriate in the presence of a diluent and if appropriate in the presence of an acid binder;
(F) with chloromonothioformic esters or chlorodithioformic esters of the formula (VIII) 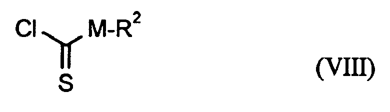
in which
M and R² are each as defined above,
if appropriate in the presence of a diluent and if appropriate in the presence of an acid binder
and
(G) with sulphonyl chlorides of the formula (IX)
R³-SO₂-Cl (IX)
in which
R³ is as defined above,
if appropriate in the presence of a diluent and if appropriate in the presence of an acid binder,
(H) with phosphorus compounds of the formula (X) 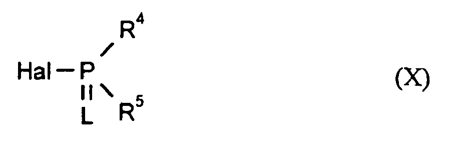
in which
L, R⁴ and R⁵ are each as defined above and
Hal represents halogen,
if appropriate in the presence of a diluent and if appropriate in the presence of an acid binder,
(I) with metal compounds or amines of the formula (XI) or (XII)
Me(OR¹⁰)ₜ (XI) 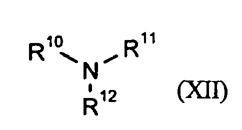
in which
Me represents a mono- or divalent metal,
t represents the number 1 or 2 and
R¹⁰, R¹¹, R¹² independently of one another each represent hydrogen or alkyl,
if appropriate in the presence of a diluent,
(J)
(α) with isocyanates or isothiocyanates of the formula (XIII)
R⁶-N=C=L (XIII)
in which
R⁶ and L are each as defined above,
if appropriate in the presence of a diluent and if appropriate in the presence of a catalyst, or
(β) with carbamoyl chlorides or thiocarbamoyl chlorides of the formula (XIV) 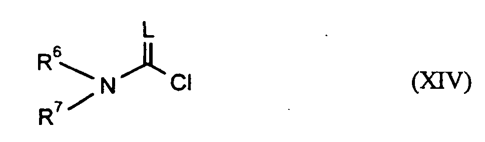
in which
L, R⁶ and R⁷ are each as defined above,
if appropriate in the presence of a diluent and if appropriate in the presence of an acid binder.

9. Compounds of the formula (II) 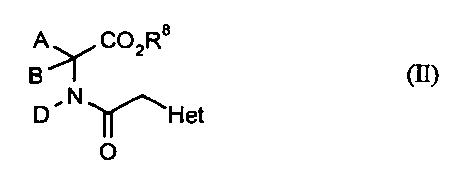
in which
A, B, D, Het and R⁸ are each as defined above,
with the exception of the compounds

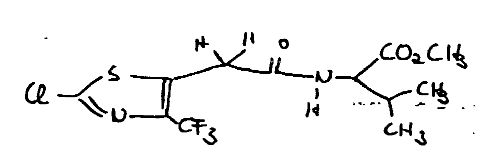

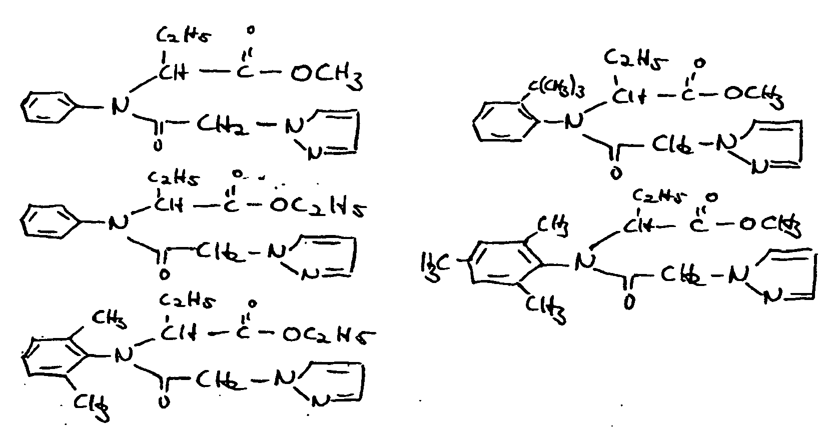

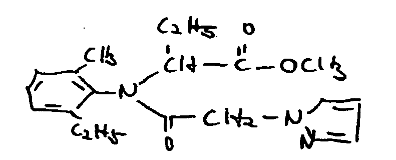

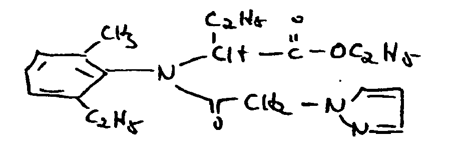

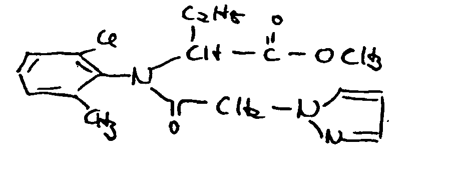

10. Compounds of the formula (XVII) 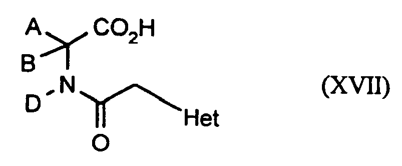
in which
A, B, D and Het are each as defined above.

11. Compounds of the formula (XXI) 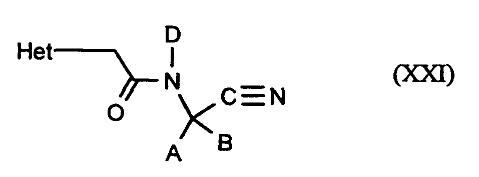
in which
A, B, D and Het are each as defined above.

12. Compounds of the formula (III) 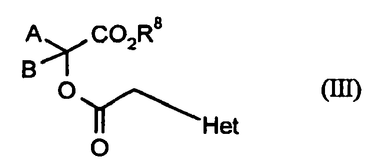
in which
A, B, Het and R⁸ are each as defined above,
with the exception of the compound

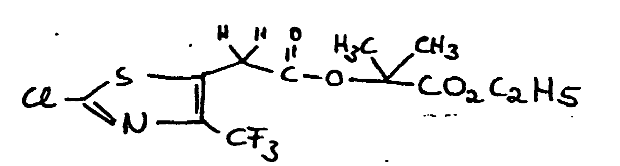

13. Compounds of the formula (IV) 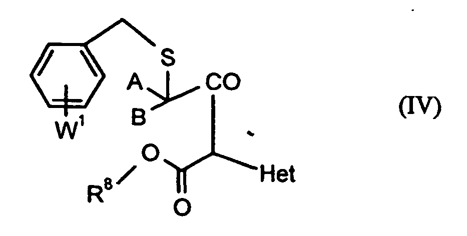
in which
A, B, W¹, Het and R⁸ are each as defined above and
W¹ represents hydrogen, halogen, alkyl or alkoxy.

14. Pesticides and herbicides, **characterized in that** they comprise at least one compound of the formula (I) according to Claim 1.

15. Use of compounds of the formula (I) according to Claim 1 for controlling pests and weeds.

16. Method for controlling pests and weeds, **characterized in that** compounds of the formula (I) according to Claim 1 are allowed to act on pests and/or their habitat or on weeds and/or their habitat.

17. Process for preparing pesticides and herbicides, **characterized in that** compounds of the formula (I) according to Claim 1 are mixed with extenders and/or surfactants.

18. Use of compounds of the formula (I) according to Claim 1 for preparing pesticides and herbicides.

## Revendications

1. Composés de formule (I) 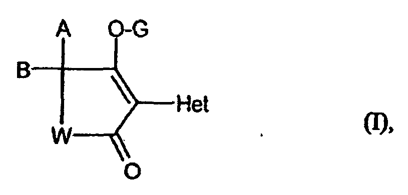
dans laquelle
W représente N-D⁽¹⁾, l'oxygène⁽²⁾ ou le soufre⁽³⁾,
Het représente un reste thiazolyle, oxazolyle ou pyrazolyle, chacun éventuellement substitué,
A représente l'hydrogène, un reste alkyle, alcényle, alkoxyalkyle, polyalkoxyalkyle, alkylthioalkyle, chacun éventuellement substitué par un halogène, un reste cycloalkyle saturé ou non saturé, éventuellement substitué, dans lequel, le cas échéant, au moins un atome du noyau est remplacé par un hétéroatome, ou bien un reste aryle, arylalkyle ou hétaryle, chacun éventuellement substitué par un radical halogéno, alkyle, halogénalkyle, alkoxy, halogénalkoxy, cyano ou nitro,
B représente l'hydrogène, un reste alkyle ou alkoxyalkyle, ou bien
A et B forment conjointement avec l'atome de carbone auquel ils sont liés un cycle saturé ou non saturé, non substitué ou substitué, contenant éventuellement au moins un hétéroatome,
D représente l'hydrogène ou un reste éventuellement substitué de la série alkyle, alcényle, alcynyle, alkoxyalkyle, polyalkoxyalkyle, alkylthioalkyle, cycloalkyle saturé ou non saturé dans lequel, le cas échéant, un ou plusieurs atomes du noyau sont remplacés par des hétéroatomes, un reste arylalkyle, aryle, hétarylalkyle ou hétaryle, ou bien
A et D forment, conjointement avec les atomes auxquels ils sont liés, un cycle saturé ou non saturé, non substitué ou substitué dans la partie A,D et contenant éventuellement au moins un hétéroatome,
G représente l'hydrogène (a) ou l'un des groupes 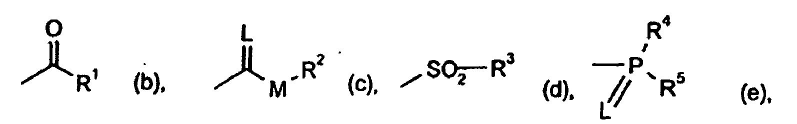 E (f) ou 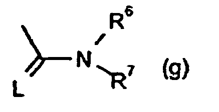
dans lesquels
E représente un équivalent d'ion métallique ou un ion ammonium,
L représente l'oxygène ou le soufre,
M représente l'oxygène ou le soufre,
R¹ représente un reste alkyle, alcényle, alkoxyalkyle, alkylthioalkyle, polyalkoxy-alkyle, chacun éventuellement substitué par un halogène, ou un reste cycloalkyle éventuellement substitué par un radical halogéno, alkyle ou alkoxy et qui peut être interrompu par au moins un hétéroatome, un reste phényle, phénylalkyle, hétaryle, phénoxyalkyle ou hétaryloxyalkyle, chacun éventuellement substitué,
R² représente un reste alkyle, alcényle, alkoxy-alkyle, polyalkoxyalkyle, chacun éventuellement substitué par un halogène, ou un reste cycloalkyle, phényle ou benzyle, chacun éventuellement substitué,
R³ représente un reste alkyle, halogénalkyle ou un reste phényle ou benzyle, chacun éventuellement substitué,
R⁴ et R⁵ représentent, indépendamment l'un de l'autre, un reste alkyle, alkoxy, alkylamino, dialkylamino, alkylthio, alcénylthio, cyclo-alkylthio, chacun éventuellement substitué par un halogène, et un reste phényle, benzyle, phénoxy ou phénylthio, chacun éventuellement substitué,
R⁶ et R⁷ représentent, indépendamment l'un de l'autre, l'hydrogène, un reste alkyle, cyclo-alkyle, alcényle, alkoxy, alkoxyalkyle, chacun éventuellement substitué par un halogène, un reste phényle éventuellement substitué, un reste benzyle éventuellement substitué, ou forment conjointement avec l'atome d'azote auquel ils sont liés un cycle éventuellement interrompu par de l'oxygène ou du soufre.

2. Composés de formule (I) suivant la revendication 1, formule dans laquelle
Het représente 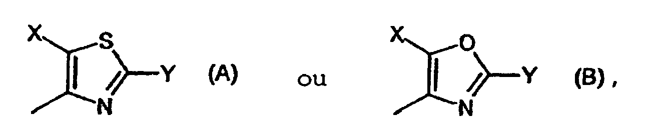
X représente l'hydrogène, un halogène, un reste alkyle en C₁ à C₆, halogénalkyle en C₁ à C₄, alkoxy en C₁ à C₆, alcényloxy en C₃ à C₆, nitro ou cyano,
Y représente un halogène, un reste alkyle en C₁ à C₆, halogénalkyle en C₁ à C₆, alkoxy en C₁ à C₆, halogénalkoxy en C₁ à C₆, alkylthio en C₁ à C₆, alkylsulfonyle en C₁ à C₆, alkylsulfoxinyle en C₁ à C₆ ou le groupe 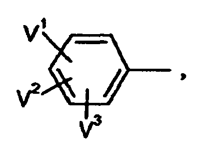
ou aussi, au cas où Het est un reste thiazolyle ((I-1-A) à (I-3-A)) un groupe 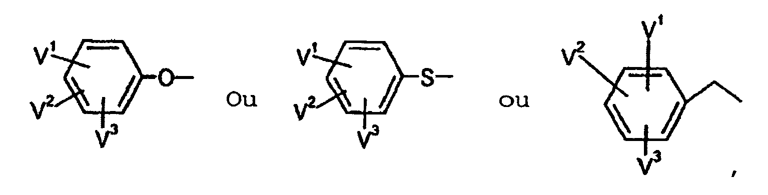
dans lequel
V¹ représente l'hydrogène, un halogène, un reste alkyle en C₁ à C₁₂, alkoxy en C₁ à C₆, alkylthio en C₁ à C₆, alkylsulfinyle en C₁ à C₆, alkylsulfonyle en C₁ à C₆, halogénalkyle en C₁ à C₄, halogénalkoxy en C₁ à C₄, nitro, cyano, phénoxy, ou bien un reste phényle, phénoxy, phénoxy-(alkyle en C₁ à C₄), phényl-(alkoxy en C₁ à C₄), phénylthio-(alkyle en C₁ à C₄) ou phényl-(alkylthio en C₁ à C₄), chacun éventuellement substitué une ou plusieurs fois par un radical halogéno, alkyle en C₁ à C₆, alkoxy en C₁ à C₆, halogénalkyle en C₁ à C₉, halogénalkoxy en C₁ à C₄, nitro ou cyano,
V² et V³ représentent, indépendamment l'un de l'autre, l'hydrogène, un halogène, un reste alkyle en C₁ à C₆, alkoxy en C₁ à C₆, halogénalkyle en C₁ à C₄ ou halogénalkoxy en C₁ à C₄,
V¹ et V² forment, conjointement avec l'atome de carbone auquel ils sont liés, un cycle pentagonal ou hexagonal saturé ou non saturé éventuellement substitué par un radical alkyle en C₁ à C₄ ou halogéno et dans lequel, le cas échéant, un à trois atomes de carbone peuvent être remplacés par de l'oxygène, du soufre ou de l'azote,
W représente N-D⁽¹⁾, l'oxygène⁽²⁾ ou le soufre⁽³⁾,
A représente l'hydrogène ou un reste alkyle en C₁ à C₁₂, alcényle en C₃ à C₈, (alkoxy en C₁ à C₆)-(alkyle en C₁ à C₆), poly(alkoxy en C₁ à C₆)-(alkyle en C₁ à C₆), (alkylthio en C₁ à C₁₀)-(alkyle en C₁ à C₆), chacun éventuellement substitué par du fluor ou du chlore, un reste cycloalkyle en C₃ à C₈ éventuellement substitué par un radical fluoro, chloro, alkyle en C₁ à C₆ ou alkoxy en C₁ à C₆ et dans lequel, le cas échéant, un ou deux chaînons du noyau non directement contigus sont remplacés par de l'oxygène et/ou du soufre, ou un reste aryle en C₆ ou C₁₀, hétaryle à noyau de 5 ou 6 atomes ou (aryle en C₆ ou C₁₀)-(alkyle en C₁ à C₆), chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un reste alkyle en C₁ à C₆ ou (alkoxy en C₁ à C₄) - (alkyle en C₁ à C₄), ou bien
A, B et l'atome de carbone auquel ils sont liés représentent un reste cycloalkyle en C₃ à C₁₀ saturé ou un reste cycloalkyle en C₅ à C₁₀ non saturé, dont un chaînon du noyau est éventuellement remplacé par de l'oxygène ou du soufre et qui sont éventuellement substitués une ou deux fois par un radical alkyle en C₁ à C₆, cycloalkyle en C₃ à C₈, halogénalkyle en C₁ à C₄, alkoxy en C₁ à C₆, alkylthio en C₁ à C₆, halogéno ou phényle, ou bien
A, B et l'atome de carbone auquel ils sont liés représentent un reste cycloalkyle en C₅ ou C₆ qui est substitué par un groupe alkylènediyle contenant éventuellement un atome d'oxygène ou de soufre, ou par un groupe alkylènedioxolyle ou par un groupe alkylènedithioyle qui forme, avec l'atome de carbone auquel il est lié, un autre noyau pentagonal ou hexagonal et qui peut éventuellement être substitué une à quatre fois par un radical alkyle en C₁ à C₄, ou bien
A, B et l'atome de carbone auquel ils sont liés représentent un reste cycloalkyle en C₃ à C₈ ou un reste cycloalcényle en C₅ à C₈, dans lesquels deux substituants forment, conjointement avec les atomes de carbone auxquels ils sont liés, un reste alcanediyle en C₂ à C₆, alcènediyle en C₂ à C₆ ou alcanediènediyle en C₄ à C₆, chacun éventuellement substitué par un radical alkyle en C₁ à C₆, alkoxy en C₁ à C₆ ou halogéno, et dont un groupe méthylène est éventuellement remplacé par de l'oxygène ou du soufre,
D représente l'hydrogène, un reste alkyle en C₁ à C₁₂, alcényle en C₃ à C₈, alcynyle en C₃ à C₈, (alkoxy en C₁ à C₁₀)-(alkyle en C₂ à C₈), poly(alkoxy en C₁ à C₈)-(alkyle en C₂ à C₈), (alkylthio en C₁ à C₁₀) - (alkyle en C₂ à C₈), chacun éventuellement substitué par un radical halogéno ou cyano, un reste cycloalkyle en C₃ à C₈ éventuellement substitué par un radical halogéno, alkyle en C₁ à C₄, alkoxy en C₁ à C₄ ou halogénalkyle en C₁ à C₄ et dans lequel, le cas échéant, un chaînon du noyau est remplacé par de l'oxygène ou du soufre, ou un reste phényle, hétaryle pentagonal ou hexagonal, phényl-(alkyle en C₁ à C₆) ou hétaryl-(alkyle en C₁ à C₆) pentagonal ou hexagonal, chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment ensemble un reste alcanediyle ou alcènediyle en C₃ à C₆, chacun éventuellement substitué, dans lesquels le cas échéant un groupe méthylène est remplacé par un groupe carbonyle, de l'oxygène ou du soufre, et
en considérant dans chaque cas comme substituants :
un halogène, un groupe hydroxy, mercapto ou un reste alkyle en C₁ à C₁₀, alkoxy en C₁ à C₆, alkylthio en C₁ à C₆, cycloalkyle en C₃ à C₇, phényle ou benzyloxy, chacun éventuellement substitué par un halogène, ou un autre groupement alcanediyle en C₃ à C₆, alcènediyle en C₃ à C₆ ou un groupement butadiényle qui est éventuellement substitué par un radical alkyle en C₁ à C₆ ou dans lequel, le cas échéant, deux substituants contigus forment avec les atomes de carbone auxquels ils sont liés un autre cycle saturé
ou non saturé pentagonal ou hexagonal (dans le cas du composé de formules (I-1-A) à (I-1-B), A et D forment alors, conjointement avec les atomes auxquels ils sont liés, par exemple les autres groupes AD-1 à AD-10 mentionnés ci-dessous), qui peut contenir de l'oxygène ou du soufre, ou dans lequel, le cas échéant, l'un des groupes suivants 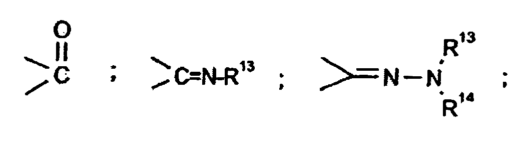 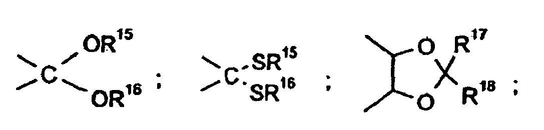 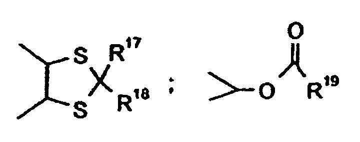 ou 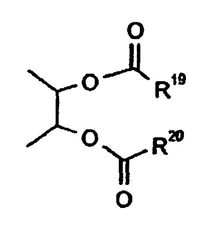
est contenu,
G représente l'hydrogène (a) ou l'un des groupes 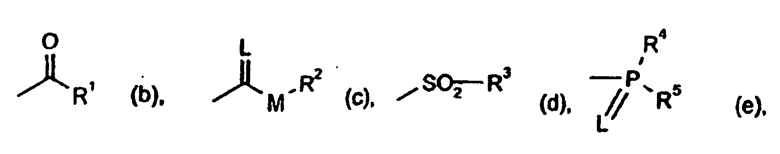 E (f) ou 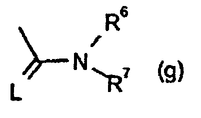
dans lesquels
E représente un équivalent d'ion métallique ou un ion ammonium,
L représente l'oxygène ou le soufre, et
M représente l'oxygène ou le soufre,
R¹ représente un reste alkyle en C₁ à C₂₀, alcényle en C₂ à C₂₀, (alkoxy en C₁ à C₈)-(alkyle en C₁ à C₈), (alkylthio en C₁ à C₈)-(alkyle en C₁ à C₈), poly(alkoxy en C₁ à C₈)-(alkyle en C₁ à C₈), chacun éventuellement substitué par un halogène, ou bien un reste cycloalkyle en C₃ à C₈ éventuellement substitué par un radical halogéno, alkyle en C₁ à C₆ ou alkoxy en C₁ à C₆ et dans lequel, le cas échéant, un ou plusieurs chaînons du noyau non directement contigus sont remplacés par de l'oxygène et/ou par du soufre,
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un reste phényle-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal éventuellement substitué par un radical halogéno, alkyle en C₁ à C₆, halogénalkyle en C₁ ou C₂ ou alkoxy en C₁ à C₄,
un reste phénoxy-(alkyle en C₁ à C₆) éventuellement substitué par un radical halogéno ou alkyle en C₁ à C₆,
un reste hétaryloxy-(alkyle en C₁ à C₆) pentagonal ou hexagonal éventuellement substitué par un radical halogéno, amino ou alkyle en C₁ à C₆,
R² représente un reste alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈), chacun éventuellement substitué par un halogène,
un reste cycloalkyle en C₃ à C₈ éventuellement substitué par un radical halogéno, alkyle en C₁ à C₆
ou alkoxy en C₁ à C₆ et dans lequel, le cas échéant, un atome du noyau est remplacé par de l'oxygène, ou bien
un reste phényle ou benzyle, chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle, chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent, indépendamment l'un de l'autre, un reste alkyle en C₁ à C₈, alkoxy en C₁ à C₈, alkylamino en C₁ à C₈, di (alkyle en C₁ à C₈)amino, alkylthio en C₁ à C₈, alcénylthio en C₂ à C₈, cycloalkylthio en C₃ à C₇, chacun éventuellement substitué par un halogène, ou un reste phényle, phénoxy ou phénylthio, chacun éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent, indépendamment l'un de l'autre, l'hydrogène, un reste alkyle en C₁ à C₈, cycloalkyle en C₃ à C₈, alkoxy en C₁ à C₈, alcényle en C₃ à C₈, (alkoxy en C₁ à C₈)-(alkyle en C₁ à C₈), chacun éventuellement substitué par un halogène, un reste phényle éventuellement substitué par un radical halogéno, halogénalkyle en C₁ à C₈, alkyle en C₁ à C₈ ou alkoxy en C₁ à C₈, un reste benzyle éventuellement substitué par un radical halogéno, alkyle en C₁ à C₈, halogénalkyle en C₁ à C₈ ou alkoxy en C₁ à Ce, ou forment ensemble un reste alkylène en C₃ à C₆ éventuellement substitué par un radical alkyle en C₁ à C₄ et dans lequel, le cas échéant, un atome de carbone est remplacé par de l'oxygène ou du soufre,
R¹³ représente l'hydrogène, un reste alkyle en C₁ à C₈ ou alkoxy en C₁ à C₈, chacun éventuellement substitué par un halogène, un reste cycloalkyle en C₃ à C₈ éventuellement substitué par un radical halogéno, alkyle en C₁ à C₄ ou alkoxy en C₁ à C₄ et dans lequel, le cas échéant, un groupe méthylène est remplacé par de l'oxygène ou du soufre, ou bien un reste phényle, phényl- (alkyle en C₁ à C₄) ou phényl-(alkoxy en C₁ à C₄) chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
R¹⁴ représente l'hydrogène ou un reste alkyle en C₁ à C₈, ou bien
R¹³ et R¹⁴ forment ensemble un reste alcanediyle en C₄ à C₆,
R¹⁵ et R¹⁶ sont identiques ou différents et représentent un reste alkyle en C₁ à C₆, ou bien,
R¹⁵ et R¹⁶ forment ensemble un reste alcanediyle en C₂ à C₄, qui est éventuellement substitué par un radical alkyle en C₁ à C₆, halogénalkyle en C₁ à C₆ ou par un reste phényle éventuellement substitué par un radical halogéno, alkyle en C₁ à C₆, halogénalkyle en C₁ à C₄, alkoxy en C₁ à C₆, halogénalkoxy en C₁ à C₄, nitro ou cyano,
R¹⁷ et R¹⁸ représentent, indépendamment l'un de l'autre, l'hydrogène, un reste alkyle en C₁ à C₈ éventuellement substitué par un halogène, ou un reste phényle éventuellement substitué par un radical halogéno, alkyle en C₁ à C₆, alkoxy en C₁ à C₆, halogénalkyle en C₁ à C₄, halogénalkoxy en C₁ à C₄, nitro ou cyano, ou bien
R¹⁷ et R¹⁸ forment, conjointement avec l'atome de carbone auquel ils sont liés, un groupe carbonyle ou un reste cycloalkyle en C₅ à C₇ éventuellement substitué par un radical halogéno, alkyle en C₁ à C₄ ou alkoxy en C₁ à C₄ et dans lequel, le cas échéant, un groupe méthylène est remplacé par de l'oxygène ou du soufre,
R¹⁹ et R²⁰ représentent, indépendamment l'un de l'autre, un reste alkyle en C₁ à C₁₀, alcényle en C₂ à C₁₀, alkoxy en C₁ à C₁₀, alkylamino en C₁ à C₁₀, alcénylamino en C₃ à C₁₀, di (alkyle en C₁ à C₁₀)amino ou di (alcényle en C₃ à C₁₀)amino.

3. Composés de formule (I) suivant la revendication 1, formule dans laquelle
Het représente 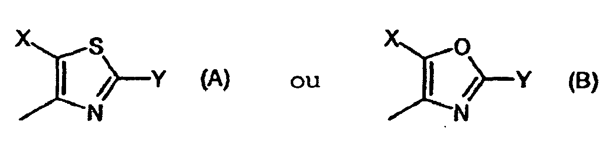
X représente l'hydrogène, le chlore, le brome, un reste alkyle en C₁ à C₄, halogénalkyle en C₁ ou C₂, nitro ou cyano,
Y représente le chlore, le brome, un reste alkyle en C₁ à C₄, halogénalkyle en C₁ à C₄, alkoxy en C₁ à C₄, halogénalkoxy en C₁ à C₄, alkylthio en C₁ à C₄, alkylsulfonyle en C₁ à C₄ ou le groupe 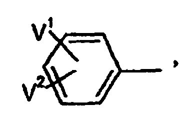
ou aussi, au cas où Het est un reste thiazolyle ((I-1-A) à (I-3-A)) un groupe 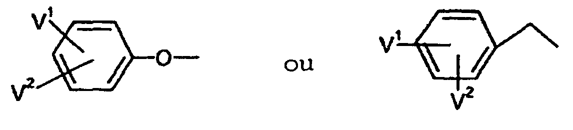
dans lequel
V¹ représente l'hydrogène, le fluor, le chlore, le brome, un reste alkyle en C₁ à C₆, alkoxy en C₁ à C₄, halogénalkyle en C₁ ou C₂, halogénalkoxy en C₁ ou C₂, nitro, cyano, phénoxy, ou un reste phényle, phénoxy, phénoxy-(alkyle en C₁ ou C₂), phényl-(alkoxy en C₁ ou C₂), phénylthio-(alkyle en C₁ ou C₂) ou phényl-(alkylthio en C₁ ou C₂), chacun éventuellement substitué une ou deux fois par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
V² représente l'hydrogène, le fluor, le chlore, le brome, un reste alkyle en C₁ à C₄, alkoxy en C₁ à C₄, halogénalkyle en C₁ ou C₂ ou halogénalkoxy en C₁ ou C₂, ou bien
V¹ et V² forment, conjointement avec les atomes de carbone auxquels ils sont liés, un cycle pentagonal ou hexagonal éventuellement substitué par un radical fluoro ou méthyle et dans lequel, le cas échéant, un ou deux atomes de carbone peuvent être remplacés par de l'oxygène,
W représente N-D⁽¹⁾, l'oxygène⁽²⁾ ou le soufre⁽³⁾,
A représente l'hydrogène, un reste alkyle en C₁ à C₁₀, (alkoxy en C₁ à C₄)-(alkyle en C₁ à C₄), chacun éventuellement substitué par du fluor, un reste cycloalkyle en C₃ à C₇ éventuellement substitué par un radical fluoro, chloro, alkyle en C₁ à C₄ ou alkoxy en C₁ à C₄ et dans lequel, le cas échéant, un chaînon du noyau est remplacé par de l'oxygène ou du soufre, ou un reste phényle ou phényl-(alkyle en C₁ ou C₂), chacun éventuellement substitué par un radical fluoro, chloro, bromo, alkyle en C₁ à C₄, halogénalkyle en C₁ à C₄, alkoxy en C₁ à C₄ ou halogénalkoxy en C₁ à C₄,
B représente l'hydrogène ou un reste alkyle en C₁ à C₄,
A, B et l'atome de carbone auquel ils sont liés représentent un reste cycloalkyle en C₅ à C₇ saturé, dans lequel le cas échéant un chaînon du noyau est remplacé par de l'oxygène ou du soufre et qui est éventuellement substitué une ou deux fois par un radical alkyle en C₁ à C₆, cycloalkyle en C₅ ou C₆, halogénalkyle en C₁ ou C₂, alkoxy en C₁ à C₆, fluoro, chloro ou phényle, ou bien
A, B et l'atome de carbone auquel ils sont liés représentent un reste cycloalkyle en C₅ ou C₆, qui est substitué par un groupe alkylènediyle contenant éventuellement un atome d'oxygène ou de soufre, ou par un groupe alkylènedioxyle, ou par un groupe alkylènedithioyle qui forme, avec l'atome de carbone auquel il est lié, un autre noyau pentagonal ou hexagonal qui peut éventuellement être substitué une à trois fois par un radical alkyle en C₁ à C₃, ou bien
A, B et l'atome de carbone auquel ils sont liés forment un reste cycloalkyle en C₅ ou C₆ ou un groupe cyclo-alcényle en C₅ ou C₆, dans lesquels deux substituants forment, conjointement avec les atomes de carbone auxquels ils sont liés, un reste alcanediyle en C₂ à C₄ ou alcènediyle en C₂ à C₄, chacun éventuellement substitué par un radical alkyle en C₁ à C₅, alkoxy en C₁ à C₅, fluoro, chloro ou bromo, où le cas échéant un groupe méthylène est remplacé par de l'oxygène ou du soufre, ou bien un reste butadiènediyle,
D représente l'hydrogène, un reste alkyle en C₁ à C₁₀, alcényle en C₃ à C₆, (alkoxy en C₁ à C₆)-(alkyle en C₂ à C₄) ou (alkylthio en C₁ à C₆)-(alkyle en C₂ à C₄) chacun éventuellement substitué par du fluor, un reste cycloalkyle en C₃ à C₇ éventuellement substitué par un radical fluoro, alkyle en C₁ à C₄, alkoxy en C₁ à C₄ ou halogénalkyle en C₁ ou C₂ et dans lequel, le cas échéant, un groupe méthylène est remplacé par de l'oxygène ou du soufre, ou bien un reste phényle, ou phényl-(alkyle en C₁ à C₄), chacun éventuellement substitué par un radical fluoro, chloro, bromo, alkyle en C₁ à C₄, halogénalkyle en C₁ ou C₂, alkoxy en C₁ à C₄ ou halogénalkoxy en C₁ ou C₂, ou bien
A et D forment ensemble un reste alcanediyle en C₃ à C₅ éventuellement substitué, dans lequel, le cas échéant, un groupe méthylène peut être remplacé par un groupe carbonyle, de l'oxygène ou du soufre, les substituants considérés étant des radicaux hydroxy, alkyle en C₁ à C₆ ou alkoxy en C₁ à C₄, ou bien
A et D forment, conjointement avec les atomes auxquels ils sont liés, l'un des groupes AD-1 à AD-10 suivants : 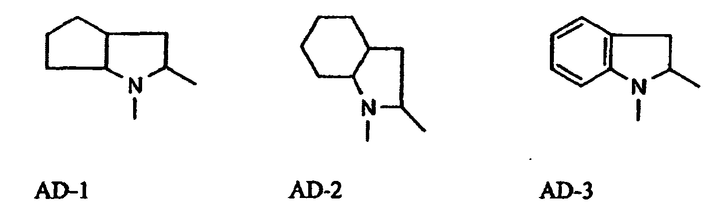 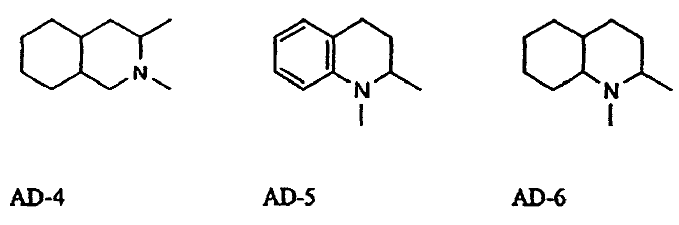 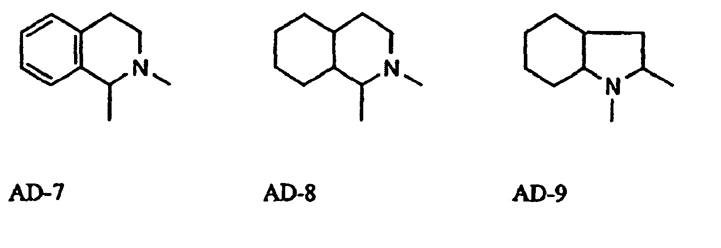 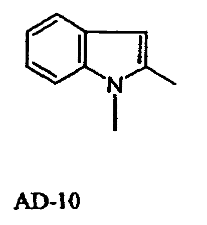
G représente l'hydrogène (a) ou l'un des groupes 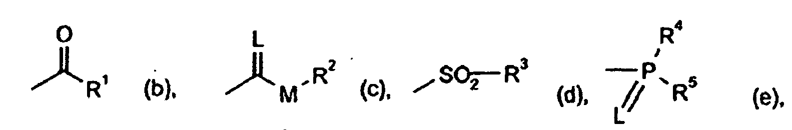 E (f) ou 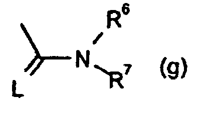
dans lesquels
E représente un équivalent d'ion métallique ou un ion ammonium,
L représente l'oxygène ou le soufre et
M représente l'oxygène ou le soufre,
R¹ représente un reste alkyle en C₁ à C₁₆, alcényle en C₂ à C₁₆, (alkoxy en C₁ à C₆)-(alkyle en C₁ à C₄), (alkylthio en C₁ à C₆)-(alkyle en C₁ à C₄), chacun éventuellement substitué par du fluor ou du chlore, ou un reste cycloalkyle en C₃ à C₇ éventuellement substitué par un radical fluoro, chloro, alkyle en C₁ à C₅ ou alkoxy en C₁ à C₅ et dans lequel, le cas échéant, un ou deux chaînons non directement contigus du noyau sont remplacés par de l'oxygène et/ou par du soufre,
un reste phényle éventuellement substitué par un radical fluoro, chloro, bromo, cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un reste phényle-(alkyle en C₁ à C₄) éventuellement substitué par un radical fluoro, chloro, bromo, alkyle en C₁ à C₄, alkoxy en C₁ à C₄, halogénalkyle en C₁ à C₃ ou halogénalkoxy en C₁ à C₃,
un reste pyrazolyle, thiazolyle, pyridyle, pyrimidyle, furannyle ou thiényle chacun éventuellement substitué par un radical fluoro, chloro, bromo, alkyle en C₁ à C₄, trifluorométhyle ou alkoxy en C₁ ou C₂,
R² représente un reste alkyle en C₁ à C₁₆, alcényle en C₂ à C₁₆ ou (alkoxy en C₁ à C₆) - (alkyle en C₂ à C₆) chacun éventuellement substitué par du fluor,
un reste cycloalkyle en C₃ à C₇ éventuellement substitué par un radical fluoro, chloro, alkyle en C₁ à C₄ ou alkoxy en C₁ à C₄, ou bien
un reste phényle ou benzyle, chacun éventuellement substitué par un radical fluoro, chloro, bromo, cyano, nitro, alkyle en C₁ à C₄, alkoxy en C₁ à C₄, halogénalkyle en C₁ ou C₂ ou halogénalkoxy en C₁ ou C₂,
R³ représente un reste alkyle en C₁ à C₆ éventuellement substitué par du fluor, ou un reste phényle éventuellement substitué par un radical fluoro, chloro, bromo, alkyle en C₁ à C₄, alkoxy en C₁ à C₄, halogénalkyle en C₁ à C₃, halogénalkoxy en C₁ à C₃, cyano ou nitro,
R⁴ est un reste alkyle en C₁ à C₆, alkoxy en C₁ à C₆, alkylamino en C₁ à C₆, di (alkyle en C₁ à C₆) amino, alkylthio en C₁ à C₆, alcénylthio en C₃ ou C₄, cycloalkylthio en C₃ à C₆ ou un reste phényle, phénoxy ou phénylthio, chacun éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃,
R⁵ est un reste alkoxy en C₁ à C₆ ou alkylthio en C₁ à C₆,
R⁶ est un reste alkyle en C₁ à C₆, cycloalkyle en C₃ à C₆, alkoxy en C₁ à C₆, alcényle en C₃ à C₆, (alkoxy en C₁ à C₆)-(alkyle en C₁ à C₆), un reste phényle éventuellement substitué par un radical fluoro, chloro, bromo, halogénalkyle en C₁ à C₃, alkyle en C₁ à C₄ ou alkoxy en C₁ à C₄, un reste benzyle éventuellement substitué par un radical fluoro, chloro, bromo, alkyle en C₁ à C₄, halogénalkyle en C₁ à C₃ ou alkoxy en C₁ à C₄,
R⁷ représente l'hydrogène, un reste alkyle en C₁ à C₆, alcényle en C₃ à C₆, ou bien
R⁶ et R⁷ forment ensemble un reste alkylène en C₄ ou C₅ éventuellement substitué par un radical méthyle ou éthyle et dans lequel, le cas échéant, un groupe méthylène est remplacé par de l'oxygène ou du soufre.

4. Composés de formule (I) suivant la revendication 1, formule dans laquelle
Het représente un groupe 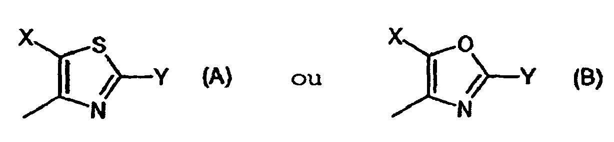
X représente l'hydrogène, le chlore, le brome, un reste méthyle, éthyle, n-propyle, iso-propyle, n-butyle ou iso-butyle,
Y est un reste méthyle, éthyle, n-propyle, iso-propyle, n-butyle, iso-butyle, tertiobutyle, trifluorométhyle ou le groupe 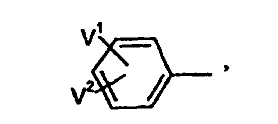
ou aussi, au cas où Het est un reste thiazolyle ((I-1-A) à (I-3-A)), un groupe 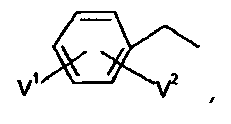
dans lequel
V¹ représente l'hydrogène, le fluor, le chlore, le brome, un reste méthyle, éthyle, n-propyle, iso-propyle, n-butyle, iso-butyle, tertiobutyle, méthoxy, éthoxy, n-propoxy, iso-propoxy, trifluorométhyle, trifluorométhoxy, nitro, cyano, phénoxy, ou un reste phényle éventuellement substitué une ou deux fois par un radical fluoro, chloro, méthyle, méthoxy, trifluorométhyle ou trifluorométhoxy,
V² représente l'hydrogène, le fluor, le chlore, un reste méthyle, éthyle, n-propyle, iso-propyle, méthoxy, éthoxy, trifluorométhyle ou trifluorométhoxy, ou bien
V¹ et V² forment ensemble un groupe -O-CH₂-O-, -O-CF₂-O- ou -O-CF₂-CF₂-O-,
W représente N-D⁽¹⁾, l'oxygène⁽²⁾ ou le soufre⁽³⁾,
A représente l'hydrogène, un reste alkyle en C₁ à C₈ ou (alkoxy en C₁ à C₄)-(alkyle en C₁ ou C₂) éventuellement substitué par du fluor, un reste cycloalkyle en C₃ à C₆ éventuellement substitué par un radical fluoro, méthyle, éthyle ou méthoxy et dans lequel, le cas échéant, un chaînon du noyau est remplacé par de l'oxygène ou du soufre, ou bien un reste phényle ou benzyle, chacun éventuellement substitué par un radical fluoro, chloro, bromo, méthyle, éthyle, n-propyle, iso-propyle, tertiobutyle, méthoxy, éthoxy, trifluorométhyle, trifluorométhoxy, cyano ou nitro,
B représente l'hydrogène, un reste méthyle ou éthyle, ou bien
A, B et l'atome de carbone auquel ils sont liés représentent un reste cycloalkyle en C₅ ou C₆ saturé, dans lequel le cas échéant un chaînon du noyau est remplacé par de l'oxygène ou du soufre et qui est éventuellement substitué une ou deux fois par un radical méthyle, éthyle, propyle, isopropyle, butyle, iso-butyle, sec.-butyle, tertiobutyle, trifluoro-méthyle, méthoxy, éthoxy, propoxy, iso-propoxy, butoxy ou iso-butoxy, ou bien
A, B et l'atome de carbone auquel ils sont liés représentent un reste cycloalkyle en C₆, qui est éventuellement substitué par un groupe alkylène-dioxyle éventuellement substitué une ou deux fois par un radical méthyle ou éthyle, et qui forme un autre noyau pentagonal ou hexagonal avec l'atome de carbone auquel il est lié, ou bien
A, B et l'atome de carbone auquel ils sont liés forment un reste cycloalkyle en C₅ ou C₆ ou cycloalcényle en C₅ ou C₆, dans lequel deux substituants forment, conjointement avec les atomes de carbone auxquels ils sont liés, un reste alcanediyle en C₂ à C₄ ou alcènediyle en C₂ à C₄, dans chacun desquels le cas échéant un groupe méthylène est remplacé par de l'oxygène ou du soufre, ou bien un reste butadiènediyle,
D représente l'hydrogène, un reste alkyle en C₁ à C₆, alcényle en C₃ ou C₄, (alkoxy en C₁ à C₄)-(alkyle en C₂ ou C₃), ou cycloalkyle en C₃ à C₆, (alkylthio en C₁ à C₄)-(alkyle en C₂ ou C₃) chacun éventuellement substitué par du fluor, dans lequel, le cas échéant, un groupe méthylène est remplacé par de l'oxygène ou du soufre, ou bien un reste phényle ou benzyle chacun éventuellement substitué par un radical fluoro, chloro, méthyle, éthyle, n-propyle, iso-propyle, méthoxy, éthoxy, trifluorométhyle ou trifluorométhoxy,
ou bien
A et D forment ensemble un reste alcanediyle en C₃ ou C₄ éventuellement substitué, dans lequel, le cas échéant, un atome de carbone est remplacé par de l'oxygène ou du soufre et qui est éventuellement substitué par un radical méthyle, ou bien
A et D (dans le cas des composés de formule (I-1)) forment conjointement avec les atomes auxquels ils sont liés, l'un des groupes AD suivants : 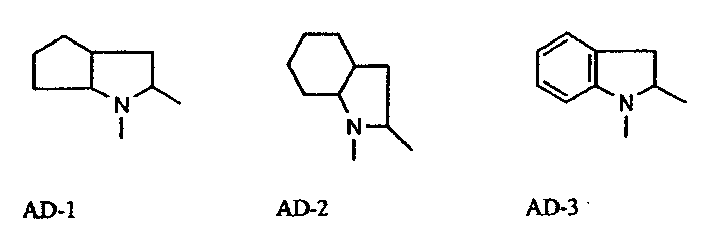 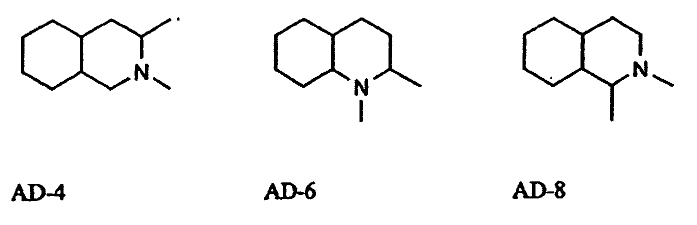 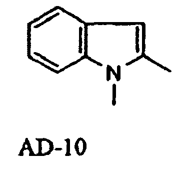
G représente l'hydrogène (a) ou l'un des groupes 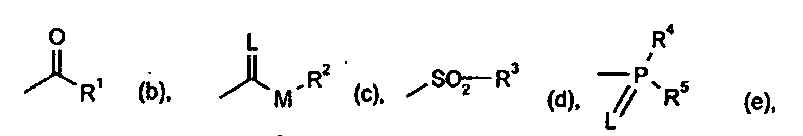 E (f) ou 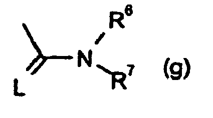
dans lesquels
E désigne un équivalent d'ion métallique ou un ion ammonium,
L représente l'oxygène ou le soufre et
M représente l'oxygène ou le soufre,
R¹ désigne un reste alkyle en C₁ à C₁₄, alcényle en C₂ à C₁₄, (alkoxy en C₁ à C₄)-(alkyle en C₁ ou C₂), (alkylthio en C₁ à C₄)-(alkyle en C₁ ou C₂), chacun éventuellement substitué par du fluor, ou un reste cyclopropyle, cyclopentyle ou cyclohexyle chacun éventuellement substitué par un radical fluoro, chloro, méthyle, éthyle ou méthoxy,
un reste phényle éventuellement substitué une ou deux fois par un radical fluoro, chloro, bromo, cyano, nitro, méthyle, éthyle, tertiobutyle, méthoxy, éthoxy, trifluorométhyle ou trifluorométhoxy,
un reste thiényle ou pyridyle chacun éventuellement substitué une fois par un radical fluoro, chloro, bromo ou méthyle,
R² représente un reste alkyle en C₁ à C₈, alcényle en C₂ à C₈ ou (alkoxy en C₁ à C₄)-(alkyle en C₂ ou C₃) chacun éventuellement substitué par du fluor,
un reste cyclohexyle éventuellement substitué par un radical fluoro, chloro, méthyle, éthyle, n-propyle, iso-propyle ou méthoxy,
ou bien un reste phényle ou benzyle, chacun éventuellement substitué par un radical fluoro, chloro, cyano, nitro, méthyle, éthyle, méthoxy, trifluorométhyle ou trifluorométhoxy,
R³ représente un reste méthyle, éthyle, n-propyle, ou un reste phényle éventuellement substitué par un radical fluoro, chloro, bromo, méthyle, tertiobutyle, méthoxy, trifluorométhyle, trifluorométhoxy, cyano ou nitro,
R⁴ représente un reste alkyle en C₁ à C₄, alkoxy en C₁ à C₄, alkylamino en C₁ à C₄, di (alkyle en C₁ à C₄)amino, alkylthio en C₁ à C₄, ou un reste phényle, phénoxy ou phénylthio chacun éventuellement substitué par un radical fluoro, chloro, bromo, nitro, cyano, alkoxy en C₁ ou C₂, fluoralkoxy en C₁ ou C₂, alkylthio en C₁ ou C₂, fluoralkylthio en C₁ ou C₂ ou alkyle en C₁ à C₃,
R⁵ représente un reste méthoxy, éthoxy, méthylthio ou éthylthio,
R⁶ est un reste alkyle en C₁ à C₄, cycloalkyle en C₃ à C₆, alkoxy en C₁ à C₄, alcényle en C₃ ou C₄, (alkoxy en C₁ à C₄)-(alkyle en C₁ à C₄), un reste phényle éventuellement substitué par un radical fluoro, chloro, bromo, trifluorométhyle, méthyle ou méthoxy, un reste benzyle éventuellement substitué par un radical fluoro, chloro, bromo, méthyle, trifluoro-méthyle ou méthoxy,
R⁷ représente l'hydrogène, un reste méthyle, éthyle, propyle ou allyle, ou bien
R⁶ et R⁷ forment ensemble un reste alkylène en C₅ ou C₆ dans lequel, le cas échéant, un groupe méthylène est remplacé par de l'oxygène ou du soufre.

5. Composés de formule (I) suivant la revendication 1, formule dans laquelle
Het représente un groupe 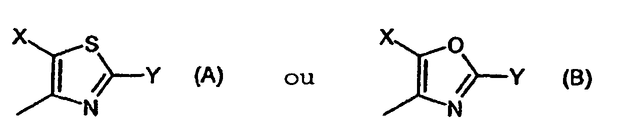
X représente l'hydrogène, le chlore, le brome, un reste méthyle, éthyle, n-propyle ou iso-propyle,
Y représente un reste méthyle, éthyle, n-propyle, isopropyle, n-butyle, iso-butyle, tertiobutyle, trifluorométhyle ou le groupe 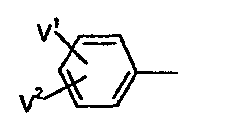 ou aussi, au cas où Het est un reste thiazolyle ((I-1-A) à (I-3-A)), un groupe 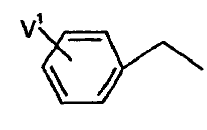
où
V¹ représente l'hydrogène, le fluor, le chlore, le brome, un reste méthyle, éthyle, n-propyle, isopropyle, n-butyle, iso-butyle, tertiobutyle, méthoxy, éthoxy, n-propoxy, iso-propoxy, trifluorométhyle, trifluorométhoxy, phénoxy, ou un reste phényle éventuellement substitué par un radical chloro ou trifluorométhyle,
V² désigne l'hydrogène, le fluor, le chlore, un reste méthyle, éthyle, méthoxy ou trifluorométhyle, ou bien
V¹ et V² forment ensemble un groupe -O-CH₂-O- ou -O-CF₂-O-,
W représente N-D⁽¹⁾, l'oxygène⁽²⁾ ou le soufre⁽³⁾,
A désigne l'hydrogène ou un reste alkyle en C₁ à C₄ ou cyclopropyle,
B représente l'hydrogène ou un reste méthyle, ou bien
A, B et l'atome de carbone auquel ils sont liés forment un reste cycloalkyle saturé en C₅ ou C₆, dans lequel le cas échéant un chaînon du noyau est remplacé par de l'oxygène ou du soufre et qui est éventuellement substitué une ou deux fois par un radical méthyle, éthyle, propyle, isopropyle, trifluorométhyle, méthoxy, éthoxy, propoxy, butoxy ou iso-butoxy, ou forment un reste cycloalkyle en C₅ ou C₆ dans lequel deux atomes de carbone non directement contigus forment un autre noyau pentagonal,
D représente l'hydrogène,
D représente aussi un reste isopropyle,
A et D forment conjointement avec les atomes auxquels ils sont liés un reste cyclohexyle dans lequel un atome du noyau peut être remplacé par du soufre,
G représente l'hydrogène (a) ou bien, au cas où Het est un reste thiazolyle (I-1-A) à (I-3-A), l'un des groupes 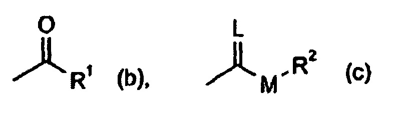
dans lesquels
L représente l'oxygène et
M représente l'oxygène,
R¹ désigne un reste alkyle en C₁ à C₄, alcényle en C₂ à C₄, (alkoxy en C₁ à C₄) - (alkyle en C₁ ou C₂), (alkylthio en C₁ à C₄) - (alkyle en C₁ ou C₂), chacun éventuellement substitué par du fluor ou du chlore,
ou bien un reste cyclohexyle ou cyclopropyle éventuellement substitué par un radical fluoro, chloro, méthyle, éthyle ou méthoxy,
un reste phényle éventuellement substitué une fois par un radical fluoro, chloro, bromo, cyano, nitro, méthyle, éthyle, n-propyle, iso-propyle, tertiobutyle, méthoxy, éthoxy, iso-propoxy, tertiobutoxy, trifluorométhyle ou trifluorométhoxy,
un reste thiényle ou pyridyle chacun éventuellement substitué une fois par un radical chloro ou méthyle,
R² représente un reste alkyle en C₁ à C₄, alcényle en C₂ à C₄ ou (alkoxy en C₁ à C₄) - (alkyle en C₂ ou C₃) chacun éventuellement substitué par du fluor,
un reste cyclohexyle éventuellement substitué par un radical méthyle, éthyle, n-propyle, iso-propyle ou méthoxy,
ou bien un reste phényle ou benzyle, chacun éventuellement substitué une fois par un radical fluoro, chloro, cyano, nitro, méthyle, éthyle, méthoxy, trifluorométhyle ou trifluorométhoxy.

6. Composés de formule (I) suivant la revendication 1, formule dans laquelle
Het représente un groupe 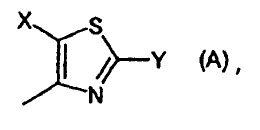
X désigne un reste méthyle, éthyle, n-propyle, isopropyle, le chlore ou le brome,
Y représente un reste méthyle, éthyle, n-propyle ou iso-propyle ou les groupes 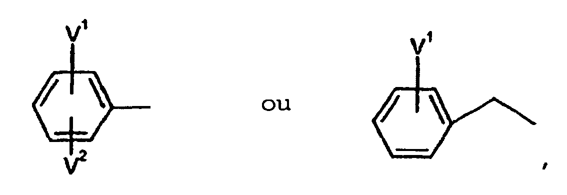
dans lesquels
V¹ représente l'hydrogène, le brome, le chlore, un reste méthyle, trifluorométhyle, phénoxy ou tertiobutyle,
ou un reste phényle éventuellement substitué par un radical chloro ou trifluorométhyle,
V² désigne l'hydrogène, le chlore, le fluor ou un reste méthoxy, ou bien
V¹ et V² forment ensemble un groupe -O-CH₂-O- ou -O-CF₂-O-,
W représente N-D⁽¹⁾, l'oxygène⁽²⁾ ou le soufre⁽³⁾,
D représente l'hydrogène ou un reste iso-propyle,
A est un reste méthyle, éthyle, n-propyle, iso-propyle ou cyclopropyle ou l'hydrogène,
B représente l'hydrogène ou le reste méthyle, ou bien
A, B et l'atome de carbone auquel ils sont liés forment un reste cyclohexyle dans lequel, le cas échéant, un atome du noyau est remplacé par de l'oxygène et qui est éventuellement substitué une ou deux fois par un radical méthyle, éthyle, méthoxy ou éthoxy, ou bien un reste cyclohexyle dans lequel deux atomes de carbone non directement contigus forment un autre noyau carboné pentagonal,
A et D forment, conjointement avec les atomes auxquels ils sont liés, un reste cyclohexyle dans lequel un atome du noyau peut être remplacé par du soufre,
G représente l'hydrogène (a) ou l'un des groupes 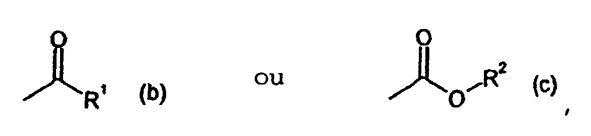
dans lesquels
R¹ est un reste méthyle, éthyle, n-propyle ou isopropyle, ou bien un reste phényle ou pyridyle chacun éventuellement substitué par du chlore,
R² est un reste méthyle, éthyle, phényle, benzyle, npropyle ou iso-propyle.

7. Composés de formule (I) suivant la revendication 1, formule dans laquelle Het est un groupe 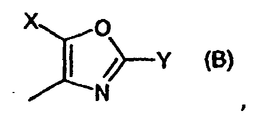
X est un reste méthyle ou éthyle,
Y est un reste phényle éventuellement substitué par du chlore,
A désigne un reste méthyle, éthyle, n-propyle ou isopropyle,
B est un reste méthyle, ou bien
A, B et l'atome de carbone auquel ils sont liés forment ensemble un reste cyclohexyle qui est éventuellement substitué par un radical méthoxy ou méthyle,
W représente N-D ou l'oxygène,
D représente l'hydrogène,
G représente l'hydrogène.

8. Procédés de production de composés de formule (I) suivant la revendication 1, **caractérisés en ce que** :
(A) on obtient des composés de formules (I-1-A-a) à (1-1-B-a) 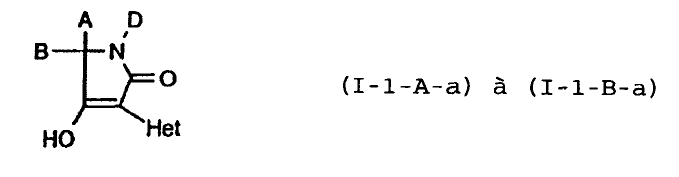
dans laquelle
A, B, D et Het ont les définitions indiquées ci-dessus,
en effectuant la condensation intramoléculaire de composés de formule (II) 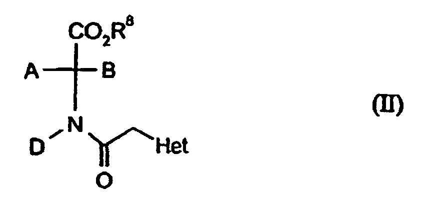
dans laquelle
A, B, D et Het ont les définitions indiquées ci-dessus,
et
R⁸ représente un reste alkyle,
en présence d'un diluant et en présence d'une base,
(B) on obtient des composés de formules (I-2-A-a) à (1-2-B-a) 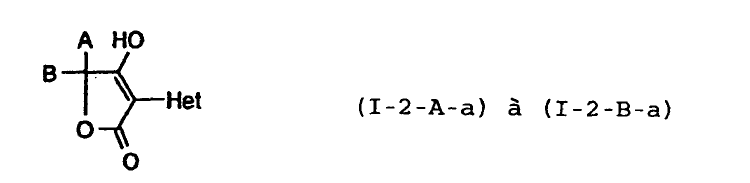
dans laquelle
A, B et Het ont les définitions indiquées ci-dessus,
en effectuant la condensation intramoléculaire de composés de formule (III) 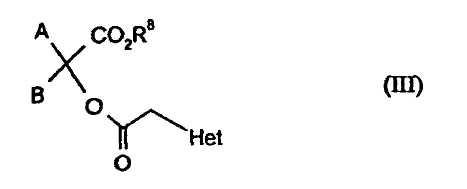
dans laquelle
A, B, Het et R⁸ ont les définitions indiquées ci-dessus,
en présence d'un diluant et en présence d'une base,
(C) on obtient des composés de formules (I-3-A-a) à (1-3-B-a) 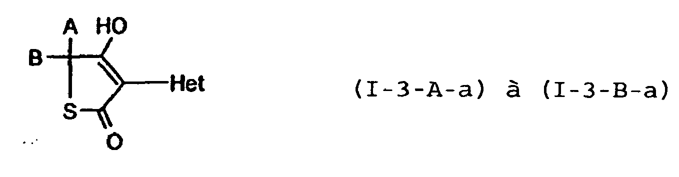
dans laquelle
A, B et Het ont les définitions indiquées ci-dessus,
en effectuant la cyclisation intramoléculaire de composés de formule (IV) 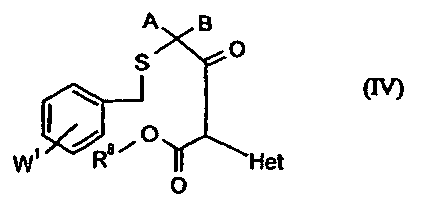
dans laquelle
A, B, Het et R⁸ ont les définitions indiquées ci-dessus, et
W¹ représente l'hydrogène, un halogène, un reste alkyle ou alkoxy,
éventuellement en présence d'un diluant et en présence d'un acide,
et le cas échéant, les composés ainsi obtenus de formules (I-1-A-a) à (I-3-B-a), dans lesquelles
A, B, D et Het ont chacun les définitions indiquées ci-dessus,
sont amenés à réagir respectivement
(D)
(α) avec des halogénures d'acides de formule (V) 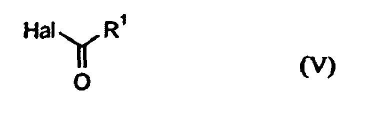
dans laquelle
R¹ a la définition indiquée ci-dessus et
Hal désigne un halogène
ou bien
(β) avec des anhydrides d'acides carboxyliques de formule (VI)
R¹-CO-O-CO-R¹ (VI)
dans laquelle
R¹ a la définition indiquée ci-dessus,
é ventuellement en présence d'un diluant et, le cas échéant, en présence d'un accepteur d'acides ;
(E) avec des esters d'acide chloroformique ou des thioesters d'acide chloroformique de formule (VII)
R²-M-CO-Cl (VII)
dans laquelle
R² et M ont les définitions indiquées ci-dessus,
éventuellement en présence d'un diluant et, le cas échéant, en présence d'un accepteur d'acides ;
(F) avec des esters d'acide chloromonothioformique ou des esters d'acide chlorodithioformique de formule (VIII) 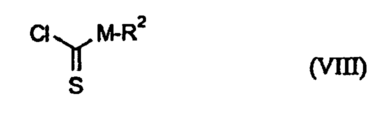
dans laquelle
M et R² ont les définitions indiquées ci-dessus,
éventuellement en présence d'un diluant et, le cas échéant, en présence d'un accepteur d'acides,
et
(G) avec des chlorures d'acides sulfoniques de formule (IX)
R³-SO₂-Cl (IX)
dans laquelle
R³ a la définition indiquée ci-dessus,
éventuellement en présence d'un diluant et, le cas échéant, en présence d'un accepteur d'acides,
(H) avec des composés phosphorés de formule (X) 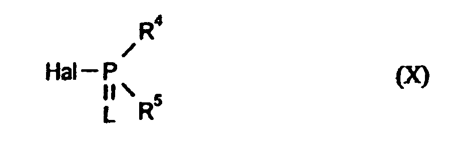
dans laquelle
L, R⁴ et R⁵ ont les définitions indiquées ci-dessus et
Hal représente un halogène,
éventuellement en présence d'un diluant et, le cas échéant, en présence d'un accepteur d'acides,
(I) avec des composés métalliques ou des amines de formule (XI) ou (XII)
Me(OR¹⁰)ₜ (XI) 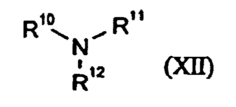
dans lesquelles
Me désigne un métal monovalent ou divalent,
t représente le nombre 1 ou 2 et
R¹⁰, R¹¹ et R¹² représentent, indépendamment les uns des autres, l'hydrogène ou un reste alkyle, éventuellement en présence d'un diluant,
(J)
(α) avec des isocyanates ou des isothiocyanates de formule (XIII)
R⁶-N=C=L (XIII)
dans laquelle
R⁶ et L ont les définitions indiquées ci-dessus,
éventuellement en présence d'un diluant et, le cas échéant, en présence d'un catalyseur, ou bien
(β) avec des chlorures d'acides carbamiques ou des chlorures d'acides thiocarbamiques de formule (XIV) 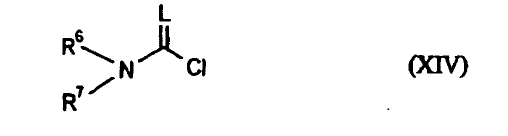
dans laquelle
L, R⁶ et R⁷ ont les définitions indiquées ci-dessus,
éventuellement en présence d'un diluant et, le cas échéant, en présence d'un accepteur d'acides.

9. Composés de formule (II) 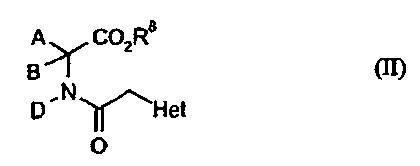
dans laquelle
A, B, D, Het et R⁸ ont les définitions indiquées ci-dessus, excepté les composés :

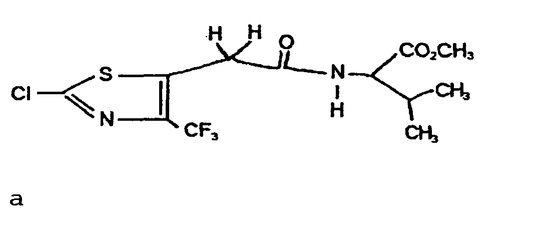

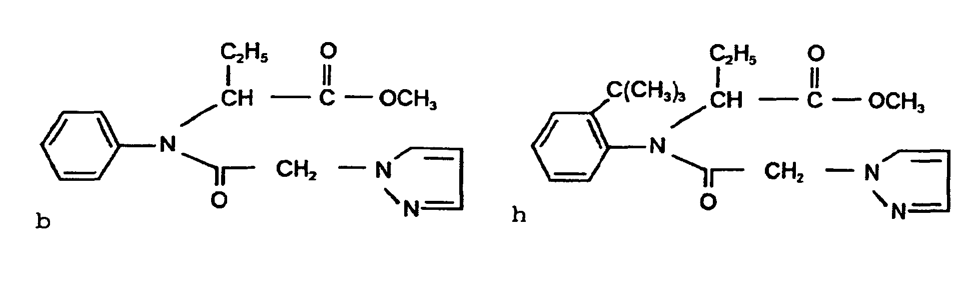

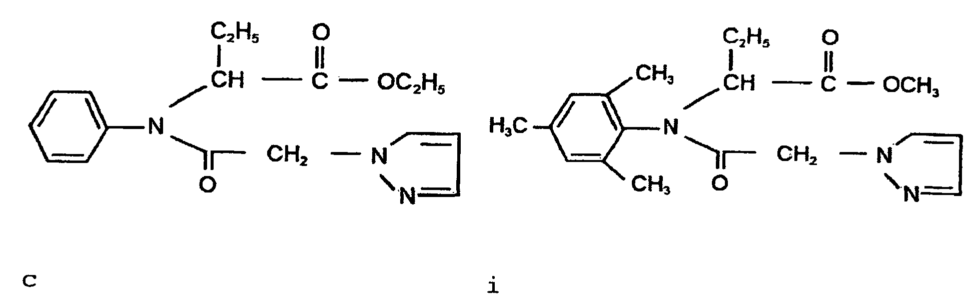

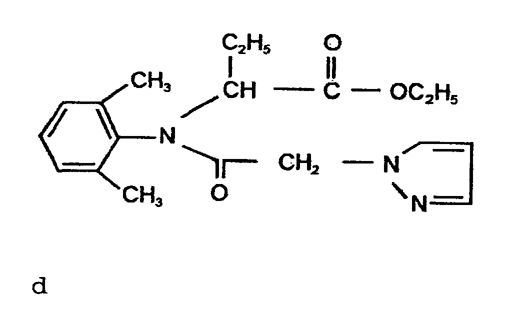

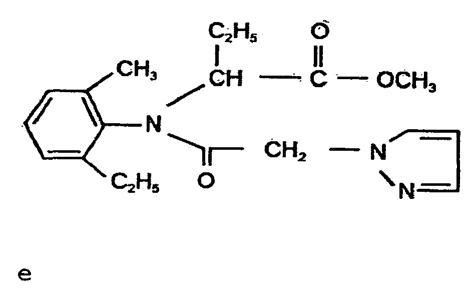

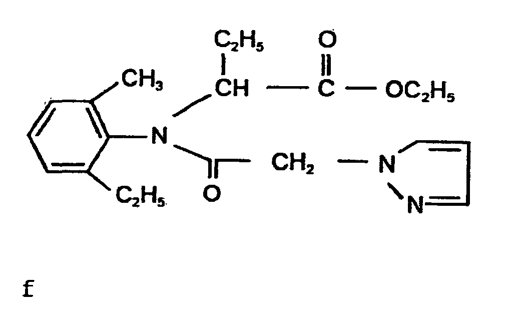

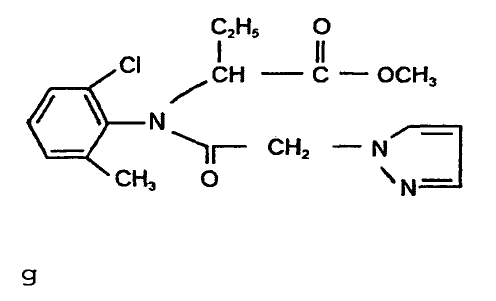

10. Composés de formule (XVII) 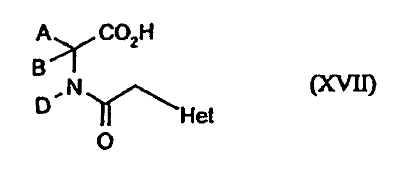
dans laquelle
A, B, D et Het ont les définitions indiquées ci-dessus.

11. Composés de formule (XXI) 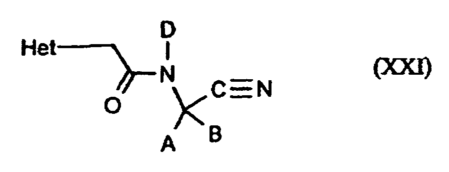
dans laquelle
A, B, D et Het ont les définitions indiquées ci-dessus.

12. Composés de formule (III) 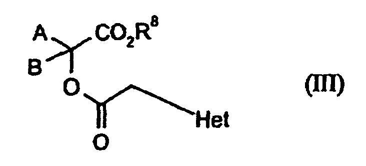
dans laquelle
A, B, Het et R⁸ ont les définitions indiquées ci-dessus, excepté le composé :

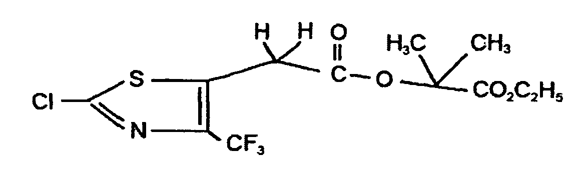

13. Composés de formule (IV) 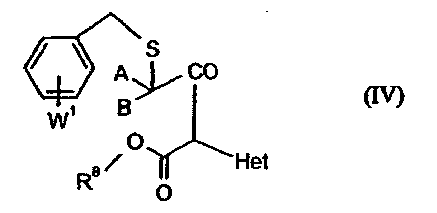
dans laquelle
A, B, W¹, Het et R⁸ ont les définitions indiquées ci-dessus, et
W¹ représente l'hydrogène, un halogène, un reste alkyle ou alkoxy.

14. Compositions pesticides et herbicides, **caractérisées par** une teneur en au moins un composé de formule (I) suivant la revendication 1.

15. Utilisation de composés de formule (I) suivant la revendication 1 pour combattre des parasites et des mauvaises herbes.

16. Procédés pour combattre des parasites et des mauvaises herbes, **caractérisés en ce que** l'on fait agir des composés de formule (I) suivant la revendication 1 sur des parasites et/ou sur leur milieu ou bien on les fait agir sur des mauvaises herbes et/ou sur leur milieu.

17. Procédés de préparation de compositions pesticides et d'herbicides, **caractérisés en ce que** l'on mélange des composés de formule (I) suivant la revendication 1 avec des diluants et/ou des agents tensioactifs.

18. Utilisation de composés de formule (I) suivant la revendication 1 pour la préparation de compositions pesticides et d'herbicides.
